# EUROPEAN PATENT APPLICATION

(11) **EP 4 821 533 A2**
(43) Date of publication of application: **14.10.2026**
(21) Application number: 26187050.5
(22) Date of filing: 10.01.2022
(51) Int. Cl.: A61M 16/20

(54) **DATA-INTEGRATED ARTIFICIAL VENTILATION SYSTEM**

(30) Priority: 08.01.2021 US 202163135263 P
(62) Document number(s) of the earlier application(s) in accordance with Art. 76 EPC: 22701807.4 / 4 274 650
(71) Applicant: Airmid Critical Care Products, Inc., Washington, DC 20002 (US)
(72) Inventor: MAGUIRE, Michael D., Washington, DC 20002 (US)
(74) Representative: Elkington and Fife LLP

(57) **Abstract**

A system for artificial ventilation including a convertible ventilator circuit having at least one inspiratory filter and at least one expiratory filter. The convertible ventilator circuit compatible with and configured to be used with either a manual ventilator or a mechanical ventilator. The convertible ventilator circuit configured to convert between manual ventilation use and mechanical ventilation use. The system including a patient manifold having one or more sensor ports and a data acquisition unit having one or more sensors configured to interface with the one or more sensor ports of the patient manifold. The data acquisition unit configured to receive electronic data associated with transmission of respiratory gases through the convertible ventilator circuit.

## Description

### CROSS REFERENCE TO RELATED APPLICATIONS

This application claims the benefit of and priority to U.S. Provisional Patent Application No. 63/135,263, filed January 8, 2021, the entire contents of which are owned by the assignee of the instant application and incorporated herein by reference in their entirety.

### FIELD OF THE INVENTION

The present invention relates generally to the field of protecting patients from harmful effects of artificial ventilation, and more specifically to obtaining data during artificial ventilation in medical or veterinarian applications.

### BACKGROUND OF THE INVENTION

Artificial ventilation is the process of providing external breathing support to augment or even take the place of natural breathing function. This is typically achieved by a sequential process of: (1) forcing pressurized breathing gas into a patient, either via a face mask or through a tube (endotracheal tube) that is advanced directly into the windpipe (or trachea); (2) allowing the pressurized breathing gas to progressively inflate the lungs; and (3) pausing forced inflation in order to allow the inflated lungs to passively deflate via lung elasticity and chest wall resistance. Alternating between active inflation and allowing for passive deflation constitutes a method to achieve artificial inspiration and expiration, thereby constituting a means for artificial ventilation.

Artificial ventilation devices and methodologies are generally divided into two categories. One category focuses on initiation of artificial ventilation followed by short-term support. The second category constitutes devices and methods for longer-term, sustained artificial ventilation.

Initiation of artificial ventilation, where a patient is susceptible to death without immediate support, must be instituted quickly. This is invariably achieved with a manual ventilator that comprises a simple device that is user powered (e.g., by hand operation) and able to be rapidly utilized by a provider. The most prominent among devices used to initiate manual ventilation is the bag-type manual ventilator, whereby an operator hand-squeezes a bag filled with inspiratory gas that, under pressure from the user's hands, then enters the patient via a face mask forming an air-tight seal over the nose and mouth of the patient.

Bag-type manual ventilators are widely used to initiate artificial ventilation for several reasons. First, their compatibility with a face mask and other airway adjuncts that can be used emergently enables them to be employed immediately without delay. Second, manual ventilators are typically simple, inexpensive devices, thereby enabling them to be economically staged in readiness in out-of-hospital emergency vehicles and throughout hospitals in multiple places (e.g., on emergency "crash" carts) to ensure they are never far from patients when needed emergently. Finally, bag-type manual ventilators have a diagnostic component in that users can palpably assess the resistance required to achieve lung inflation, which may be instructive when treating a patient whereby the cause of respiratory compromise has yet to be established (such as a patient being treated by emergency personnel with unknown internal injuries).

Upon initiation of manual ventilation, patients can be expected to either rapidly recover or require sustained support. Patients expected to rapidly recover will continue being manually ventilated with a face mask, while patients that are expected to require sustained support will undergo a delicate medical procedure that inserts an endotracheal tube into the trachea (or windpipe). Use of an endotracheal tube has the advantage that it ensures a definitive air-tight passageway to the lungs. Accordingly, after initiation of manual ventilation, short-term support can continue either via a face mask or endotracheal tube.

It should be noted that several alternative embodiments of an endotracheal tube exist. An endotracheal tube is inserted directly into the trachea by oral access through the mouth. A nasotracheal tube is identical to an endotracheal tube except that it is typically of a longer length to enable the nasotracheal tube to be inserted directly into the trachea via the right or left nostril of the nose and through the nasopharynx. Some endotracheal and nasotracheal tubes have other features that are equally applicable to the scope of the present invention, and it shall be construed that all references to an endotracheal tube include all embodiments of endotracheal and nasotracheal tubes.

Offsetting the aforementioned benefits of bag-type manual ventilators is the fact they currently lack any ability to precisely control breath size, breath rate, and other key variables associated with the safety of artificial ventilation. Some studies have shown that some providers inadvertently and endemically over-inflate the lungs when delivering breaths manually, with data suggesting over-inflation could be sufficiently excessive to cause tearing lung tissue injury after as little as 20 minutes of severe over-inflation. This lack of control has been suggested as a principal cause of VILI, however manual ventilators also lack the ability to obtain any data to qualify and/or quantify potentially harmful ventilation. Therefore, the true nature of potential patient harm represented by bag-type manual ventilators during patient care is both unmeasurable and beyond ability to mitigate.

For patients who have an endotracheal tube placed, ventilation can then be transitioned from manual hand-delivered breaths to the second category of devices that are designed for sustained, automated, hand-free support. For patients undergoing manual ventilation via a face mask, endotracheal intubation must generally first take place before a patient can be placed on a mechanical ventilator.

Even after endotracheal intubation has been successfully completed, there remain several additional steps associated with transitioning a patient from manual ventilation to mechanical ventilation. First, mechanical ventilators, typically being very costly medical devices, cannot be staged in readiness to the same degree as manual ventilators, resulting in the need to first transport the ventilator to the patient bedside. Second, a set-up and programming procedure must be completed to establish breath delivery settings suitable for the individual patient. Once the mechanical ventilator is ready, the manual ventilator can then be disconnected from the endotracheal tube - temporarily exposing the patient to airborne pathogens in the room atmosphere (as well as exposing room occupants to patient exhaled gases) - and the mechanical ventilator then connected to the endotracheal tube via a tube "circuit." The ventilator circuit typically comprises two tubes that provide fluid connections between the mechanical ventilator and the patient, with one tube providing 1-way inspiratory gas flow from the ventilator to the patient, with the second tube providing 1-way expiratory gas flow from the patient back to the ventilator. Since the endotracheal tube has only a single fluid connection, the ventilator circuit typically has an elbow or wye that has three connections for: (1) the inhalation tube (or limb) from the ventilator; (2) the exhalation tube (or limb); and (3) the endotracheal tube. Once the circuit is connected to both the ventilator and the patient, mechanical ventilation can be initiated.

Notably, there are occasions where patients undergoing mechanical ventilation are temporarily switched back to support via a manual ventilator. In the event of suspected ventilator malfunction (e.g., from power failure and/or loss of compressed gas source), a manual ventilator is typically stored in readiness within reach of the bedside so the patient does not die from apnea when the mechanical ventilator becomes inoperative with no alternative at hand. Additionally, mechanical ventilators typically used in hospitals are floor-mounted, presenting challenges during patient transport within the hospital. In the event the mechanical ventilator cannot be maneuvered in exact tandem with the patient bed, tractive forces placed on the ventilator circuit could cause ventilator circuit disconnection or, worse, inadvertent removal of the endotracheal tube altogether (a life-threatening complication). For these reasons, mechanical ventilators are generally unsuitable for use during patient transport, whereby a manual ventilator will be typically used for this purpose. Finally, manual ventilators are most suitable for use during clinical emergencies. For example, if a patient on a mechanical ventilator requires emergency resuscitation, one of the first actions will be to disconnect the patient from the mechanical ventilator circuit in order to then emergently connect a manual ventilator. Accordingly, most patients undergoing mechanical ventilation are exposed to mode changes between manual and mechanical ventilation that are numerous, repetitive, and commonplace.

In addition to a face mask and endotracheal tube, there are additional components that are often used during artificial ventilation that require servicing. For example, a heat-moisture exchanger (HME) is a device that captures exhaled humidity that is at body temperature. When positioned between a ventilator and endotracheal tube, the HME uses captured heat and humidity to condition fresh inspiratory gas for each subsequent inspiration. These devices are susceptible to becoming clogged with exhaled patient secretions and affecting ventilation in other ways, resulting in frequent changing as a preventative, but wasteful, measure to offset patient risk.

### SUMMARY OF THE INVENTION

The present invention provides a synergistic combination of devices and methodologies that at least partially address the unmet needs in the field. A basic understanding of the invention can be ascertained from the following summary, with more details to follow in the detailed description and associated drawings.

The invention generally comprises a multitude of components for artificial ventilation that share compatibility with each other, thereby constituting a system for artificial ventilation. For example, in place of manual ventilators that are incompatible with ventilation circuits used with mechanical ventilators, the present invention provides: (1) a convertible ventilator circuit having inspiratory and expiratory filters, with said circuit compatible with and able to be used with either a manual ventilator or a mechanical ventilator, thus constituting a ventilator circuit able to convert from use during manual ventilation to use during mechanical ventilation (and vice versa); (2) a data acquisition unit (DAU) having one or more sensors that interface with one or more sensor ports of the convertible ventilator circuit, thereby adding data integration to the convertible ventilator circuit such that data is acquired from respiratory gases as they pass through the now data-integrated convertible ventilator circuit (DiCVC); (3) the DAU as described with one or more additional sensors that interface with one or more sensor ports of an airway adjunct in physical contact with a patient; (4) a data processing unit (DPU) that processes, retains and/or electronically transmits data obtained from the DAU and may be directly and/or indirectly to one or more interfacing components connected to the DiCVC (and/or otherwise connected electrically, wirelessly, pneumatically, or by other means), and which may be shared in a single housing with the DAU, contained in a separate housing, or contained within the housing of a neighboring device (e.g., a mechanical ventilator, bedside monitor, defibrillator, capnography device, or other electronic medical device); (5) a manual ventilator compatible with the DiCVC, with said manual ventilator preferably having one or more built-in settings to control one or more attributes of manual ventilation (such as tidal volume and/or positive end-expiratory pressure), and with said manual ventilator capable of being electronically detected by the DAU and/or DPU when connected to the inspiratory and/or expiratory limb ports of the DiCVC; (6) a mechanical ventilator compatible with a DiCVC, including one or more means for data transfer between the DAU and/or DPU on the DiCVC and a computer processor and/or logic circuit of a mechanical ventilator, and capable of being electronically detected by the DAU and/or DPU when connected to the inspiratory and/or expiratory limb ports of the DiCVC; (7) a heat-moisture exchanger (HME) having compatibility with a DiCVC that can monitor the HME for patency and/or gas flow such that HME replacement need only occur when HME function is measurably diminished; (8) one or more airway adjuncts (e.g., a face mask, supraglottic airway, or endotracheal tube) that are compatible with the DiCVC and capable of being electronically detected by the DAU and/or DPU when connected to the patient port of the DiCVC; and (9) a DiCVC cap that can be used to cover and seal the patient port when the DiCVC is staged prior to use and/or temporarily disconnected from a patient endotracheal tube, with said cap capable of being electronically detected by the DAU and/or DPU when connected to the patient port of the DiCVC.

These compatible components of the present invention enable a multitude of new methodologies that fundamentally provide an integrated approach to artificial ventilation which is inclusive of multiple phases of care that, currently, are distinct, separate procedures that utilize fundamentally different and incompatible devices. For example, as previously mentioned, manual ventilation is provided with devices that are incompatible with devices that provide mechanical ventilation, and that patients typically repetitively must pass back-and-forth between these different modalities. In contrast, the present invention provides a manual ventilator that is compatible with the DiCVC component of the invention, which is itself compatible with the airway adjunct that directly interfaces with the patient. When the patient transitions from manual to mechanical ventilation, the DiCVC remains connected to the airway adjunct - thereby providing data continuity - while the manual ventilator is disconnected and replaced with the DiCVC-compatible mechanical ventilator. Device and data continuity is also preserved when mechanical ventilation reverts back to manual ventilation.

Through this fundamental compatibility among components of the present invention, it can immediately be seen that having a DAU and DPU interfacing with a DiCVC that remains connected to the patient airway adjunct throughout the entire period of artificial ventilation, and even throughout conversions between manual and mechanical phases of ventilation, provides multiple elements of synergy that diminishes (or even eliminates) certain risks associated with VILI. First, it can be easily seen how the DiCVC enables data acquisition immediately upon initiation (i.e., from "breath one") of artificial ventilation with a manual ventilator. This is because the DiCVC is compatible with, and connected to, the compatible manual ventilator, thereby enabling data acquisition to occur from gas movement generated by the manual ventilator even though the manual ventilator itself need not have its own built-in sensors. Data acquired during manual ventilation is archived on the DPU and, upon the patient converting to mechanical ventilation by replacing the manual ventilator with a DiCVC-compatible mechanical ventilator, the historical record of artificial ventilation from "breath one" can then be exported to the DiCVC-compatible mechanical ventilator for display and/or redundant data storage and/or analysis. Similarly, data export can occur in the opposite direction from a DiCVC-compatible mechanical ventilator to the DPU so that, when a patient must be transitioned back to manual ventilation (e.g., in order to be moved within the hospital), the data "follows" the patient at all times. When then switching back to another mechanical ventilator, the previous ventilator settings can be relayed to the new mechanical ventilator, thereby protecting the patient from harm should new ventilator settings be suboptimal or even injurious. These elements for expanded data utilization and continuity provide both: (1) a continuous record from "breath one" of artificial ventilation during all phases of manual and mechanical ventilation, thereby constituting a means to identify at what point an occurrence of injurious ventilation may have taken place; and (2) an efficient means to achieve substantially homogenous artificial ventilation by means of the DPU being capable of maintaining and/or propagating ventilator settings even among variable modes (e.g., manual or mechanical) and/or multiple DiCVC-compatible mechanical ventilators.

The methodologies described above resulting in new acquisition of data is subsequently utilized to mitigate or even eliminate some causes of VILI. Data acquired by the DiCVC during initial manual ventilation will actively inform providers on the safety of manual ventilation itself which, as previously shown, has been demonstrated to potentially represent high patient risk for inadvertent and unrecognized over-inflation and subsequent mechanism for VILI. Sensor data acquired by the DAU can be used to formulate one or more new metrics for safety and/or compliance. In the event one or more elements of artificial ventilation are being delivered outside acceptable limits of one or more metrics for safety and/or compliance monitored by the DPU, the DPU can immediately warn providers that potentially injurious manual ventilation is taking place. This prompts the provider to take immediate corrective action that mitigates patient exposure to the associated mechanism for VILI. Once on a mechanical ventilator, settings for automated support undergo similar analysis to ensure settings are consistent with safety and compliance. Thereafter, when patients transition to care on a different mechanical ventilator, these settings that are already aligned with safety and compliance will then be automatically conveyed to the subsequent mechanical ventilator, thereby providing a means to homogenize artificial ventilation while further reducing risk for injurious ventilation settings.

The present invention can also be seen to provide multiple methodologies to protect patients from VAP. The DiCVC contains inspiratory and expiratory filters that both: (1) prevent passage of airborne pathogens in the ambient atmosphere from infecting the patient being ventilated; and (2) prevent passage of airborne pathogens in the patient being ventilated from infecting providers and/or neighboring patients. This means that once the DiCVC is connected to an airway adjunct, and since the DiCVC need not be disconnected from the airway adjunct to achieve conversions between manual and mechanical ventilators, that there is a material reduction in absolute patient exposures causing VAP due to constant presence of filtration. Additionally, in the event patients are being disconnected for other reasons, possibly in non-compliance with safety and/or hospital guidelines, then the data acquisition as previously described will record these events, as well as the length of time the patient was disconnected, thereby affecting the total risk that the patient was exposed to airborne pathogens capable of causing VAP.

The present invention also provides a means for sensors interfaced with the DAU to sample one or more parameters from an airway adjunct. The ability to connect a DAU pressure sensor to the endotracheal or tracheal tube cuff port will provide automatic ability to derive one or more metrics for safety and compliance capable of protecting patients from VAP (the harmful effect of an underinflated cuff) and tracheal ischemia or necrosis (the harmful effect of an overinflated cuff).

Further objects, features and advantages will come to be understood from the detailed description together with the associated drawings.

### DESCRIPTION OF THE DRAWINGS

The advantages of the invention described above may be better understood by referring to the following description taken in conjunction with the accompanying drawings. The drawings are not necessarily to scale, with some structural elements enlarged to highlight structure and function, with emphasis generally placed upon illustrating the principles of the invention.
Fig. 1 is a side view of the primary physical components of an exemplar artificial ventilation system including, from left to right: (1) a combined DAU+DPU sharing a singular housing; (2) convertible ventilator circuit having a patient manifold; and (3) an exemplar manual ventilator compatible with the convertible ventilator circuit. The two limbs of the convertible ventilator circuit are depicted in vertical alignment on the same vertical plane for illustrative purposes (so both limbs are visible).
Fig. 2 provides another side view of the same components as Fig. 1, however with the DAU and DPU preferably comprising separate housings. The two limbs of the convertible ventilator circuit are depicted in horizontal alignment on the same horizontal plane, causing the limb depicted in the foreground to obscure view of the limb in the background.
Fig. 3 depicts the identical components as Fig. 2 in the same side view, with the DAU and DPU shown installed on the convertible ventilator circuit in their functional positions. An exemplar manual ventilator is also shown installed with the DiCVC in its functional position.
Fig. 4 depicts most of the same components as Fig. 3 in the same side view, with the exception comprising replacement of the exemplar manual ventilator with an exemplar mechanical ventilator. Additionally, the DPU is shown in a preferably recessed position in a receptacle within the housing of the mechanical ventilator.
Fig. 5 depicts an exemplar convertible circuit patient manifold in a configuration representative of gas flow during an inspiratory phase of a single breath cycle.
Fig. 6 depicts the same components as Fig. 5 in a configuration representative of temporary zero gas flow during an inspiratory hold phase of a single breath cycle.
Fig. 7 depicts the same components as Fig. 6 in a configuration representative of gas flow during an expiratory phase of a single breath cycle.
Fig. 8 depicts an exemplar convertible circuit patient manifold specifically highlighting the approximate aggregate area for sensor measurement of gas during all three phases of a breath cycle. This area also represents the total volume of gases under pressure during an inspiratory hold phase of a single breath cycle.
Fig. 9 is an identical depiction of Fig. 8 at a larger scale showing the entirety of the convertible ventilator circuit, specifically highlighting the small relative area for sensor measurement of gas during all three phases of a breath cycle. This area also represents the total volume of gases under pressure during an inspiratory hold phase of a single breath cycle.
Fig. 10 depicts an exemplar ventilator circuit that is non-convertible and designed to be used with mechanical ventilators only, with said non-convertible circuit not having a patient manifold, and specifically showing the approximate aggregate area for sensor measurement of gas during all three phases of a breath cycle. This aggregate area also represents the total volume of gases under pressure during an inspiratory hold phase of a single breath cycle.
Fig. 11 shows a side view of a preferred embodiment of a convertible ventilator circuit patient manifold whereby the connectors to each limb result in a substantially side-by-side arrangement aligned on a horizontal plane, causing the inspiratory limb connector in the foreground to obscure the view of the expiratory limb connector in the background.
Fig. 12 shows a bottom-up view of the same preferred embodiment of a convertible ventilator circuit patient manifold shown in Fig. 11, with this view enabling both the inspiratory limb connector and expiratory limb connector to be simultaneously visible.
Fig. 13 shows an end-on view of the same preferred embodiment of a convertible ventilator circuit patient manifold shown in Fig. 12, whereby the gas passageway for inspiratory gas flow transitions from a left-to-top arrangement, while the gas passageway for expiratory gas flow transitions from a bottom-to-right arrangement.
Fig. 14 shows the convertible ventilator circuit patient manifold of Fig. 11, with further depiction of one or more sensor mounting ports that provide for one or more sensors to access gas passageways, along with a preferred location for an in-line flow sensor.
Fig. 15 shows the convertible ventilator circuit patient manifold of Fig. 12, with further depiction of one or more sensor mounting ports that provide for one or more sensors to access gas passageways.
Fig. 16 shows the convertible ventilator circuit patient manifold of Fig. 13, with further depiction of one or more sensor mounting ports that provide for one or more sensors to access gas passageways, along with a preferred location for an in-line flow sensor.
Fig. 17a shows a side view of a DAU, with Fig. 17b providing an end-on view.
Fig. 18 shows a bottom-up view of the DAU of Figs. 17a and 17b.
Fig. 19 informs on a preferred embodiment of convertible ventilator circuit patient manifold and DAU, whereby the outer surface of the convertible ventilator circuit patient manifold and inner surface of the DAU are configured to facilitate an installation process that can be completed by sliding the DAU over the convertible ventilator circuit patient housing along the plane depicted.
Fig. 20 shows the relative positioning of the same components of Fig. 19 upon completion of the installation process.
Fig. 21 shows a bottom-up view of the convertible ventilator circuit patient manifold of Fig. 15 and DAU of Fig. 18 upon completion of the installation process.
Fig. 22 shows an end-on view of the convertible ventilator circuit patient manifold of Fig. 16 and DAU of Fig. 17b upon completion of the installation process.
Fig. 23 shows a side view of a preferred embodiment of convertible ventilator circuit patient manifold of Fig. 11, further showing exemplar locations of electrical transmission and sensing components.
Fig. 24 shows a side view of the DAU of Fig. 17a, further showing exemplar locations of electrical transmission and sensing components.
Fig. 25a shows a side view of the DAU of Fig. 24, while Fig. 25b shows a side view of the convertible ventilator circuit patient manifold of Fig. 23, with both components in an uninstalled configuration.
Fig. 26 shows the side view of the of the DAU of Fig. 25a and the side view of the convertible ventilator circuit patient manifold of Fig. 25b in an installed configuration.
Fig. 27a shows an end-on view of an exemplar manual ventilator having a rotational control for a ventilation delivery parameter, including electrical and sensory components enabling an interfaced DAU/DPU to ascertain the rotational position of the rotational control. Fig. 27b shows the same components of Fig. 27a in a side view.
Figs. 28a and 28b show the same embodiment of exemplar manual ventilator shown in Figs. 27a and 27b after a one-quarter anti-clockwise turn of the exemplar manual ventilator.
Fig. 29a shows a rotational control for a ventilation delivery parameter, whereby the ventilation delivery parameter is tidal volume with the rotational control in a configuration representative of a tidal volume setting of 250mL; Fig. 29b shows the same components of Fig. 29a, except the rotation control has been rotated anti-clockwise to a configuration representative of a tidal volume setting of 500mL.
Figs. 30a and 30b show an alternative embodiment in the same views and configuration for a rotational control for a ventilation delivery parameter.
Figs. 31a and 31b show a linear control for a ventilation delivery parameter, whereby the ventilation delivery parameter is a partial flow restrictor. Fig. 31a shows the flow restrictor in a fully open configuration; Fig. 31b shows the relative position of the linear control and associated electrical and sensing components.
Figs. 32a and 32b show the same components of Figs. 31a and 32b, except Fig. 32a shows the flow restrictor in a fully engaged configuration; Fig. 32b shows the relative position of the linear control.
Fig. 33 shows components also seen in Fig. 3 in an identical left side view, further showing associated electrical and sensing components of a DPU, convertible ventilator circuit inspiratory limb connector, and manual ventilator inspiratory limb connector.
Fig. 34 shows the same components of Fig. 33 in a right side view, except a convertible ventilator circuit expiratory limb connector and manual ventilator expiratory limb connector are visible in this view in place of the convertible ventilator circuit inspiratory limb connector, and manual ventilator inspiratory limb connector that are visible in Fig. 33.
Fig. 35 shows an airway adjunct connector with associated electrical and sensing components.
Fig. 36 shows the same components of Fig. 26 plus Fig. 35, showing the airway adjunct connector installed in the convertible ventilator circuit patient manifold airway adjunct receptacle, with the outer surface of said airway adjunct connector fitting inside the inner surface of the said convertible ventilator circuit patient manifold airway adjunct receptacle.
Fig. 37 shows the same components of Fig. 36, except an alternative embodiment of airway adjunct connector installed on the convertible ventilator circuit patient manifold airway adjunct receptacle, with the inner surface of said airway adjunct connector fitting outside the outer surface of the said convertible ventilator circuit patient manifold airway adjunct receptacle.
Fig. 38a shows the same components of Fig. 36, except the airway adjunct connector is partially installed in the convertible ventilator circuit patient manifold airway adjunct receptacle. Fig. 38b shows the same components of Fig. 37, except the airway adjunct connector is partially installed on the convertible ventilator circuit patient manifold airway adjunct receptacle.
Fig. 39 shows a DAU installed on a convertible ventilator circuit patient manifold, with the latter also interfaced with an airway adjunct in the form of a preferred embodiment of a face mask that includes a face mask cuff, whereby said face cuff has a pressure tube that is connected to an auxiliary sensor that is a pressure sensor on said DAU. The face mask cuff is seen in a substantially slack configuration.
Fig. 40 shows the same components of Fig. 39, except the face mask cuff is seen in a substantially taut configuration from being firmly applied to the face of a patient.
Fig. 41 shows a DAU installed on a convertible ventilator circuit patient manifold, with the latter also interfaced with an airway adjunct in the form of a preferred embodiment of an endotracheal tube having an endotracheal tube cuff, whereby said endotracheal tube cuff has a pressure tube that is connected to an auxiliary sensor that is a pressure sensor on said DAU.
Fig. 42 shows a DAU installed on a convertible ventilator circuit patient manifold, with the latter also interfaced with an airway adjunct in the form of a preferred embodiment of a tracheal tube having a tracheal tube cuff, whereby said tracheal tube cuff has a pressure tube that is connected to an auxiliary sensor that is a pressure sensor on said DAU.
Fig. 43 shows a side view of a Heat-Moisture Exchanger (HME) and associated electrical and sensing components.
Fig. 44a shows a side view of the HME of Fig. 43, rotated approximately 15 degrees on the vertical axis, showing ports for temperature and/or humidity probes. Fig. 44b shows an identical side view of the HME of Fig. 44a, further showing temperature and/or humidity probes installed.
Fig. 45a shows a bottom-up view of the HME shown in Fig. 44a, further showing the lowermost sensor port for a temperature and/or humidification probe. Fig. 45b shows the same components and view of Fig. 45a, additionally showing lowermost temperature and/or humidity probe installed.
Fig. 46 shows the HME in the identical view of Fig. 43, further showing an airway adjunct connector installed in the airway adjunct port of the HME.

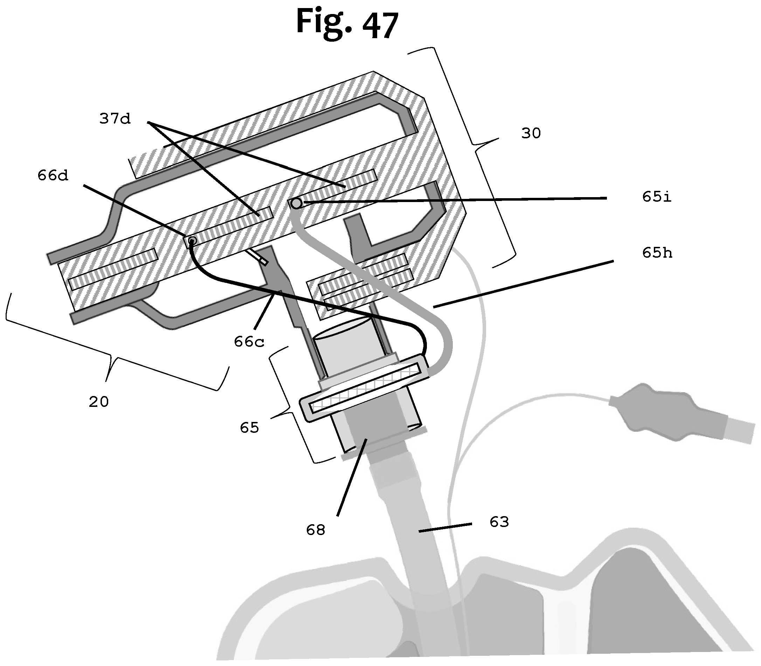

Fig. 47 shows a DAU installed on a convertible ventilator circuit patient manifold, whereby an HME is installed in the convertible ventilator circuit patient manifold airway adjunct receptacle, with an airway adjunct installed in the HME patient airway adjunct receptacle, with the said airway adjunct comprising and endotracheal tube. Fig. 47 further shows an HME pressure tube connected to an auxiliary sensor that is a pressure sensor on said DAU. Fig. 47 further shows an HME temperature and/or humidification probe that is connected to an auxiliary sensor that is a temperature and/or humidification sensor.

### DETAILED DESCRIPTION OF PREFERRED VERSIONS OF THE INVENTION

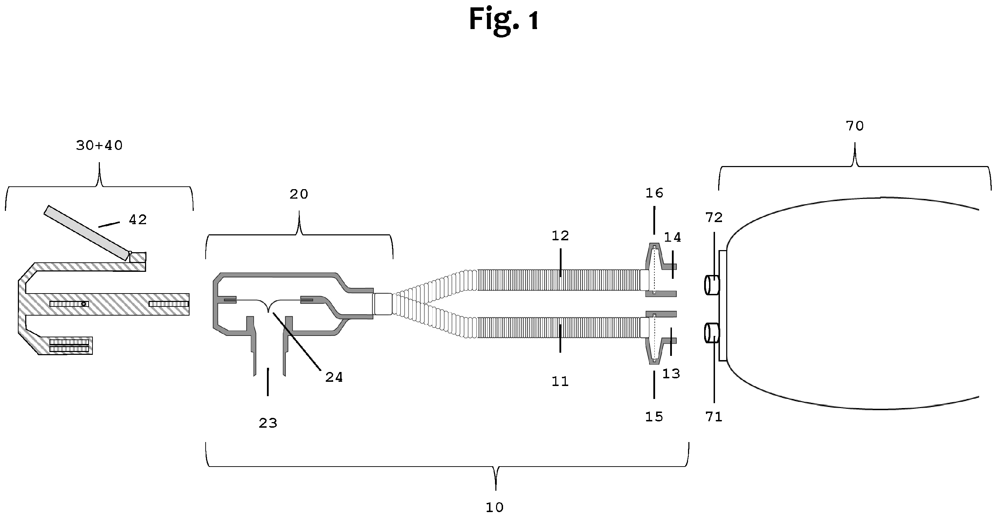

Fig. 1 illustrates a combination comprising a convertible ventilator circuit **10** having an inspiratory limb **11**, with said inspiratory limb having an inspiratory particle, poison and pathogen filter **15** and ventilator inspiratory connection **13**. The convertible ventilator circuit **10** also comprises an expiratory limb **12** having an expiratory pathogen filter **16** and ventilator expiratory connection **14**. The convertible ventilator circuit **10** also comprises a patient manifold **20** that has fluid connections with the inspiratory limb **11** and expiratory limb **12**, and also has an airway adjunct receptacle **23** that provides a fluid connection to an airway adjunct that interfaces with the patient. A preferred embodiment for an inspiratory limb non-return valve **24** is positioned to alternate fluid connectivity between either: (1) the inspiratory limb **11** and airway adjunct receptacle **23**; or (2) the expiratory limb **12** and airway adjunct receptacle **23**. This configuration for an inspiratory limb non-return valve **24** results in materially uniform direction of gas flow in the inspiratory limb **11** and expiratory limb **12**. Specifically, during the inspiration phase of a singular breath, inspiratory gas expelled by a ventilator **70** connected to the ventilator inspiratory connection **13** will travel through the inspiratory particle, poison and pathogen filter **15**, then continue through the inspiratory limb **11** toward the patient manifold **20**, through the patent inspiratory limb non-return valve **24** and out the airway adjunct receptacle **23**. During the expiratory phase of a singular breath, expiratory gas expelled by the patient via the airway adjunct connected to the airway adjunct receptacle **23** will be prevented from entering the inspiratory limb **11** by the closed inspiratory limb non-return valve **24**, and will therefore enter the expiratory limb **12**, travel through the expiratory pathogen filter **16** toward the ventilator **70** connected to the ventilator expiratory connection **14**.

The inspiratory particle, poison and pathogen filter serves a critical role in protecting patients. The inspiratory particle, poison and pathogen filter **15** ensures that inspiratory gas to be inhaled by the patient is free of pathogens that could cause infection, and/or particles that could contribute to pathologic injury. Public safety and/or military versions can include ability to filter poisonous inhalation hazards if use is anticipated for hazardous surroundings to protect from chemical inhalation.

Similarly, the expiratory pathogen filter **16** protects providers and other proximate persons in the event the patient being treated has a communicable airborne infection, such that any airborne pathogens exhaled by the infected patient are prevented from entering the ambient atmosphere. Notably, the position of these filters is in close proximity to the connections to a ventilator, enabling filtration function to be preserved during conversions between manual and mechanical ventilation.

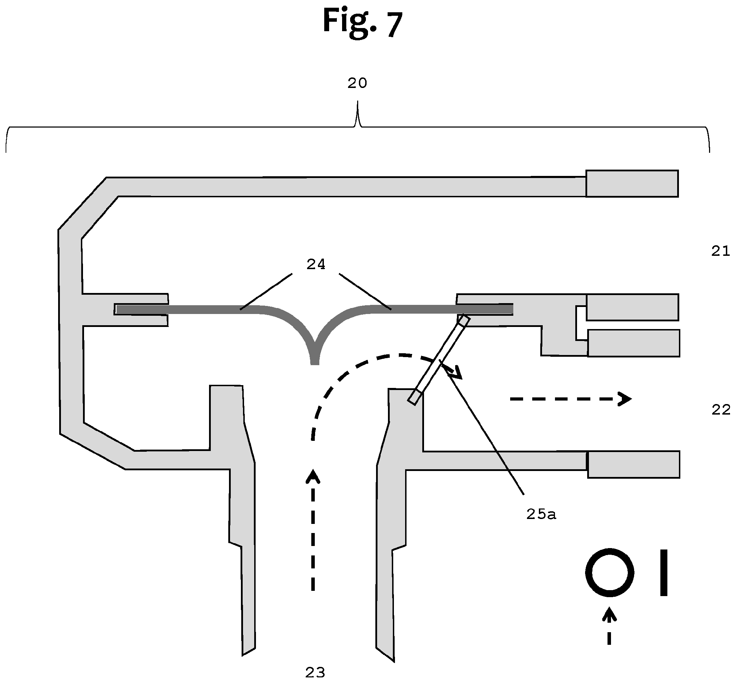

Components of the convertible ventilator circuit **10**, to include the patient manifold **20**, inspiratory limb **11** and expiratory limb **12**, with corresponding inspiratory particle, poison and pathogen filter **15** and expiratory particle pathogen filter **16**, are preferably manufactured of biocompatible materials and free of harmful leachable compounds that could diffuse into respiratory gas transported within. Additionally, the inspiratory limb **11**, expiratory limb **12**, and patient manifold **20** are preferably comprised of a material that is resistant to expansion when containing pressurized respiratory gases. The inspiratory limb **11** and expiratory limb **12** are also preferably comprised of a corrugated design (or materially equivalent substitute) that facilitates ability to lengthen, compress and/or shape into variable configurations that enable the convertible ventilator circuit to be inherently suitable for use with various embodiments of artificial ventilators and/or use scenarios. This particularly provides for the ability for the inspiratory limb **11** and expiratory limb **12** to be compressed when connected to a manual ventilator **70** (as shown), which when in use is typically positioned close to the patient for ideal use, and for the inspiratory limb **11** and expiratory limb **12** to alternatively be in a stretched or partially stretched configuration when connected to a mechanical ventilator (shown in Fig. 7), which when in use is typically positioned 1-2 meters from the patient for ideal use. In this depiction shown in Fig. 1, the inspiratory limb **11** and expiratory limb **12** are depicted in this side view as being vertically aligned on the same vertical plane (i.e., the expiratory limb **12** is depicted in Fig. 1 above the inspiratory limb **11** enabling both to be visible simultaneously). It can be further seen that in this exemplar version of the invention, a combined DAU+DPU **30+40** comprises a single integrated unit with the DAU and the DPU sharing a singular housing. This results in a display and audible emitter **42** being located in proximity to the patient manifold **20**. The combined DAU+DPU **30+40** in this depiction is in a ready position to be installed on the patient manifold **20**.

It should be understood the DPU **40** includes components typical to small devices capable of processing, analyzing storing and/or transmitting data, with such components to include a computer processing unit and/or logic circuit, memory, wired/wireless communication, and interfaces for other interfacing components. In the present invention, the DPU also includes a triggering capability constituting an active exhalation valve controller capable of activating an interfaced exhalation valve. The DPU also contains one or more controllers for visual, audible and/or haptic feedback to be conveyed to a user, power storage capability (e.g., battery) and other components that will be subsequently described in additional detail. Finally, both the DAU and the DPU contain one or more motion sensors enabling detection of possible movement artifacts, with exemplar sensor types used for this purpose being a triaxial accelerometer and/or a gyroscopic sensor.

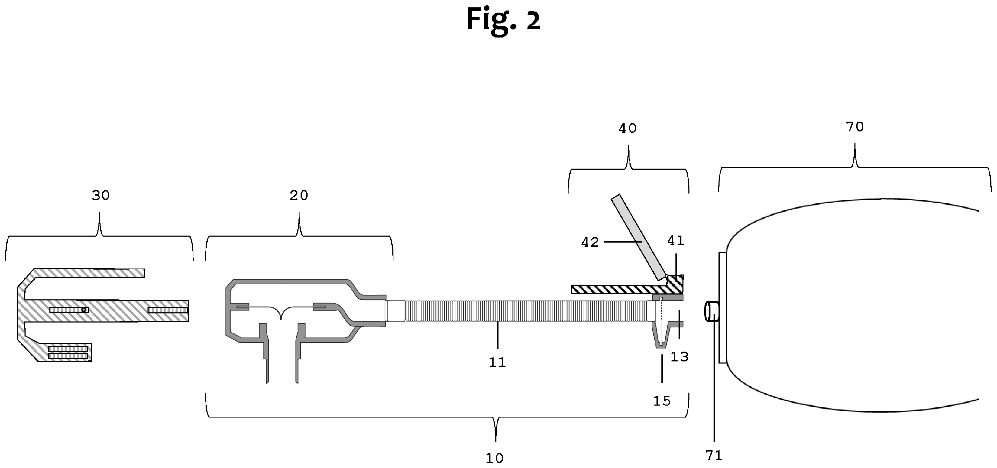

Fig. 2 illustrates a side view of the same combination of components shown in Fig. 1, except some attributes of the invention are shown in preferred configurations. Inspiratory limb **11** and expiratory limb (not visible) are depicted as being on the same horizontal plane, whereby the expiratory limb in this view is obscured behind the inspiratory limb **11** in the foreground. It can be further seen that, in this preferred embodiment of the invention, the DAU **30** is provided its own separate housing from the DPU **40**. In this preferred configuration, it results in the display and audible emitter **42** being located in proximity to the manual ventilator **70** and able to be physically supported by the connected manual ventilator **70** during use, thereby causing the weight of the DAU **30** to be materially less than the weight of the integrated DAU+DPU as previously shown in Fig. 1.

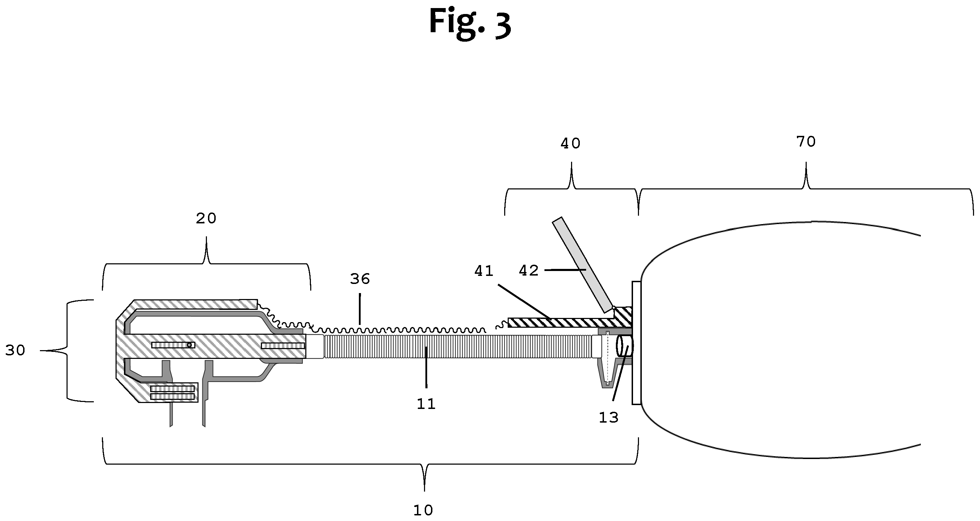

Fig. 3 shows the same components of Fig. 2, except the DAU **30** and DPU **40** are both installed on the convertible ventilator circuit **10**, thereby resulting in a data-integrated convertible ventilator circuit (DiCVC). Similarly, the manual ventilator **70** is shown connected to the DiCVC **10**, completing a system for obtaining data during manual ventilation. Also shown is a wired connection **36** between the DAU **30** and DPU **40**, which can be of a general type (e.g., universal serial bus) capable of preferably rapid bidirectional data exchange.

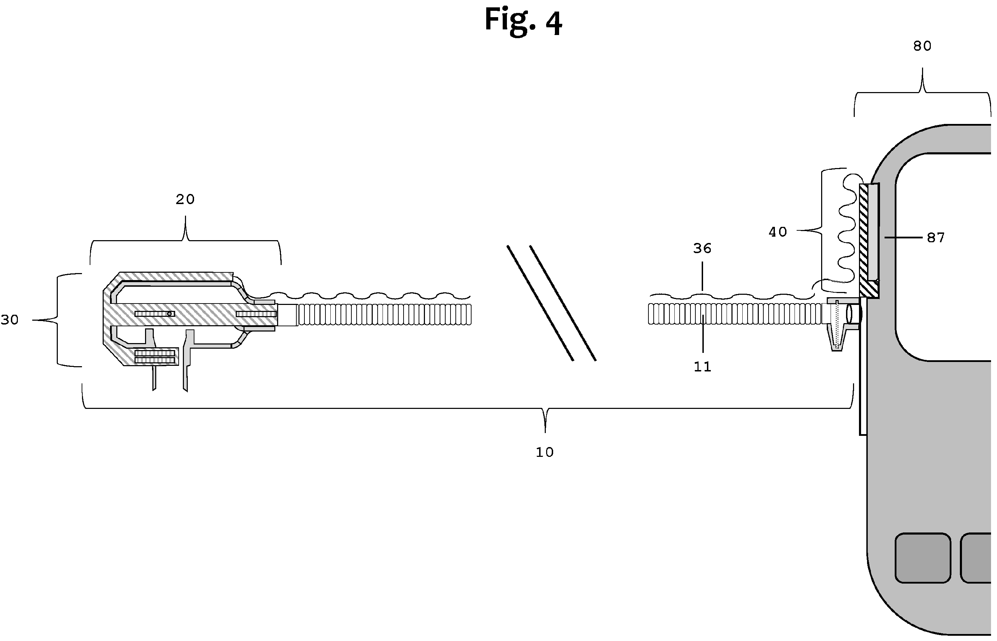

Fig. 4 illustrates the same combination of components shown in Fig. 3, except the manual ventilator has been replaced with a mechanical ventilator **80**. As opposed to the configurations depicted in Fig. 3, it can be seen in this Fig. 4 that the inspiratory limb **11** is in an expanded configuration that is preferable during use with a mechanical ventilator **80**. In a preferred embodiment of mechanical ventilator **80**, it can be seen that the DPU **40** is able to be positioned into an interface receptacle **87** specifically designed to accommodate the DPU **40**, thereby enabling the DPU **40** to be substantially shielded from inadvertent contact and/or displacement. A wired connection **36** enables data exchange between the DAU **30** and DPU **40**, with an alternative embodiment comprising a wireless means for materially similar data exchange. A preferred embodiment of mechanical ventilator **80** will include ability for the DPU to exchange data, either through a similar wired connection, wireless means (e.g., Bluetooth and/or WiFi protocols), or other means, such that the mechanical ventilator, having its own processor unit and/or logic circuit, may be able to utilize data acquired by the DAU **30** and, similarly, exchange data with the DPU **40**. A preferred embodiment of wired connection **39** between the DAU **30** and DPU **40** provides for the wired connection **39** to be embedded in or closely affixed to either the inspiratory limb **11** or expiratory limb (not visible).

It should be understood that the versions of the invention as depicted in Figs. 1-4 are meant to be merely exemplary and that the invention is not limited to the components shown. For example, it is common in the field for one or more accessories to be able to be inserted into one or more of the circuit limbs, such as an accessory for delivering aerosolized and/or nebulized medications, for heated humidification, and/or other purposes. It should be understood that such accessories shall not materially affect the scope of the present invention. Additionally, it is to be understood that alternative embodiments are possible that are within the scope of the present invention. For example, referring to Fig. 1, instead of a separate inspiratory limb **11** and expiratory limb **12**, a single dual-lumen tube could be provided comprising a single tube that maintains separate passageways for inspiratory and expiratory gases. Such dual-lumen tube could comprise a side-by-side approach or a tube-within-tube approach, with simple modifications made to the connections to a convertible ventilator circuit patient manifold **20** and ventilator inspiratory connection **13** and ventilator expiratory connection **14** to accommodate the alternative configuration.

The components of the convertible ventilator circuit **10** and convertible ventilator circuit patient manifold **20**, to include all 10 series and 20 series numerals, are intended to be economically manufactured for single patient use and facilitating disposability after use. A preferred embodiment provides for these components to have enhanced biodegradation and/or suitability for incineration to facilitate environmentally sound disposal. Alternatively, the named components could be manufactured with durable materials suitable for economic sterilization between use on various patients. In contrast, components with 30 and 40 series numerals are intended to be constructed of durable materials such that, after uninstalling from a convertible ventilator circuit **10** after use on a patient, allow the durable components to be easily removed, cleaned, and installed on a subsequent convertible ventilator circuit **10** prior to use on a following patient.

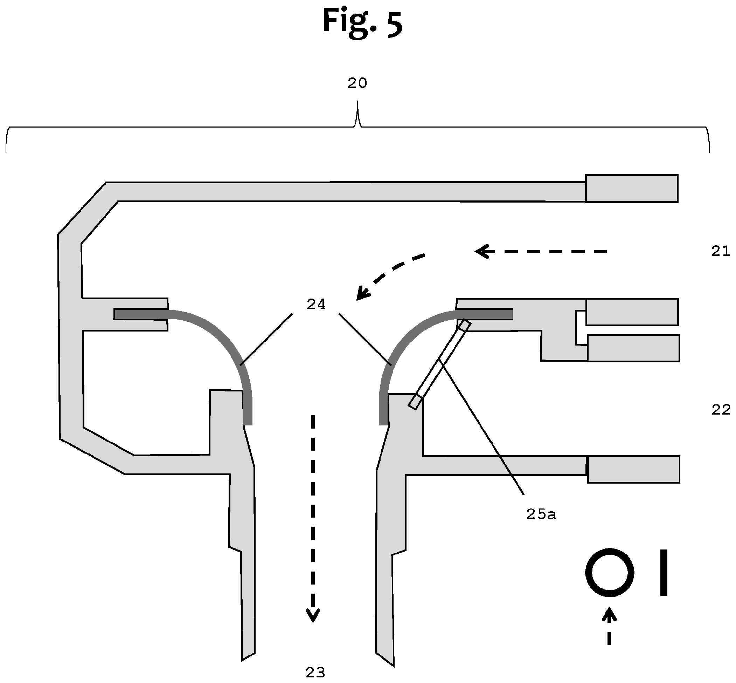

Fig. 5 illustrates the convertible ventilator circuit patient manifold **20** whereby a connection for an inspiratory limb **21** is depicted above a connection for an expiratory limb **22**. A patient airway adjunct receptacle **23** is also provided which is designed to interface with one of several types of patient airway adjunct that will be in physical contact with the patient. Examples of airway adjuncts include a face mask (whereby an air-tight seal is obtained between the patient's face and interfacing surface of the face mask), a supraglottic airway that inserts into a patient's throat (whereby an air-tight seal is obtained between the surfaces of the patient's throat and interfacing surface of the supraglottic airway), an endotracheal tube that inserts into a patient's trachea (whereby an air-tight seal is obtained between the surface of the patient's trachea and an interfacing surface on an inflatable cuff on the endotracheal tube), or, in patients that are anticipated to undergo long-term artificial ventilation, a tracheal tube that inserts into the patient's trachea through a surgical opening in the neck.

Fig. 5 also illustrates a preferred embodiment of an inspiratory limb non-return valve **24** in a configuration consistent with the inspiratory phase of a singular breath, whereby the exemplar non-return valve **24** provides two functions. First, the inspiratory limb non-return valve **24** passively opens when inspiratory gases from a ventilator flow into the convertible circuit patient manifold **20** by means of the inspiratory limb connection **21**. This connectivity provides a continuous fluid passageway from said inspiratory limb connection **21** to the connection for a patient airway adjunct **23**, thereby providing a means for inspiratory gas to be directed through the convertible ventilator circuit patient manifold **20** to the patient. Second, it can be seen that in the exemplar version shown, the non-return valve **24** is configured to simultaneously obstruct fluid passageway between the expiratory limb connection **22** and either: (1) the inspiratory limb connection **21**; or (2) the patient airway adjunct receptacle **23**. This configuration ensures that, during an inspiratory phase of a singular breath, gas flow is substantially unidirectional from the ventilator via the inspiratory limb connection **21**, through the open non-return valve **24**, and toward the patient airway adjunct receptacle **23**, thereby achieving the means for inspiratory gas to have a singular route through the convertible ventilator circuit patient manifold **20** from a ventilator to the patient. More specifically, the configuration of the non-return value is such that inspiratory gas cannot bypass the patient by directly flowing between the inspiratory limb connection **21** and the expiratory limb connection **22**.

Several alternative embodiments of the components as discussed are consistent with the scope of the present invention. For example, a non-return valve **24** could be substituted by a pair of active one-way valves each controlling the patency of flow from an inspiratory limb connection **21** and an expiratory limb connection **22,** which would provide an identical unidirectional flow pattern from the inspiratory limb connection **21** and the patient airway adjunct receptacle **23**, with the said one-way valve governing flow via the inspiratory limb connection **21** being open and the said one-way valve governing flow via the expiratory limb connection **22** being closed. Similarly, while the non-return valve **24** as shown is of a substantially duckbill design, it should be understood that the invention also includes alternatives to include dome, umbrella and/or cross-slit valve approaches that have materially applicable utility in the application as described.

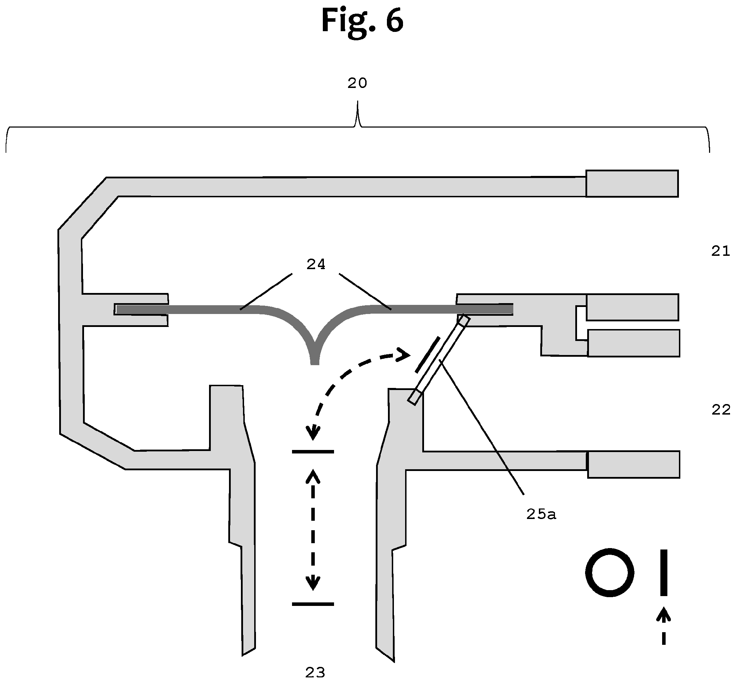

Fig. 6 illustrates a preferred embodiment of an inspiratory limb non-return valve **24** in a configuration consistent with an inspiratory hold phase of a singular breath, whereby after complete delivery of a breath, the volume of gas delivered to the lungs is held for a brief period of time between an inspiratory (or lung inflating) phase and expiratory (or lung deflating) phase. In this configuration, the exemplar non-return valve **24** is closed, obstructing retrograde gas flow toward the inspiratory limb connection **21** from either the patient airway adjunct receptacle **23** or expiratory limb connection **22**. In addition, in this preferred embodiment, an active electrostatic actuating expiratory limb valve **25a** is activated and forced closed, preventing gas flow from the patient airway adjunct receptacle **23** toward the expiratory limb connection **22**. This electrostatic actuating expiratory limb valve **25a** is actuated via an electrical circuit that communicates with the external surface of the patient manifold **20** such that, when a DAU is installed on the patient manifold **20**, an electrical connection is provided (not visible) enabling the DAU to electrically activate the electrostatic actuating expiratory limb valve **25.** As a result, in the configuration shown, no gas flow is permitted between any of the named components, thereby constituting the necessary apparatus to achieve an inspiratory hold maneuver regardless of whether the convertible ventilator circuit is connected to either a manual or mechanical ventilator.

As previously explained and shown in Fig. 5, alternative embodiments of the named components as discussed are to be understood as within the scope of the present invention which would provide an identical ability to achieve an inspiratory hold maneuver. Alternative actuation mechanisms of an actuating expiratory limb valve **25a** include direct mechanical actuation, pneumatic actuation, and/or alternative electrical actuation via electromagnetic or piezoelectric actuation.

Fig. 7 illustrates a preferred embodiment of an inspiratory limb non-return valve **24** in a configuration consistent with an expiratory phase of a singular breath. As shown in Fig. 6, the exemplar non-return valve **24** is closed, obstructing retrograde gas flow toward the inspiratory limb connection **21** from either the patient airway adjunct receptacle **23** or expiratory limb connection **22**. In addition, in this embodiment the active electrostatic actuating expiratory limb valve **25a** is deactivated, enabling it to passively re-open and enable gas flow from the patient airway adjunct receptacle **23** toward the expiratory limb connection **22**, thereby achieving the means for an exhalation phase of a breath cycle whereby expired gas from the patient enters the convertible ventilator circuit patient manifold **20** through the patient airway adjunct receptacle **23**, through the deactivated and patent electrostatic actuating expiratory limb valve **25a** and toward the ventilator via the expiratory limb connection **22**.

As previously shown, alternative embodiments of the components as discussed are consistent within the scope of the present invention which would provide an identical ability to achieve lung expiration. Alternative actuation mechanisms of an actuating expiratory limb valve **25a** could be identically configured to provide for free gas flow when the active expiratory valve **25a** is deactivated.

While the embodiment in Figs. 5-7 has been described with both an inspiratory and expiratory limb as shown in Fig. 1 (**11** and **12**, respectively), it should also be understood that functionality as described will be fully preserved without an actual expiratory limb **12** present and connected to the expiratory limb connection **22**. In this case, exhaled gases from the patient will not flow back to a ventilator connected to the ventilator expiratory limb connector **14**, but will instead simply vent into the ambient atmosphere from the expiratory limb connection **22** as shown in Fig. 1. An airborne pathogen filter could be positioned near the expiratory limb connection **22** so that, when no expiratory limb is connected, expiratory gases from the patient will be free of contaminants that could infect providers and/or neighboring patients. Accordingly, referring to Fig. 1, an alternative embodiment of the present invention includes a single-limb design that excludes an expiratory limb **12**. Further, the present invention also provides for an embodiment allowing an expiratory limb **12** to be added only when deemed desirable by an operator (e.g., prior to use with a mechanical ventilator). This configuration may provide certain advantages similar to a single, dual-lumen convertible ventilator circuit as previously described.

Referring again to Figs. 5-7, while the devices described support active gas movement propelled by a ventilator, it should also be understood that the components as provided will also function passively in the event the patient regains partial or full consciousness. Under these conditions, in the event a patient spontaneously initiates a breath, thereby creating a vacuum at the point of the patient airway adjunct receptacle **23**, the non-return valve **24** will passively open in response to gas flow being drawn from the inspiratory limb connection **21**.

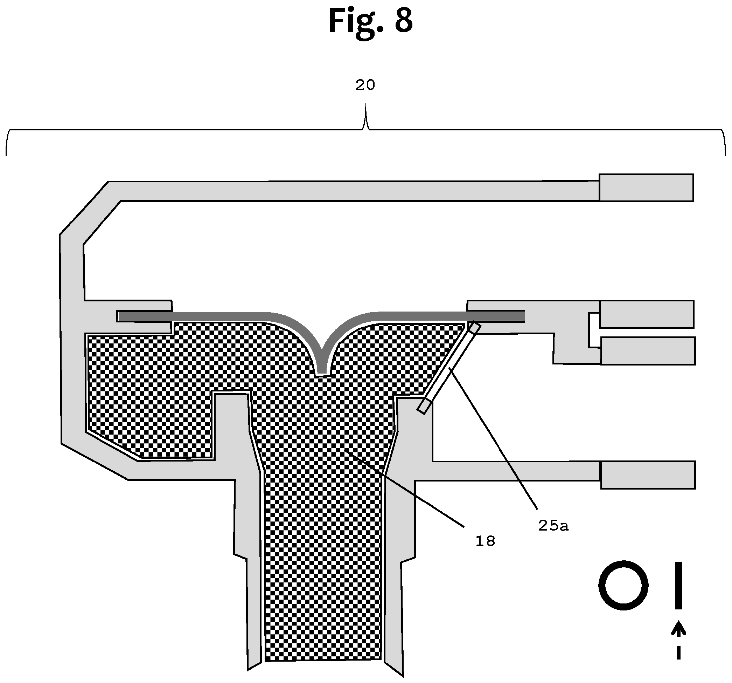

Fig. 8 illustrates the deadspace area **18** within the convertible ventilator circuit patient manifold **20**. This is an important variable for purposes of obtaining certain measurements on gas flow, gas pressure, and/or partial pressure/admixture of constituent gases. In particular, during a inspiratory hold maneuver where the active exhalation valve **25a** is actuated, creating zero gas flow, an accurate pressure measurement can be obtained without confounding pressure resulting from resistance to gas flow.

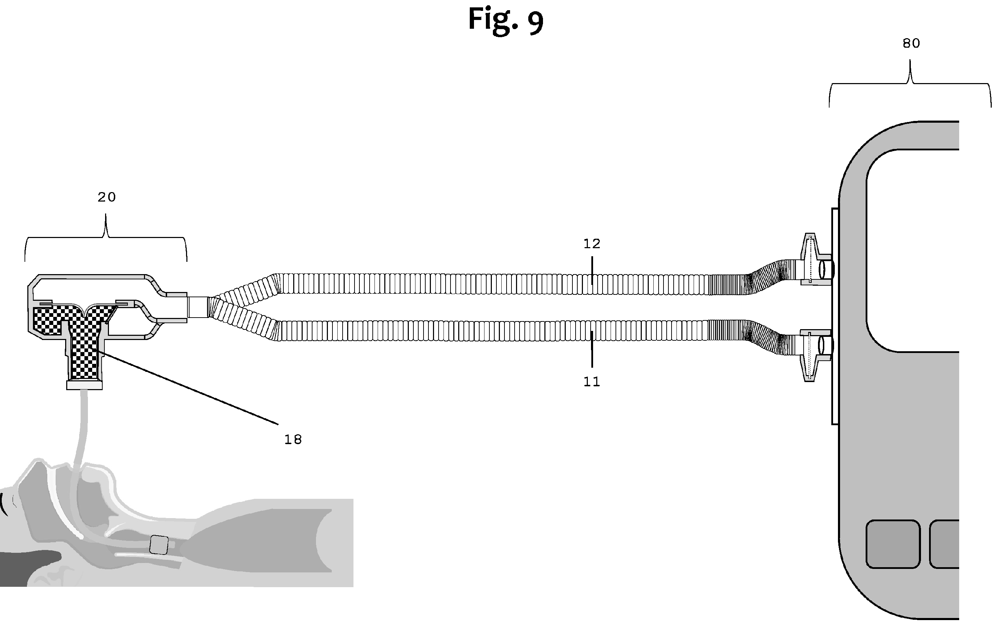

Fig. 9 illustrates the same components of Fig. 8 in an expanded view where the convertible ventilator circuit **10** is connected to a mechanical ventilator **80**. This view enables subjective comparison of total deadspace area **18** relative to the total volume of gas in the inspiratory limb **11** and expiratory limb **12**.

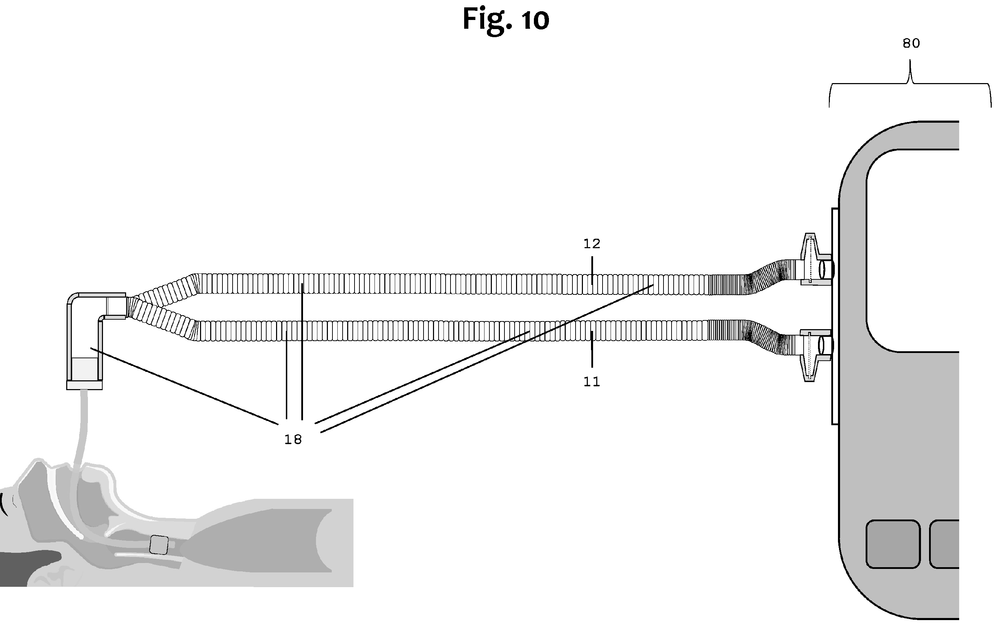

Fig. 10 illustrates comparative deadspace area **18** in a non-convertible ventilator circuit that is solely compatible with a mechanical ventilator **80**. Such non-convertible ventilator circuits often contain no one-way valves, with unidirectional air flow throughout both inspiratory and expiratory limbs controlled by internal one-way valves contained within the ventilator itself. In this configuration, a pressure measurement obtained at any point within the circuit is an aggregate measurement of the lungs together with the entirety of the deadspace area **18** contained in both limbs of the non-convertible ventilator circuit. In this configuration, the deadspace area **18** far exceeds the actual lung volume of the patient.

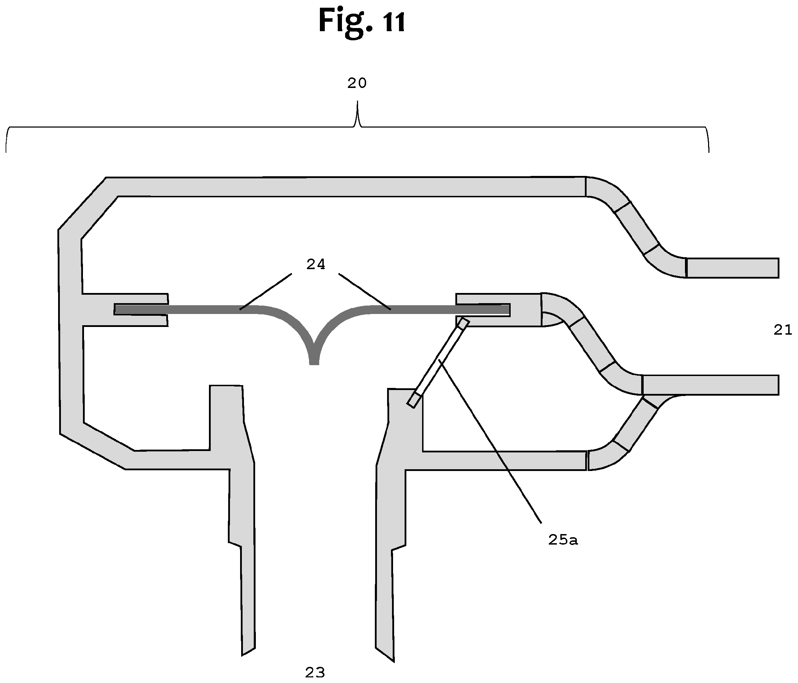

Fig. 11 illustrates a preferred embodiment of convertible ventilator circuit patient manifold **20** that provides for a horizontally homogenous (left-right) configuration of the inspiratory limb connection **21** and expiratory limb connector (in background behind inspiratory limb connection **21** in this view), with other named components remaining identical as shown in Fig. 7.

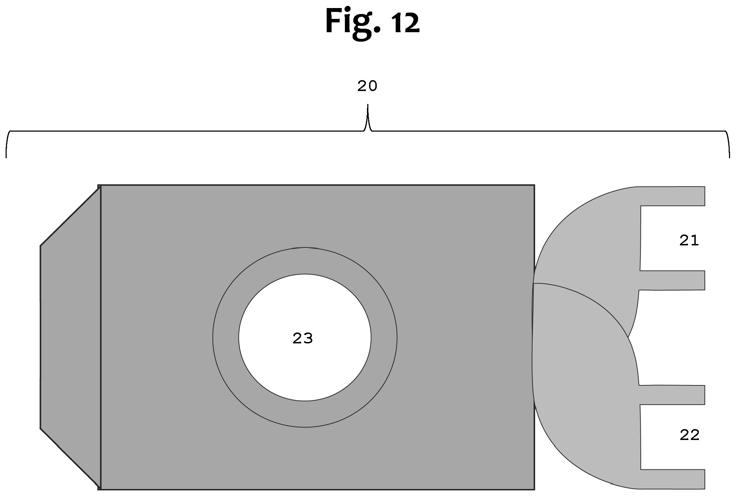

Fig. 12 illustrates the same preferred embodiment of convertible ventilator circuit patient manifold **20** as shown in Fig. 11 from a bottom-up perspective. In this view the horizontally homogenous configuration of the inspiratory limb connection **21** and expiratory limb connection **22** can be seen relative to the location of the patient airway adjunct receptacle **23**.

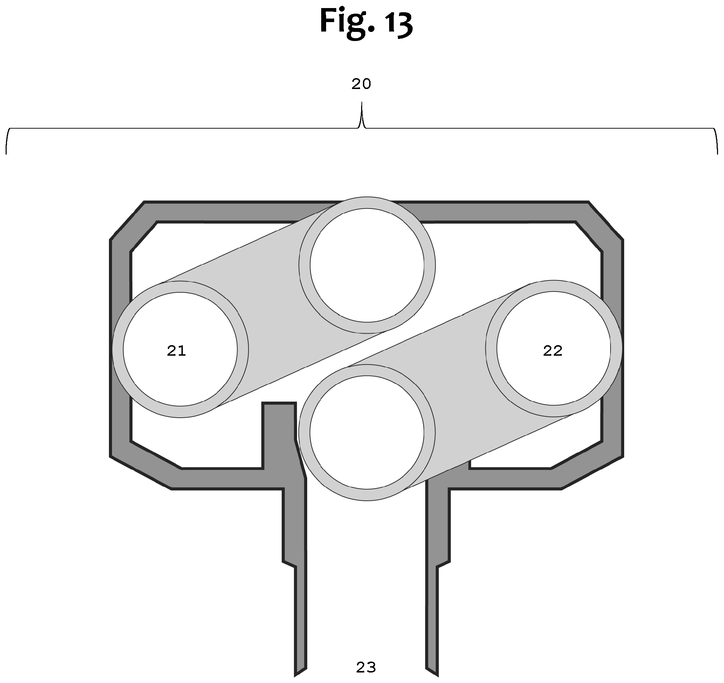

Fig. 13 illustrates the same preferred embodiment of convertible ventilator circuit patient manifold as shown in Figs. 11-12 from an end-on perspective. In this view the inspiratory limb connection **21** can be seen to be on the left, then directing the gas passageway upwards and to the right. Similarly, the expiratory limb connection **22** can be seen to be on the right, receiving gas flow from the lower left. This perspective effectively demonstrates how an upper-lower (vertically homogenous) configuration of inspiratory/expiratory gas flow can convert to a left-right (horizontally homogenous) configuration.

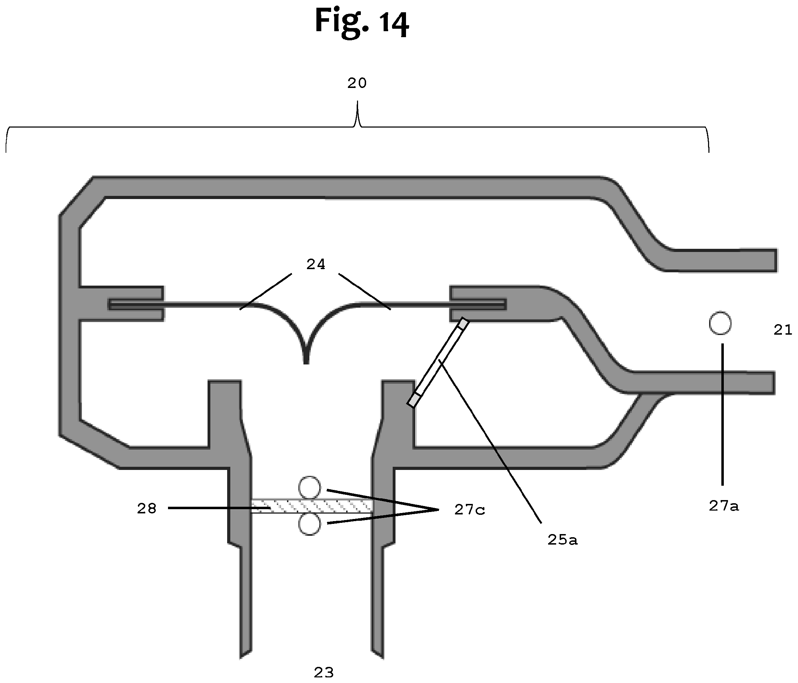

Fig. 14 illustrates the preferred embodiment of convertible ventilator circuit patient manifold **20** of Fig. 11, also depicting preferred locations for one or more sensor ports **27a** and **27c** that provide a means for one or more sensor(s) to gain access to the gas passageway beneath. These sensor ports can be capped or plugged to prevent gas leakage when vacant with no sensor present in the port. A preferred embodiment for sensor ports **27a** and **27c** is for a passive, multi-cuspid, cross-slit valve design similar to a human heart valve where the pressure inside the circuit will press the valve closed to prevent leakage when no sensor is present, but will deflect under pressure upon the tip of a sensor pushing on the exterior surface of the valve, such that proper positioning of a sensor will cause sensor ports to open and permit communicable access of a sensor tip into the gas passageway. It should be understood that common alternative forms of one-way valves are to be construed to be provided by the present invention. For example, duckbill, ball, umbrella, and other common one-way valve designs would function in the role described in the present invention.

It can also be seen in Fig. 14 that, depending on the location of sensor ports **27a** and **27c** on the convertible ventilator circuit patient manifold **20**, sensors can be configured to communicate with specific areas of the convertible ventilator circuit patient manifold **20** in order to provide sensor information specific to one or more phases of a breath cycle. For example, the inspiratory limb non-return valve **24** would be between a pressure sensor installed in the sensor port **27a** located close to the inspiratory limb connection **21** and the patient airway adjunct receptacle **23**. During active inspiration when the inspiratory limb non-return valve **24** is open (as depicted in Fig. 5), the pressure sensor installed in the sensor port **27a** as previously described will be in contact with gas that has a continuous fluid passageway to the patient airway adjunct receptacle **23,** and thus the lungs of the patient. This will mean the pressure measured at sensor port **27a** will be reflective of the aggregate of (1) pressure in the lungs of the patient and (2) additional pressure generated as a result of airway resistance against active gas flow. However, during an inspiratory hold phase or exhalation phase, whereby the inspiratory limb non-return valve **24** is closed in the configuration shown in Fig. 14, the pressure sensor installed in the sensor port **27a** as previously described will not reflect pressure in the patient airway adjunct receptacle **23,** and thus be isolated from the pressure in the lungs of the patient. This is due to the closed configuration of the inspiratory limb non-return valve **24** which acts to interrupt the gas passageway between the pressure sensor installed in the sensor port **27a** as previously described and the patient airway adjunct receptacle **23**. This means a pressure sensor installed in the sensor port **27a** will be free of confounding pressure sources other than the desired pressure measurement from a ventilator connected to the inspiratory limb connection **21**. Similar benefit of a pressure sensor located in a sensor port close to the expiratory limb connection (not visible) will be free of confounding pressure sources apart from the desired pressure measurement during active exhalation.

In contrast to sensors located in a sensor port close to either the inspiratory or expiratory limb connections, sensors located in one or more sensor ports **27c** close to the patient airway adjunct receptacle **23** will be in contact with gas having a continuous fluid passageway with the patient lungs at all times throughout all breath cycles. For example, an oxygen sensor installed in a sensor port **27c** as previously described will be capable of measuring the concentration of oxygen being administered to the patient during inspiration, and also be capable of measuring the concentration of oxygen being exhaled by the patient during and at the end of expiration. This can be highly diagnostic of oxygen uptake, whereby the difference between oxygen concentration during inspiration and expiration is proportional to the amount of oxygen that was absorbed by the blood (which can inform on diagnostic factors including lung function, pulmonary blood flow, and hemoglobin status). Similarly, a carbon dioxide sensor installed in a sensor port **27c** as previously described will be capable of measuring the concentration of carbon dioxide being administered to the patient during inspiration (which should be materially zero) while measuring the concentration of carbon dioxide exhaled by the patient at the end of expiration. Exhaled carbon dioxide concentration can also inform on diagnostic factors including lung function, pulmonary blood flow, and other factors. Finally, a pressure sensor installed in a sensor port **27c** as previously described will be capable of measuring pressure during inspiration, an inspiratory hold maneuver, and expiration.

Fig. 14 depicts a preferred embodiment where multiple sensor ports **27c** located close to the patient airway adjunct receptacle **23** are provided, implying a total of four sensor ports **27c** (top, bottom, foreground & background) as depicted in this location.

Fig. 14 also illustrates a preferred location for a flow sensor screen **28** capable of measuring gas flow in either direction during all breath phases. A preferred embodiment of a pressure differential pneumotach is depicted in the drawing, which also provides for a known area in proximity of the flow sensor screen **28** such that volume of gas passing through the sensor over a given unit of time can be mathematically determined. It is to be understood that the present invention includes other types of common flow sensors that could provide materially same functionality as described.

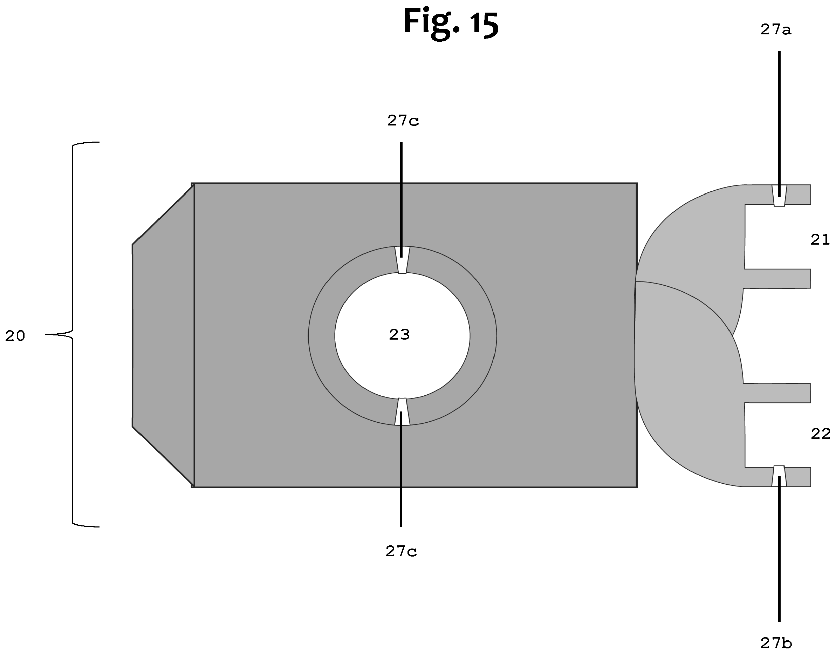

Fig. 15 illustrates the preferred embodiment of convertible ventilator circuit patient manifold **20** of Fig. 12, also depicting preferred locations for sensor ports **27a**, **27b**, and **27c** configured to communicate with specific areas of the convertible ventilator circuit patient manifold **20**. In this bottom-up view, respective locations can be seen for sensor port **27a** close to the inspiratory limb connection **21,** and sensor port **27b** close to the expiratory limb connection **22**. As previously described, this enables a sensor (e.g., a pressure sensor) installed in sensor port **27a** to provide "clean" non-confounded data specific to inspiration, as well as a sensor installed in port **27b** to provide similar non-confounded data specific to expiration. Also shown are preferred locations for sensor ports **27c** in close proximity to the patient airway adjunct receptacle **23**.

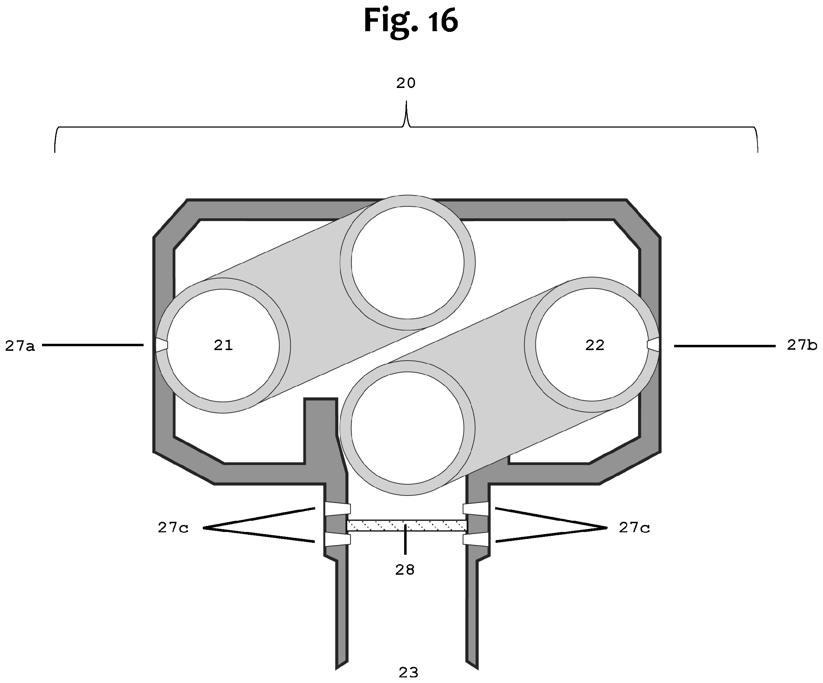

Fig. 16 illustrates the preferred embodiment of convertible ventilator circuit patient manifold **20** of Fig. 13, also depicting preferred locations for sensor ports **27a**, **27b**, and **27c** configured to communicate with specific areas of the convertible ventilator circuit patient manifold **20**. This perspective most clearly shows how sensor ports **27a** and **27b** can be respectively positioned in close proximity to the inspiratory limb connection **21** and expiratory limb connection **22**. Similarly, this perspective most clearly shows how four exemplar sensor ports **27c** can be preferably positioned in close proximity to the patient airway adjunct receptacle **23**.

The end-on perspective provided in this Fig. 16 also most clearly illustrates how all sensor ports **27a, 27b** and **27c** are, in this preferred embodiment, accessible from the left and right sides of the convertible ventilator circuit patient manifold **20**, making them easily accessible to installation of the DAU and associated sensors.

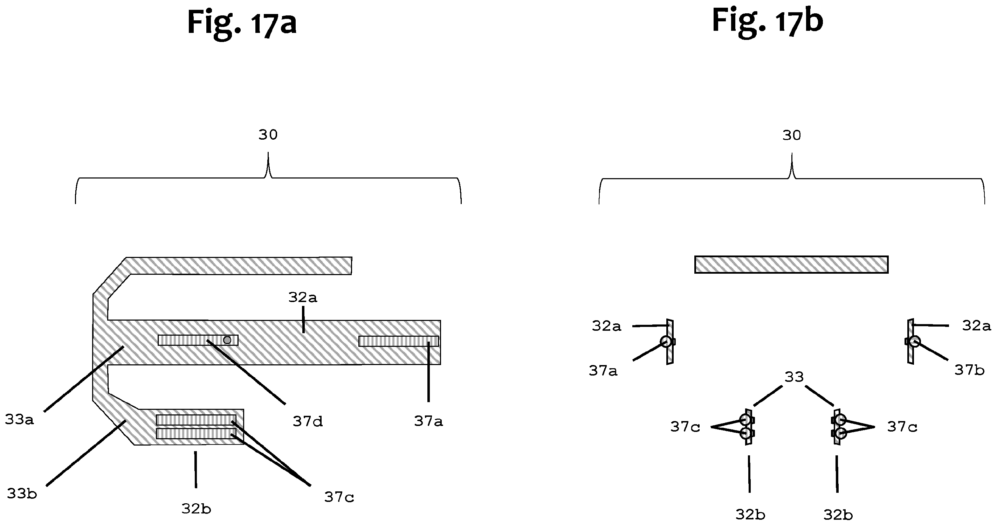

Fig. 17a illustrates a side view of an embodiment of DAU **30**, with Fig. 17b providing a corresponding end-on view of an embodiment of DAU **30**. In Fig. 17a a single sensor housing upper arm **32a** in the foreground can be seen. Similarly, a single sensor housing lower arm **32b** in the foreground can be seen. In Fig. 17b, both sensor housing upper arms **32a** are visible and preferably oriented on the same horizontal plane, with both sensor housing lower arms **32b** similarly visible and oriented. Fewer, or additional, sensor housing arms could be provided in alternative embodiments while remaining within the scope of the present invention.

A preferred embodiment of sensor housing upper arms **32a** contain a sensor housing upper arm neck **33a** which comprises either an active or passive means for temporary alteration in the angle of the sensor housing upper arms **32a**, such that left and right sensor housing upper arms **32a** can be widened apart during installation on the convertible ventilator circuit patient manifold. Similarly, sensor housing lower arms **32b** contain a sensor housing lower arm neck **33b** which comprises either an active or passive means for temporary alteration in the angle of the sensor housing lower arms **32b** to facilitate installation with the convertible ventilator circuit patient manifold. A preferred embodiment of sensor housing upper arms **32a** and/or sensor housing lower arms **32b** comprise an alteration in the characteristics of the material used to comprise the upper arm housing necks **33a** and/or lower arm housing necks **33b**, such that a memory feature causes the upper arm housing necks **33a** and/or lower arm housing necks **33b** to accommodate an altered, widened angle under manual force during installation of the DAU **30** on to a convertible ventilator circuit patient manifold, but then revert to the original angle upon full advancement of the sensor housing upper arms **32a** and sensor housing lower arms **32b** over the convertible ventilator circuit patient manifold. In this preferred embodiment, the mating surfaces of the DAU and convertible ventilator circuit patient manifold are configured to provide one or more features that allow the two components to be reversibly secured together once installation is complete, thereby providing predictable and consistent relative positioning between sensors on the DAU and corresponding sensor ports that are positioned on the convertible ventilator circuit patient manifold. After use, the DAU **30** can be released from the convertible ventilator circuit patient manifold by applying manual outward pressure on the sensor housing upper arms **32a** and sensor housing lower arms **32b**, such that the DAU **30** is able to be slid off and uninstalled from the convertible ventilator circuit patient manifold.

Continuing to refer to Figs. 17a and 17b, sensors can be installed on the DAU **30** in various position to obtain data from specific positions representing a specific stage of singular breath cycles. One or more inspiratory zone sensor(s) **37a** can be positioned to align with one or more corresponding sensor port(s) in the convertible ventilator circuit patient manifold allowing said inspiratory zone sensor(s) **37a** to directly interface with inspiratory gases. One or more expiratory zone sensor(s) **37b** can be positioned to align with one or more corresponding sensor port(s) in the convertible ventilator circuit patient manifold allowing said expiratory zone sensor(s) **37**b to directly interface with expiratory gases. One or more patient zone sensor(s) **37c** can be positioned to align with one or more corresponding sensor port(s) in the convertible ventilator circuit patient manifold allowing said patient zone sensor(s) **37c** to directly interface with both inspiratory and expiratory gases. One or more auxiliary sensors **37d** can be positioned on the convertible ventilator circuit patient manifold with necessary connections to provide connectivity with one or more accessories that may include a component of an airway adjunct, a medication nebulizer, an inspiratory gas humidifier, or other accessory to be utilized during use of the convertible ventilator circuit.

The present invention includes a preferred embodiment where sensors can be fitted as needed based on intended need and anticipated level of care expected to be provided, with sensor(s) including pressure measurements and/or measurements of partial pressure/admixture of constituent gases. For example, it is possible for a DAU to initially be fitted with a single pressure sensor and single sensor for measuring carbon dioxide concentration as may be sufficient during initial on-scene care of an out-of-hospital patient by basic skill level providers, with a higher-trained paramedic subsequently arriving and adding additional sensor(s) to heighten the sophistication of care if deemed necessary. This approach maximizes operational and budgetary flexibility of the DAU for use in varying applications.

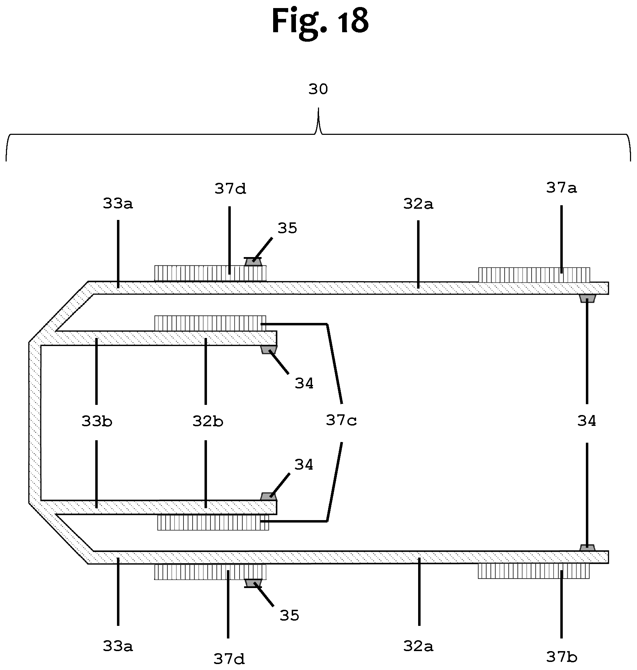

Fig. 18 shows the DAU **30** unit from a bottom-up view, more clearly showing the configuration of the left and right sensor housing upper arms **32a** relative to the left and right sensor housing lower arms **32b**. It can also be seen that sensor tips **34** are provided that enable sensors to obtain a fluid connection with sensor ports. For example, an inspiratory zone sensor **37a** and expiratory zone sensor **37b** are depicted to respectively interface with an inspiratory limb sensor port and expiratory limb sensor port on the convertible ventilator circuit patient manifold. Similarly, two patient zone sensors **37c** are shown to interface with patient zone sensor ports on the convertible ventilator circuit patient manifold. Auxiliary sensors **37d** intended for use with accessories external to the convertible ventilator circuit are also shown.

Affixed to each sensor is a sensor tip **34** configured to create an airtight seal when inserted into a sensor port, preferably including a tapered, generally conical contour to interface with one-way valves previously mentioned as part of preferred sensor ports. Instead of sensor tips on auxiliary sensors **37d**, a preferred embodiment utilizes auxiliary hubs **35** that are configured to be pneumatically or electrically compatible with accessories that may be used with the convertible ventilator circuit as previously described.

This view of the DAU **30** also clearly depicts how sensor housing upper arm necks **33a** and sensor housing lower arm necks **33b** can be positioned to facilitate widening of the gap between each respective arm to facilitate installation on a convertible ventilator circuit patient manifold.

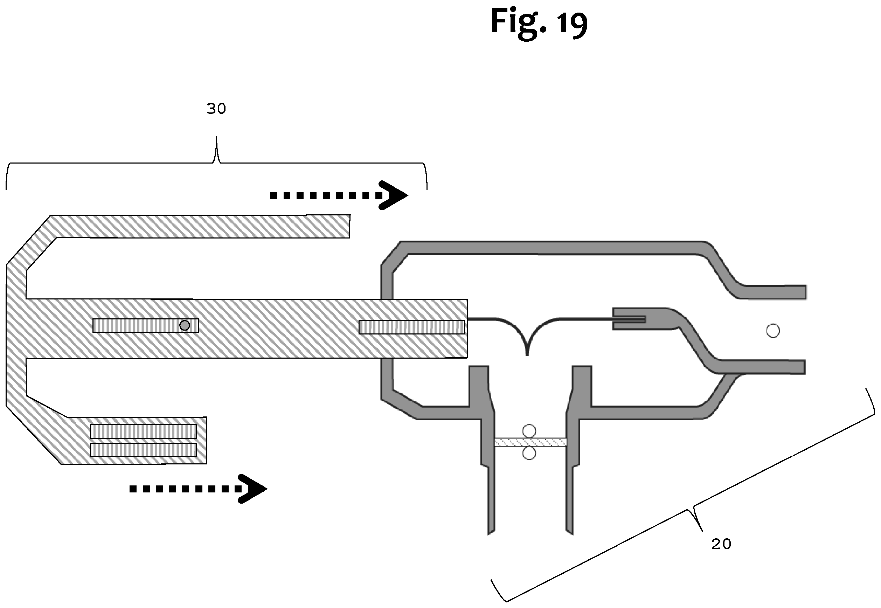

Fig. 19 illustrates a preferred embodiment of DAU **30** and convertible ventilator circuit patient manifold **20**, whereby the DAU **30** has an inner surface and the convertible ventilator circuit patient manifold **20** has an outer surface, and whereby the configuration of the inner surface of the DAU **30** is preferably shaped to interface with the configuration of the outer surface of the convertible ventilator circuit patient manifold **20**. It can be seen how the DAU **30** can be substantially installed on the convertible ventilator circuit patient manifold **20** by means of a linear sliding motion.

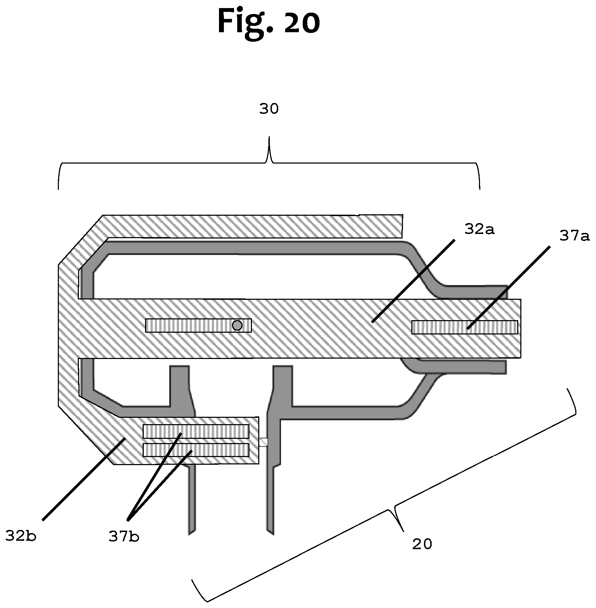

Fig. 20 illustrates a side view of the convertible ventilator circuit patient manifold **20** of Fig. 14, also showing the DAU **30** fully installed on the convertible ventilator circuit patient manifold **20**. It can be seen that the DAU sensor housing upper arm **32a** positions the inspiratory zone sensor **37a** to interface with its corresponding inspiratory zone sensor port (view obscured), while the DAU sensor housing lower arm **32b** and associated patient zone sensors are aligned to interface with patient zone sensor ports (view obscured).

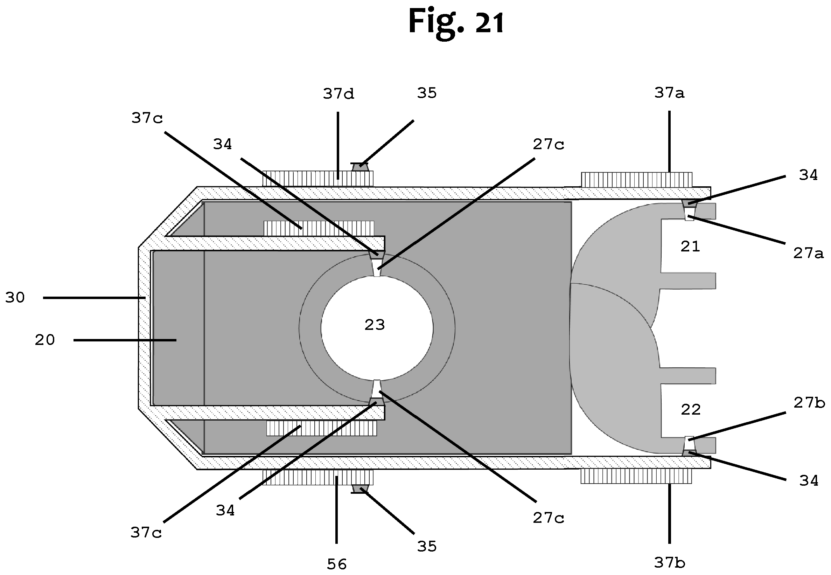

Fig. 21 shows a bottom-up view of the DAU **30** of Fig. 20 fully installed on the convertible ventilator circuit patient manifold **20**. The detailed alignment can be clearly seen between sensors of the DAU **30** with their corresponding sensor ports of the convertible ventilator circuit patient manifold **20**. For example, the inspiratory zone sensor **37a** is aligned so its sensor tip **34** inserts into and obtains an airtight seal with the inhalation zone sensor port **27a**, thereby enabling inspiratory zone sensor **37a** to have fluid connectivity with inspiratory gases. Similarly, the expiratory zone sensor **37b** is aligned so its sensor tip **34** inserts into and obtains an airtight seal with the exhalation zone sensor port **27b**, thereby enabling expiratory zone sensor **37b** to have fluid connectivity with expiratory gases. Patient zone sensors **37c** are aligned so their sensor tips **34** insert into and obtain airtight seals with their corresponding patient zone sensor ports **27**, thereby enabling patient zone sensors **37c** to have constant fluid connectivity with inspiratory and expiratory gases. Auxiliary sensors **37d** are fitted with an auxiliary hub enabling it to be interfaced with various accessories to the convertible ventilator circuit as previously described.

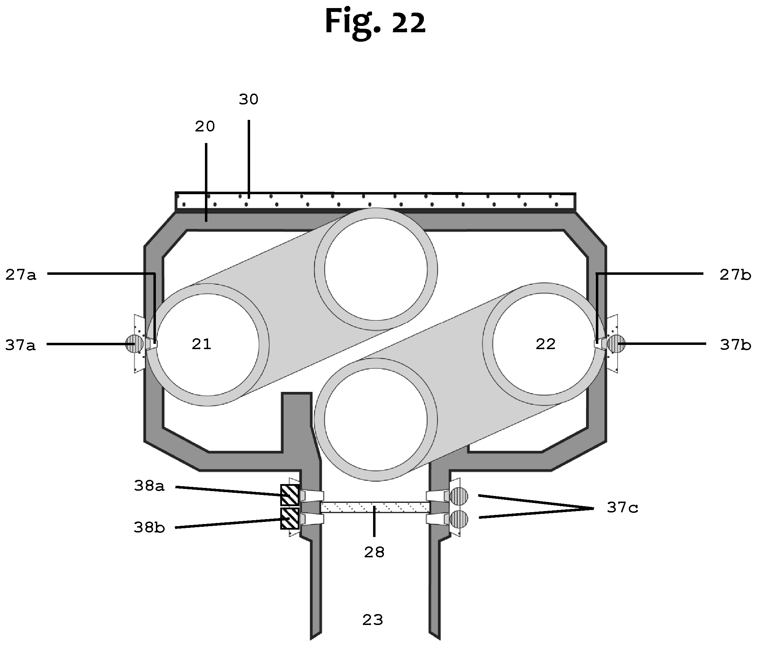

Fig. 22 shows an end-on view of the convertible ventilator circuit patient manifold **20** of Fig. 21, also showing the DAU **30** as fully installed on the convertible ventilator circuit patient manifold **20**. An inspiratory zone sensor **37a** is aligned so its sensor tip **(**not labeled due to diagrammatic constraints) inserts into and obtains an airtight seal with the inhalation zone sensor port **27a**, thereby enabling inspiratory zone sensor **37a** to have fluid connectivity with inspiratory gases in close proximity to the inspiratory limb connection **21**. Similarly, the expiratory zone sensor **37b** is aligned so its sensor tip (not labeled due to diagrammatic constraints) inserts into and obtains an airtight seal with the exhalation zone sensor port **27b**, thereby enabling expiratory zone sensor **37b** to have fluid connectivity with expiratory gases in close proximity to the expiratory limb connection **22**. Patient zone sensors **37c** are aligned so their sensor tips **(**not labeled due to diagrammatic constraints) insert into and obtain airtight seals with their corresponding patient zone sensor ports **(**not labeled due to diagrammatic constraints), thereby enabling patient zone sensors **37c** to have constant fluid connectivity with inspiratory and expiratory gases in close proximity to the patient airway adjunct receptacle **23**.

Fig. 22 also shows how a proximal flow sensor **38a** and a distal flow sensor **38b** each insert into and obtain an airtight seal with patient zone sensor ports **(**not labeled due to diagrammatic constraints) on either side of the flow sensor screen **28**. This enables differential pressure measurements on either side of the flow sensor screen **28** be mathematically derived consistent with generally known principles of this methodology to measure flow and cumulative flow over time (which equates to volume). It should be understood that alternative methodologies to measure flow can be integrated while remaining within the scope of the present invention.

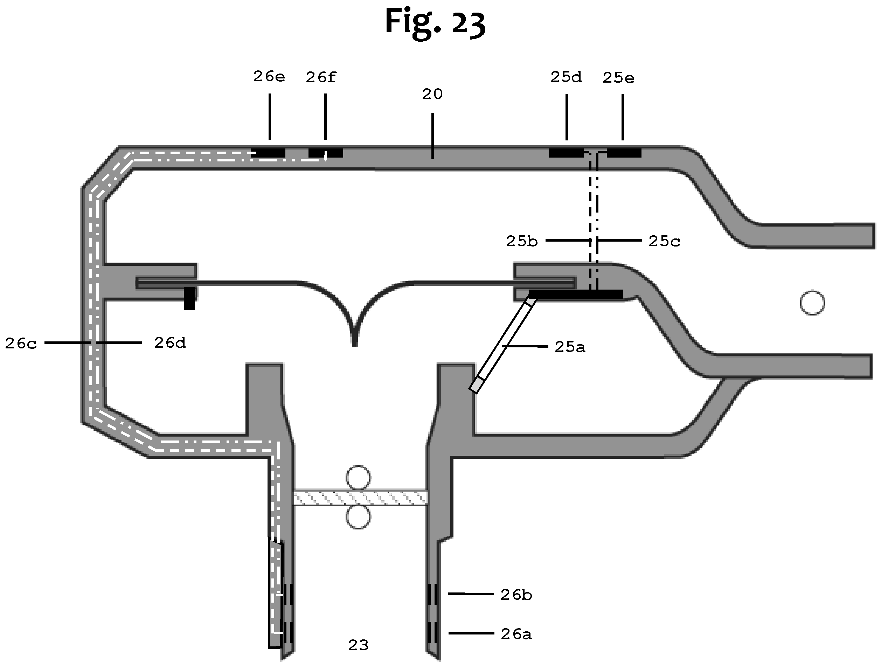

Fig. 23 shows a convertible ventilator circuit patient manifold **20** comprising additional attributes that favor data acquisition and electrically triggered functionality. An enhancement to a patient airway adjunct receptacle **23** includes a patient airway adjunct transmitting electrode ring **26a** and patient airway adjunct receiving electrode ring **26b**, each of which comprise a substantively circular conductive ring around the inner and outer surfaces of the patient airway adjunct receptacle **23**. The patient airway adjunct transmitting electrode ring **26a** is electrically continuous with an airway adjunct outer surface transmitting electrode **26e** by means of an airway adjunct transmitting conduit **26c**, the latter of which may be embedded in and/or on the structure of the convertible ventilator circuit patient manifold **20**. Similarly, the patient airway adjunct receiving electrode ring **26b** is electrically continuous with an airway adjunct outer surface receiving electrode **26f** by means of an airway adjunct receiving conduit **26d**, the latter of which may be embedded in and/or on the structure of the convertible ventilator circuit patient manifold **20**. Collectively, these components enable an electronic impulse beginning from the patient airway adjunct outer surface transmitting electrode **26e** to continue via the airway adjunct transmitting conduit **26c** to arrive at the patient airway adjunct transmitting electrode ring **26a**. In the event a patient airway adjunct is installed in the patient airway adjunct receptacle **23**, with said patient airway adjunct including an electrically conductive component capable of completing an electrical circuit between the airway adjunct transmitting electrode ring **26a** and the patient airway adjunct receiving electrode ring **26b**, then the electrical impulse originating from the patient airway adjunct outer surface transmitting electrode **26e** will be able to return to the adjacent patient airway adjunct outer surface receiving electrode **26f** by means of the airway adjunct receiving conduit **26d**.

Also shown in this Fig. 23 is an active expiratory valve outer surface transmitting electrode **25d** that is electrically continuous with an active expiratory limb valve **25a** via an active expiratory valve transmitting conduit **25b**. The active expiratory limb valve **25a** is also electrically continuous with an active expiratory valve outer surface receiving electrode **25e** via an active expiratory valve receiving conduit **25c**. In the event the active expiratory valve outer surface transmitting electrode **25d** and the active expiratory valve outer surface receiving electrode **25e** are connected to an external device capable of generating an electrical current, then such current will activate the active expiratory valve **25a** and cause it to close to permeable gas flow.

Alternative embodiments can be intuitively derived from this preferred description of electrical components enhancing the function of a convertible ventilator circuit patient manifold **20**. For example, in the case of a patient airway adjunct receptacle **23** being fitted with electrical transmitting and receiving rings that enable detection of the presence or absence of a patient airway adjunct by means of completing an electrical circuit between an airway adjunct transmitting electrode ring **26a** and airway adjunct receiving electrode ring **26b**, it can be easily seen where a pressure sensing and/or mechanical switch or trigger can be used as a mechanism for a data sensing means to record whether an airway adjunct is present or not in the airway adjunct receptacle **23**. Similarly, instead of an active expiratory valve **25a** being activated electrically when the valve needs to be actively closed, a pneumatic means to operate the active expiratory valve **25a** can be intuitively derived by substituting a pneumatic conduit such that a pneumatic receptacle located materially similar to the active expiratory valve outer surface transmitting electrode **25d** can transmit a pneumatic impulse in order to operate the active expiratory valve **25a**. These and other materially similar alternative embodiments are consistent with the scope of the invention.

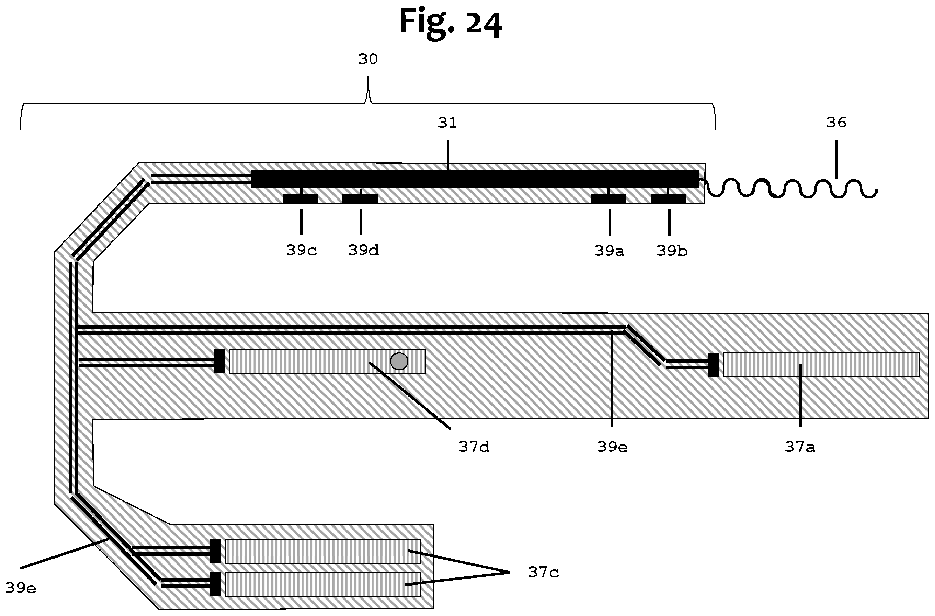

Fig. 24 shows a DAU **30** comprising additional components facilitating data acquisition and electrically triggered functionality of the active exhalation valve. A data acquisition unit electrical bus **31** has electrical connectivity with an active expiratory valve transmitting electrode **39a** and active expiratory valve receiving electrode **39b** that are each located on the outer surface of the DAU **30** such that they will contact the outer surface of the convertible ventilator circuit patient manifold when the DAU **30** is installed in its functional position on the convertible ventilator circuit patient manifold. The data acquisition unit electrical bus **31** also has electrical connectivity with an airway adjunct transmitting electrode **39c** and airway adjunct receiving electrode **39d** that are each located on the outer surface of the DAU **30** such that they will contact the outer surface of the convertible ventilator circuit patient manifold when the DAU **30** is installed in its functional position on the convertible ventilator circuit patient manifold. Also shown are electrical bus conduits **39e** that provide electrical connectivity between the data acquisition unit electrical bus **31** and sensors affixed to the DAU, including an inspiratory zone sensor **37a**, expiratory zone sensor (obscured in this view), patient zone sensors **37c** and auxiliary sensors **37d**. The electrical bus conduits **39e** could be embedded in the structure of the DAU **30** in a preferred embodiment. Additionally, as previously mentioned, each of the sensors, including an inspiratory zone sensor **37a**, expiratory zone sensor (obscured in this view), patient zone sensors **37c** and auxiliary sensors **37d** are preferably removable from the DAU **30** to enable various user configurations, including those that omit one or more sensors that may not be needed for a particular clinical application. It can be seen that, as a result of the apparatus provided, an inspiratory zone sensor **37a**, expiratory zone sensor (obscured in this view), patient zone sensors **37c** and auxiliary sensors **37d** all have electrical continuity with the data acquisition unit electrical bus **31** and, via the wired connection **36**, the DPU or any other partnering device that is capable of communicating with the DAU **30**.

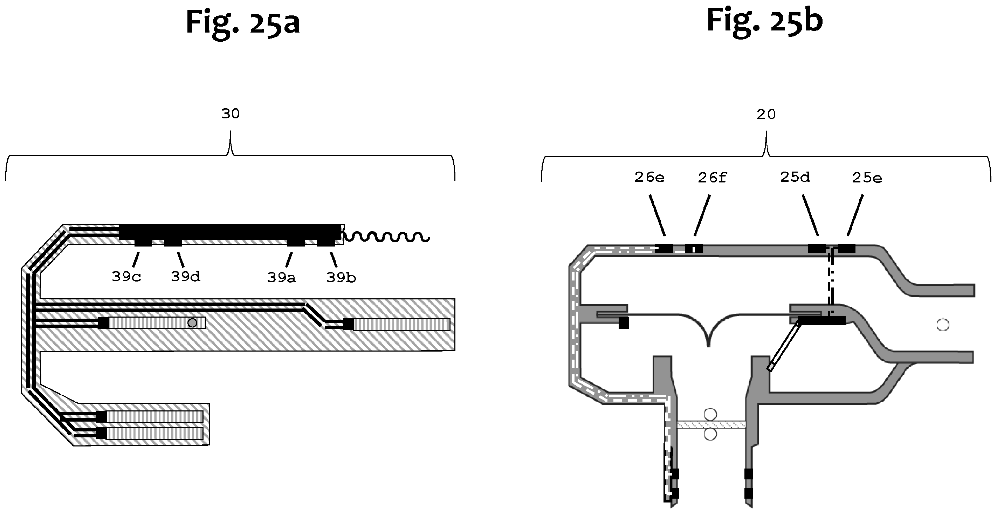

Fig. 25a shows the same components seen in Fig. 24, while Fig. 25b shows the same components seen in Fig. 23. It should be noted how, on the DAU **30**, the relative positioning of the active expiratory valve transmitting electrode **39a** and active expiratory valve receiving electrode **39b** appearing in Fig. 25a corresponds to, on the convertible ventilator circuit patient manifold **20** appearing in Fig. 25b, the relative positioning of the active expiratory valve outer surface transmitting electrode **25d** and the active expiratory valve outer surface receiving electrode **25e**. Similarly, it should be noted how, on the DAU **30**, the relative positioning of the airway adjunct transmitting electrode **39c** and airway adjunct receiving electrode **39d** corresponds to, on the convertible ventilator circuit patient manifold **20**, the relative positioning of the patient airway adjunct transmitting electrode **26e** and the patient airway adjunct outer surface receiving electrode **26f**.

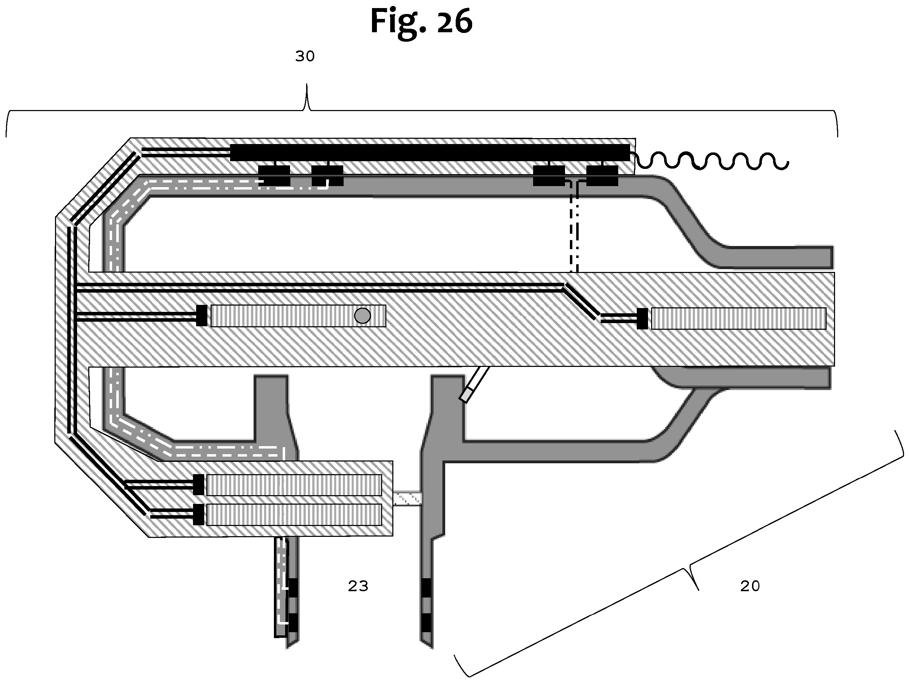

Fig. 26 shows the same components seen in Figs. 25a and 25b, except the DAU **30** is seen in its fully installed configuration on the convertible ventilator circuit patient manifold. Electrical structures as previously seen in Figs. 25a and 25b **(**not labeled due to diagrammatic constraints) can now be seen to be in physical contact that enables electrical contact between electrical components of the DAU **30** and convertible ventilator circuit patient manifold **20**.

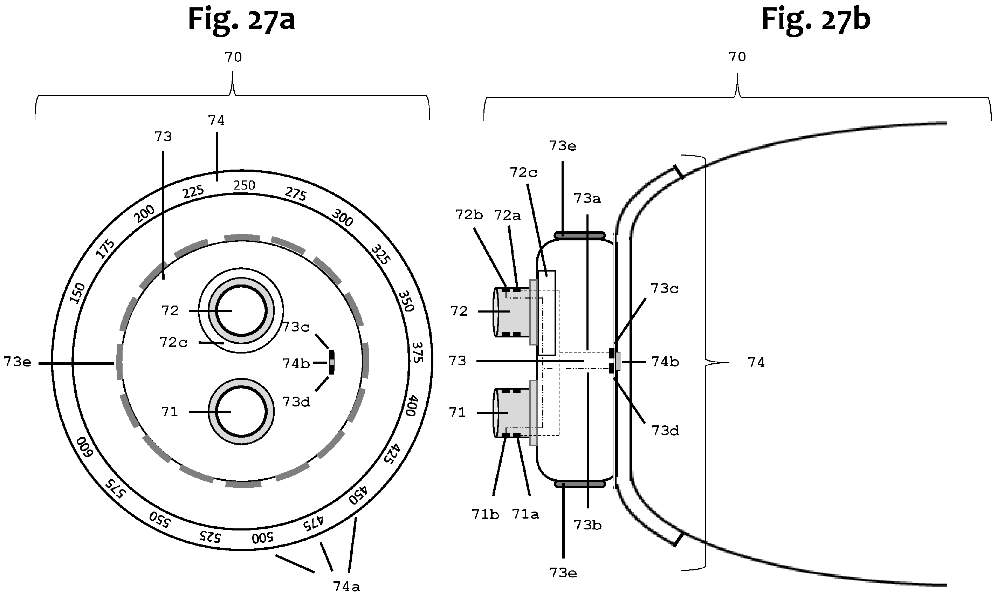

Fig. 27a shows an end-on view of an exemplar manual ventilator **70** having a neck **73** and manual ventilator parameter control **74** enabling adjustment of one or more ventilator parameters. In the embodiment appearing in Fig. 27a, the manual ventilator parameter control **74** enables adjustment of tidal volume (or breath size), with multiple parameter setting stops **74a**, however it should be understood one or more separate ventilation parameters may be controlled while remaining within the scope of the present invention. Fig. 27a also shows neck grasp grips **73e** that facilitate an ability for a user to grasp the manual ventilator **70** and hold via the neck **73**. Fig. 27a also shows an inspiratory limb connection **71** that provides for output flow from the manual ventilator **70** to enter an inspiratory limb connected via the inspiratory limb connection **71**. Flow of exhaled gases from the patient expiratory limb connected via the expiratory limb connection **72** vents to the open atmosphere via one or more expiratory vents **72c** that can be contained within the neck **73**, with said expiratory vents **72c** also representing a potential location for a positive end-expiratory pressure valve.

In the embodiment shown in Fig. 27a, it is possible for a user to grasp the manual ventilator by neck grasp grips **73e** in one hand while rotating the parameter adjustor **74** with the other hand. When such adjustment occurs, the 73-numbered components housed on and/or continuous with the neck **73**, as well as the inspiratory limb connection **71** and expiratory limb connection **72**, will remain stationary relative to the rotational orientation of the parameter selector **74** as it is manually rotated.

Fig. 27b shows the same components of Fig. 27a in a top-down view, along with additional attributes of the invention more readily visible from this perspective. The inspiratory limb connection **71** has an inspiratory limb signal transmission ring **71a** that is electrically continuous with a neck signal transmitting conduit **73a** and manual ventilator parameter transmitting electrode **73c**. The expiratory limb connection **72** similarly has an expiratory limb signal transmission ring **72a** that is also electrically continuous with the neck signal transmitting conduit **73a** and manual ventilator parameter transmitting electrode **73c**. A manual ventilator parameter receiving electrode **73d** can be seen to be electrically continuous with a neck signal receiving conduit **73b** which is simultaneously continuous with an inspiratory limb signal receiving ring **71b** and expiratory limb signal receiving limb **72b**.

The components provided constitute a means for an electrical signal to potentially be transmitted from one of the transmitting rings (**71a** and/or **72a**) to one of the receiving rings (**71b** and/or **72b**) depending on whether electrical connectivity exists between the parameter transmitting electrode **73c** and parameter receiving electrode **73d**. For example, in the event an electrical signal is able to be propagated from the manual ventilator parameter transmitting electrode **73c** to the manual ventilator parameter receiving electrode **73d**, then it can be seen such electrical signal, should it originate from either the inspiratory limb signal transmission ring **71a** and/or expiratory limb signal transmission ring **72a**, that the said electrical signal would propagate to the inspiratory limb signal receiving ring **71b** and/or expiratory limb signal receiving ring. Similarly, in the event an electrical signal is not able to be propagated from the manual ventilator parameter transmitting electrode **73c** to the manual ventilator parameter receiving electrode **73d**, then it can be seen that, should an electrical signal originate from either the inspiratory limb signal transmission ring **71a** and/or expiratory limb signal transmission ring **72a**, that the said electrical signal would not propagate to the inspiratory limb signal receiving ring **71b** and/or expiratory limb signal receiving ring **72b**.

In both Figs. 27a and 27b, a control setting electrode **74b** is shown in a configuration that would provide electrical continuity between the manual ventilator parameter transmitting electrode **73c** to the manual ventilator parameter receiving electrode **73d**. This control setting electrode **74b** contains one or more attributes capable of exerting a differentiating effect that modifies the electrical signal passing between the manual ventilator parameter transmitting electrode **73c** to the manual ventilator parameter receiving electrode **73d**, thereby enabling the said modification in signal to constitute a sensing means. For example, the differentiating effect could be produced when a control setting electrode **74b** exerts a known load resistance such that the electrical signal changes between the manual ventilator parameter transmitting electrode **73c** to the manual ventilator parameter receiving electrode **73d**. When the transmitted signal returns after modification by the differentiating effect, the resulting characteristics of the returning signal can be read by a processing unit, logic circuit, or other means to ascertain which parameter setting stop **74a** is operative at a given time during use. The control setting electrode **74b** could also provide a differentiating effect by means of comprising a semiconductor memory device, or the manual ventilator parameter transmitting electrode **73c** could propagate a radiofrequency interrogation impulse that results in the control setting electrode **74b** reflecting digital data that is subsequently received by the manual ventilator parameter receiving electrode **73d**, thereby providing the differentiating effect.

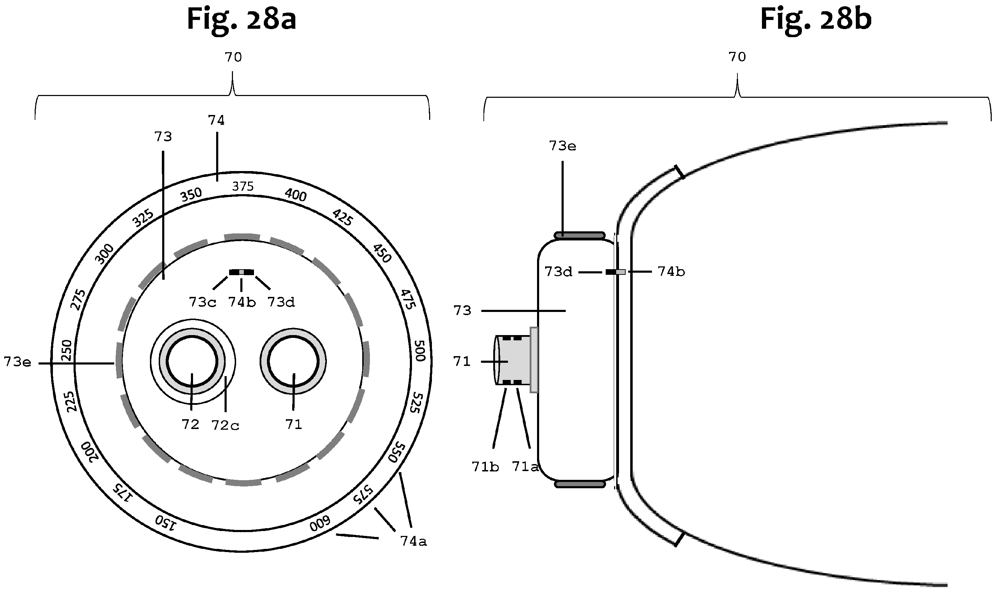

Figs. 28a and 28b respectively provide an end-on and side view of the same components of Figs. 27a and 27b. In this perspective, the spatial relationship between the control setting electrode **74b** and manual ventilator parameter receiving electrode **73d** can be better appreciated in that, should the parameter adjustor **74** be rotated, the control setting electrode **74b** will be rotationally displaced such that it will no longer be in contact with the manual ventilator parameter receiving electrode **73d** and, in the background behind the manual ventilator parameter receiving electrode **73d**, the manual ventilator parameter transmitting electrode **73c**.

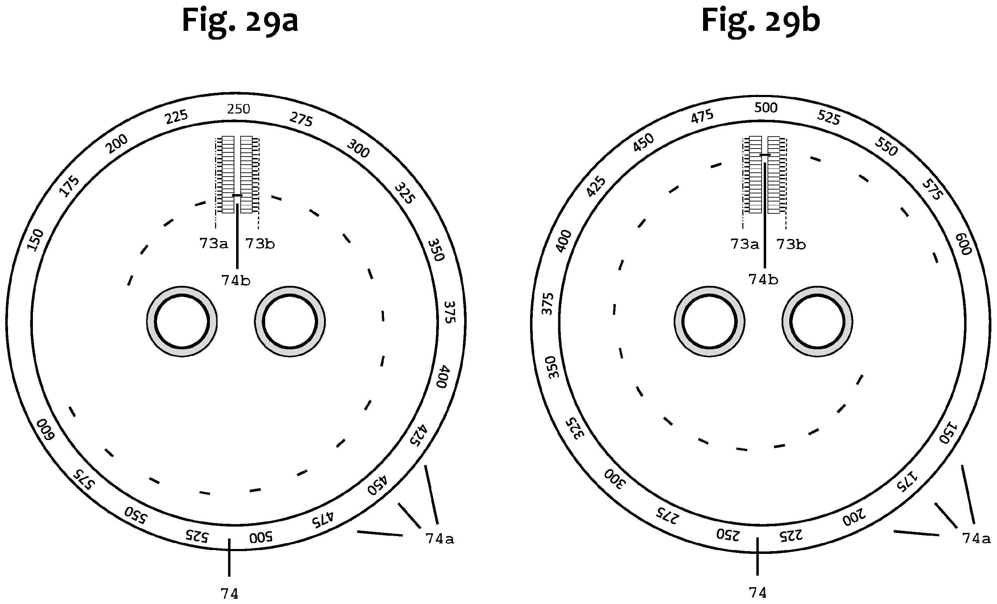

Fig. 29a shows an embodiment where multiple control setting electrodes **74b** are installed on the parameter adjustor **74,** which each control setting electrode **74b** corresponding to a single parameter setting stop **74a**. The location of each control setting electrode **74b** can be seen to have a specific distance between the center of the parameter adjustor **74** and its corresponding parameter setting stop **74a**, with no two control setting electrodes **74b** having the same distance between the center of the parameter adjustor **74** and its corresponding parameter setting stop **74a**. In Fig. 29a, the parameter adjustor **74** is shown with the 250 milliliter parameter setting stop **74a** is shown in the 12 o'clock position.

In Fig. 29b, the same components appear that are shown in Fig. 29a in an alternative configuration where the parameter adjustor **74** has been rotated anti-clockwise such that the 500 milliliter parameter setting stop **74a** is shown in the 12 o'clock position.

In the embodiment shown in Figs. 29a and 29b, there is a bank of manual ventilator transmitting electrodes **73c** equal in number to parameter setting stops **74a** and control setting electrodes **74b**, with the same number of manual ventilator receiving electrodes **73d** position such that, for any given parameter setting stop **74a**, a single control setting electrode **74b** will provide electrical continuity between a single manual ventilator parameter transmitting electrode **73c** and manual ventilator parameter receiving electrode **73d**. This configuration enables each control setting electrode **74b** to be identical with respect to any modification of electrical signal that may be induced upon it between its passage from the manual ventilator parameter transmitting electrode **73c** and manual ventilator parameter receiving electrode **73d**. This enables individual manual ventilator parameter transmitting electrodes **73c** and/or manual ventilator parameter receiving electrodes **73d** to exert the differentiation effect to enable a processing unit, logic circuit, or other means to ascertain which parameter setting stop **74a** is operative at a given time during use. Further clarifying this function, Fig. 29a shows a control setting electrode **74b** fifth up from the bottom of the array of manual ventilator parameter transmitting electrodes **73c** and manual ventilator parameter receiving electrodes **73d** that, in this view, corresponds to a tidal volume setting of 250 milliliters. In contrast, Fig. 29b shows the same components after adjustment of the parameter adjustor **74** in an anti-clockwise direction such that a tidal volume setting of 500 milliliters is selected, resulting in a control setting electrode **74b** completing the circuit fifth down from the top between the array of manual ventilator parameter transmitting electrodes **73c** and manual ventilator parameter receiving electrodes **73d**.

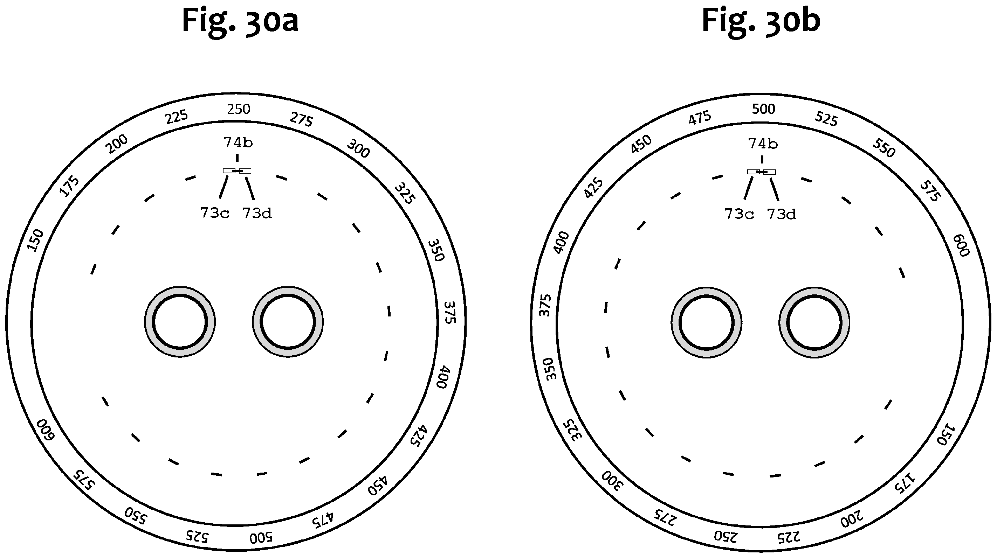

Figs. 30a and 30b show an alternative embodiment of the components shown in Figs. 29a and 29b. In this embodiment, a single manual ventilator parameter transmitting electrode **73c** and manual ventilator parameter receiving electrode **73d** is shown. Additionally, while a single control setting electrode **74b** still exists for each parameter setting stop **74a**, it can be seen that each single control setting electrode **74b** in this configuration has an identical distance between the center of the parameter adjustor **74** and a point toward the outer edge of the parameter adjustor **74** such that each control setting electrode **74b** is positioned to enable electrical contact with the single manual ventilator parameter transmitting electrode **73c** and manual ventilator parameter receiving electrode **73d** when the rotational configuration of the parameter adjustor **74** enables these components to spatially align on the rotational plane. This configuration allows each control setting electrode **74b** to provide the differentiating effect that enables a processing unit, logic circuit, or other means to ascertain which parameter setting stop **74a** is operative at a given time during use.

It should be understood that, based on these descriptions of the invention, one or more alternative embodiments can be easily provided that similarly enables a processing unit, logic circuit, or other data processing means to ascertain the position of a rotational parameter adjustor. Manual ventilator transmitting and receiving electrodes can be substituted by a magnetic switch that can be actuated by a magnetized element that serves the same role as a control setting electrode. A direct mechanical connection between a rotational parameter adjustor and an electrical rheostat, potentiometer, encoder, or other similar generic device will provide an identical capability as forms the basis of the current invention. It is also possible for all electrical components to be pneumatic that utilize changes in flow and/or operating pressure to determine the differentiating effect enabling the position of a rotational parameter adjustor to be ascertained.

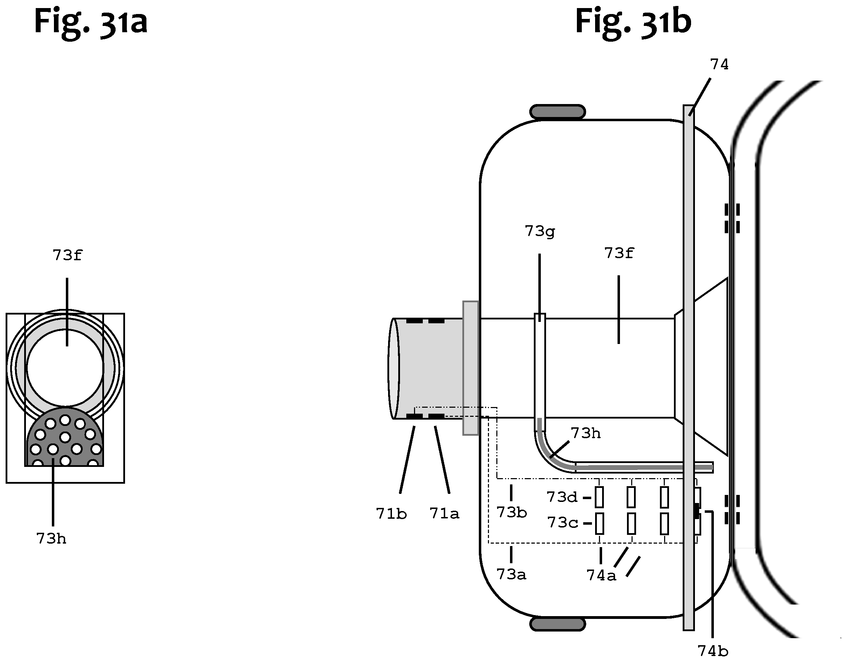

One or more alternative embodiments for a manual ventilator parameter adjustor may also be configured for linear action in place of rotational action. Fig. 31a shows an end-on view of a neck outflow adjustor **73h** that may serve to modify outflow characteristics in a neck outflow conduit **73f**, whereby the neck outflow adjustor **73h** comprises a flexible material with one or more perforations such that, upon being progressively advanced within a neck outflow conduit **73f**, the user can impede outflow to lengthen the amount of time allocated for breath delivery and/or to create a gradient between pressure on either side of the neck outflow adjustor **73h**. As seen in Fig. 31a, the neck outflow adjustor **73h** is in a position where it is substantively withdrawn from the neck outflow conduit **73h**.

Fig. 31b shows a side view of the same components described in Fig. 31a, further showing a neck outflow adjustor groove **73g** that contains the neck outflow adjustor **73h**. It can further be seen the neck outflow adjustor **73h** is structurally connected to a linear parameter adjustor **74,** such that linear displacement of the parameter adjustor **74** will cause the neck outflow adjustor **73h** to move within the neck outflow adjustor groove **73g**.

Fig. 31b also shows four exemplar parameter setting stops **74a**, and, for each parameter setting stop **74a**, a manual ventilator setting transmitting electrode **73c** and manual ventilator setting receiving electrode **73d**. As previously described, each manual ventilator setting transmitting electrode **73c** and/or manual ventilator setting receiving electrode **73d** is capable of exacting a differentiation effect that can enable a processing unit, logic circuit, or other means to ascertain which parameter setting stop **74a** is operative at a given time during use. In the embodiment shown, each manual ventilator setting transmitting electrode **73c** is electrically continuous with neck signal transmitting conduit **73a** that is itself continuous with both an inspiratory limb signal transmission ring **71a** and expiratory limb signal transmission ring (not visible in this view). In the configuration shown, a control setting electrode **74a** is structurally connected to the parameter adjustor **74** such that, with the parameter adjustor **74** in the position shown, the control setting electrode **74a** provides electrical continuity between the rightmost manual ventilator setting transmitting electrode **73c** and manual ventilator setting receiving electrode **73d**.

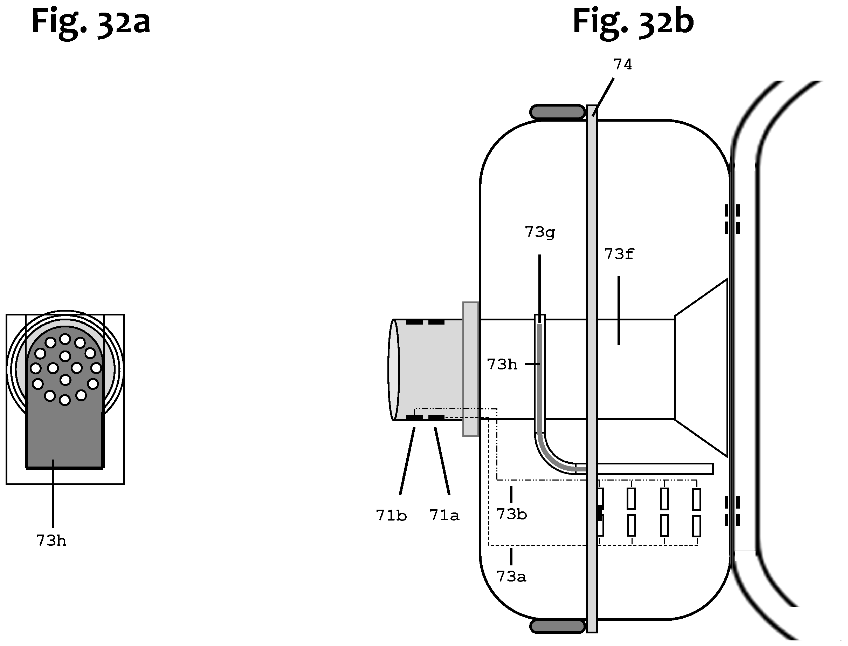

Figs. 32a and 32b show the same components and views of Figs. 31a and 31b. (Some components appearing in Fig. 32b are unlabeled for clarity of the drawing; please refer to Fig. 31b for labeling.) The parameter adjustor **74** can be seen to be in a position that causes the neck outflow adjustor **73h** to be in a position within the neck outflow conduit **73f** that results in partial obstruction of neck outflow. It can also be seen that the control setting electrode **74b** is providing electrical continuity between a different manual ventilator setting transmitting electrode 73b and manual ventilator setting receiving electrode **73d** that are different from those shown in Fig. 31b, thereby enabling a processing unit, logic circuit, or other means to ascertain which parameter setting stop **74a** is operative based on the differentiating effect as previously described.

It should be understood that the components shown resulting in a processing unit, logic circuit, or other means, to ascertain a manual ventilator parameter setting that is adjusted by a parameter setting adjustor with linear action, can be utilized to adjust a parameter other than neck outflow. For example, a parameter setting adjustor with linear action can be used by a user to adjust tidal volume, positive end-expiratory pressure, and/or a neck inflow device positioned in a manual ventilator that adjusts the time necessary for re-inflation.

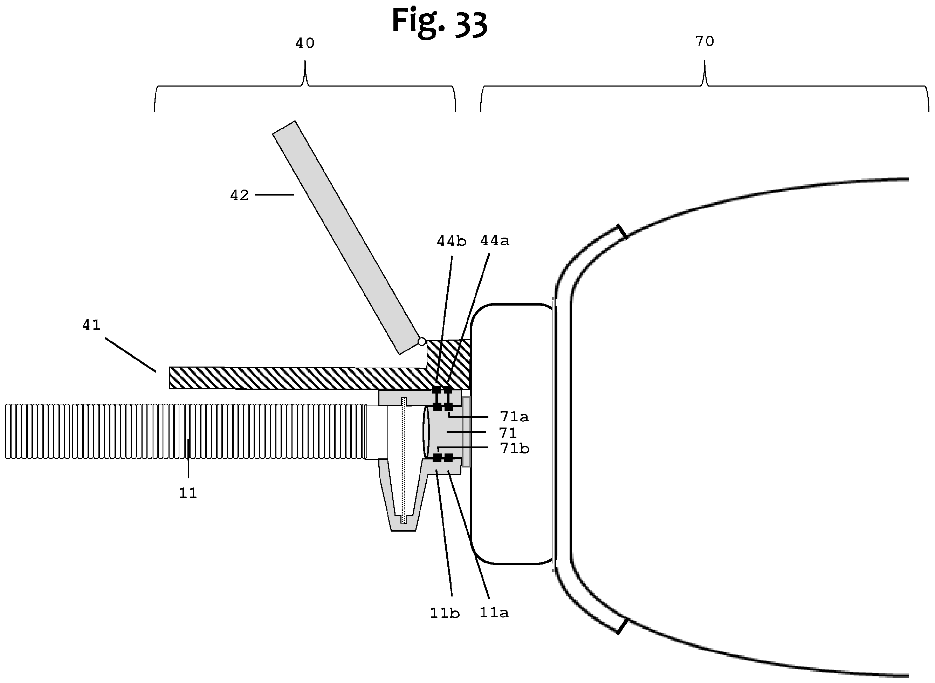

Fig. 33 shows a side view of a manual ventilator **70** having an inspiratory limb connection **71** that is connected to an inspiratory limb **11**. The manual ventilator **70** also has an expiratory limb connection that is connected to an expiratory limb, with those components in the background in this perspective. Attached to the top of the inspiratory limb **11** is a DPU **40** having a DPU housing **41** and display and audible emitter **42**. The DPU housing **41** contains an inspiratory limb transmitting electrode **44a** that interfaces with an inspiratory limb transmission signal ring **11a.** The DPU housing **41** also contains an inspiratory limb receiving electrode **44b** that interfaces with an inspiratory limb receiving signal ring **11b**. The inspiratory limb transmission signal ring **11a** of the inspiratory limb **11** can be seen to be in electrical contact with the inspiratory limb transmitting ring **71a** of the inspiratory limb connection **71** of the manual ventilator **70**. As previously described in reference to Fig. 27b, a transmitting signal originating at a manual ventilator inspiratory limb transmission ring **71a** is able to interface with a control setting electrode **74b** that is then received by a manual ventilator inspiratory limb receiving ring **71b**. Since the DPU **40** is interfaced as described with both the manual ventilator inspiratory limb transmission signal ring **71a** and manual ventilator inspiratory limb receiving signal ring **71b**, these components enable a DPU **40** to ascertain which parameter setting stop **74a** of a manual ventilator **70** is operative at a given time during use.

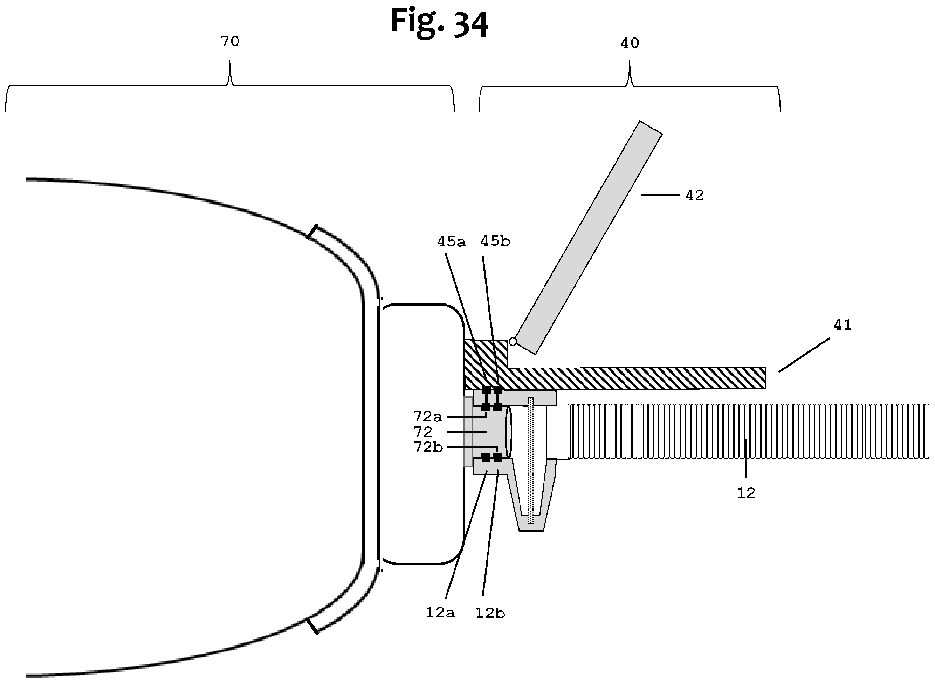

Fig. 34 shows the opposite side view of the manual ventilator **70** that was shown in Fig. 33. In this view, an expiratory limb **12** having an expiratory limb transmission signal ring **12a** and expiratory limb receiving signal ring **12b** is visible. The DPU housing **41** contains an expiratory limb transmitting electrode **45a** that interfaces with an expiratory limb transmission signal ring **12a**. The DPU housing **41** also contains an expiratory limb receiving electrode **45b** that interfaces with an expiratory limb receiving signal ring **12b**. It can be seen the expiratory limb transmission signal ring **12a** of the expiratory limb **12** is in electrical contact with the expiratory limb transmitting ring **72a** of the expiratory limb connection **72** of the manual ventilator **70**. As previously described in reference to Fig. 27b, a transmitting signal originating at a manual ventilator expiratory limb transmission ring **72a** is able to interface with a control setting electrode **74b** that is then received by a manual ventilator expiratory limb receiving ring **72b**. Since the DPU **40** is interfaced as described with both the manual ventilator expiratory limb transmission signal ring **72a** and manual ventilator expiratory limb receiving signal ring **72b**, these components enable a DPU **40** to ascertain which parameter setting stop **74a** of a manual ventilator **70** is operative at a given time during use.

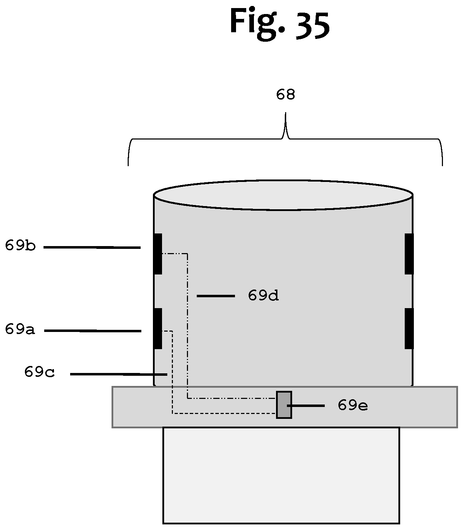

Fig. 35 shows an airway adjunct connector **68** that constitutes the means by which an airway adjunct such as a face mask, supraglottic airway, endotracheal tube, or tracheal tube, may be connected to a ventilation device. In the embodiment shown, the airway adjunct is of a design whereby it is inserted into a connector of the receiving device such that the internal surface of the receiving device interfaces with the external surface of the airway adjunct connector **68**. The external surface of the embodiment shown in Fig. 35 includes an airway adjunct transmission signal ring **69a** that is electrically continuous with an airway adjunct signal transmission conduit **69c** that interfaces with an airway adjunct identification chip **69e** capable of exacting a differentiation effect that can enable a processing unit, logic circuit, or other means to ascertain one or more specific identifying factor(s) that may be chosen to be programmed and/or otherwise configured to be unique to one or more attributes of the particular airway adjunct the airway adjunct connector **68** is physically attached to. The airway adjunct identification chip **69e** is electrically continuous with an airway adjunct signal receiving conduit **69d**, which is itself electrically continuous with an airway adjunct receiving signal ring **69b**.

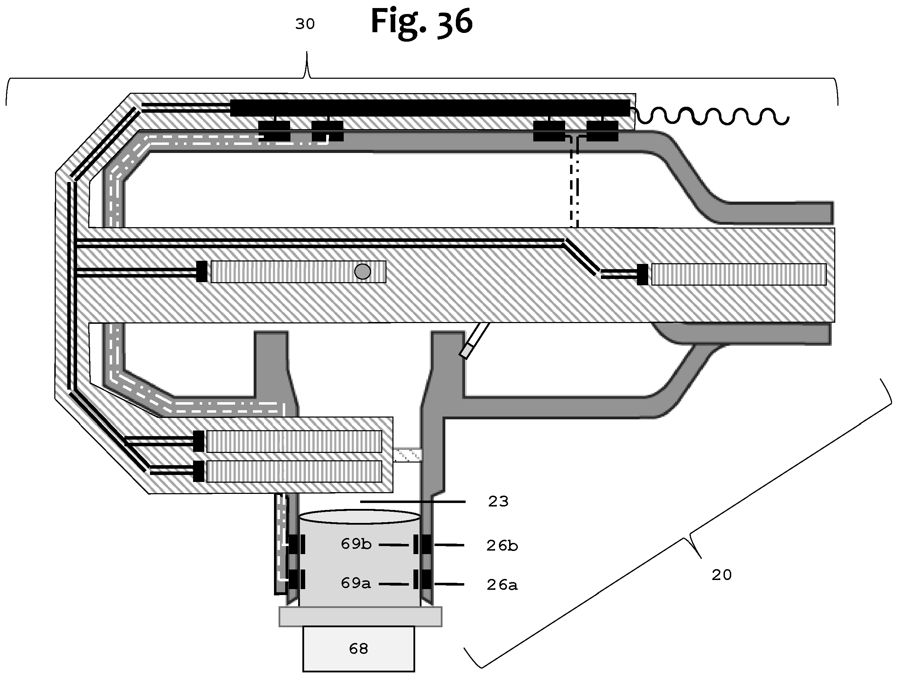

Fig. 36 shows the airway adjunct connector **68** previously described and shown in Fig. 36 in combination with components of a convertible ventilator circuit patient manifold **20** and DAU **30** as previously described and shown in Fig. 26. Referring to Figs. 25a and 25b, it was previously described that a DAU **30** having an airway adjunct transmitting electrode **39c** establishes an electrical connection with an airway adjunct outer surface transmitting electrode **26e** of a convertible ventilator circuit patient manifold **20**, and as shown in Fig. 23, that said airway adjunct outer surface transmitting electrode **26e** is electrically continuous with a patient airway adjunct transmitting electrode ring **26a**. As shown in Fig. 36, the patient airway adjunct transmitting electrode ring **26a** is able to establish an electrical connection with the airway adjunct signal transmission ring **69a** of the airway adjunct connector **68** when it is properly installed in the airway adjunct receptacle **23** of the convertible ventilator circuit patient manifold **20**, such that an electrical impulse originating from the DAU **30** is ultimately able to propagate to the airway adjunct transmitting electrode ring **26a**. As previously described and as shown in Fig. 35, this establishes connectivity enabling the DAU to transmit a signal to the airway adjunct identification chip **69e**.

Referring to Figs. 25a and 25b, it was previously described that a DAU **30** having an airway adjunct receiving electrode **39d** establishes an electrical connection with an airway adjunct outer surface receiving electrode **26f** of a convertible ventilator circuit patient manifold **20**, and, as shown in Fig. 23, said airway adjunct receiving electrode ring **26b**. As shown in Fig. 36, the patient airway adjunct receiving electrode ring **26b** is able to establish an electrical connection with the airway adjunct signal receiving rind **69b** of the airway adjunct connector **68** when it is properly installed in the airway adjunct receptacle **23** of the convertible ventilator circuit patient manifold **20**, such that an electrical impulse originating from the DAU **30** that has previously propagated to an airway adjunct identification chip **69e** is able to be conveyed back to the DAU **30** by means of the airway adjunct receiving signal ring **69b**, convertible ventilator circuit patient manifold airway adjunct receiving conduit **26d** (as shown in Fig. 23), which is electrically continuous with a convertible ventilator circuit patient manifold airway adjunct outer surface receiving electrode **26f** that is in contact with an airway adjunct receiving electrode **39d** of the DAU **30**.

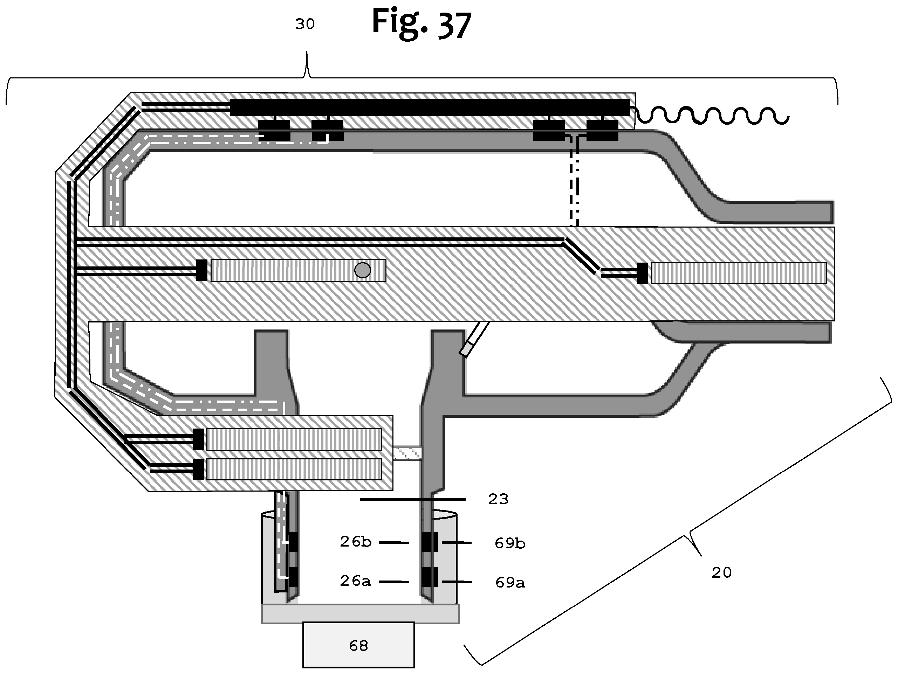

Fig. 37 shows all components of Fig. 36 in the same side view, with the airway adjunct connector **68** being shown in an alternative embodiment whereby the airway adjunct connector **68** of the airway adjunct fits over the outside of the airway adjunct receptacle **23** of the convertible ventilator circuit patient manifold **20**. The convertible ventilator circuit patient manifold airway adjunct receptacle **23** has a patient airway adjunct transmitting electrode ring **26a** that is able to form an electrical connection with an airway adjunct transmission signal ring **69a** when the airway adjunct connector **68** is properly installed on the convertible ventilator circuit patient manifold airway adjunct receptacle **23**. Similarly, the convertible ventilator circuit patient manifold airway adjunct receptacle **23** has a patient airway adjunct receiving electrode ring **26b** that is able to form an electrical connection with an airway adjunct receiving signal ring **69b** when the airway adjunct connector **68** is properly installed on the convertible ventilator circuit patient manifold airway adjunct receptacle **23**. This alternative embodiment for a patient airway adjunct connector **68** provides the same electrical connectivity previously described where a DAU **30** is able to interface with an airway adjunct identification chip **69e** as previously described and shown in Fig. 35.

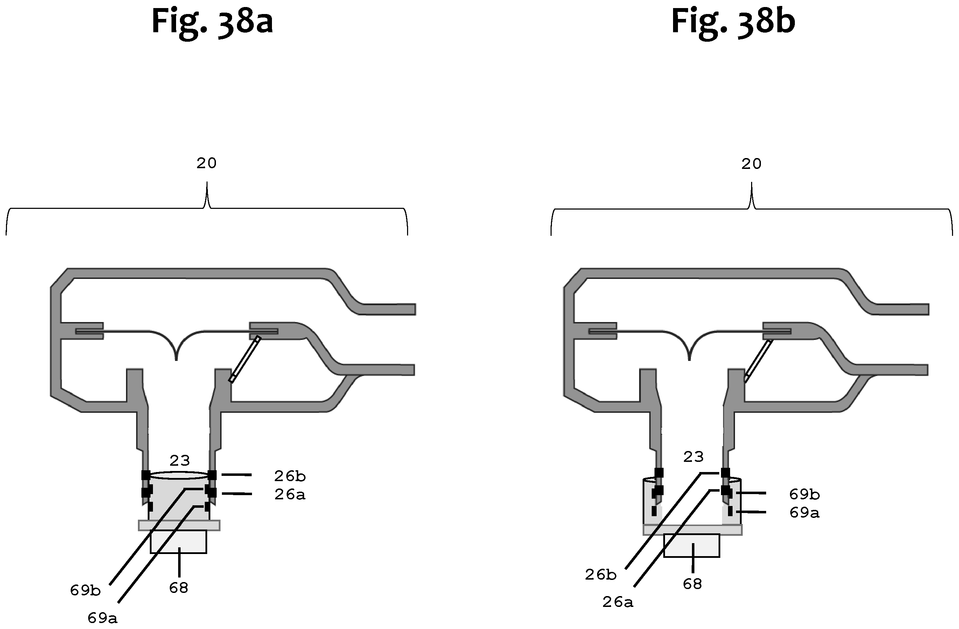

Fig. 38a shows the airway adjunct connector **68** in the embodiment shown in Fig. 36. Fig. 38b shows the airway adjunct connector **68** in the embodiment shown in Fig. 37. In both Figs. 38a and 38b it can be seen the airway adjunct connector **68** is incompletely installed in the convertible ventilator circuit patient manifold airway adjunct receptacle **23**, such that, an airtight seal remains between the convertible ventilator circuit patient manifold airway adjunct receptacle **23** and the patient airway adjunct connector **68**, providing for ongoing gas movement between the convertible ventilator circuit patient manifold **20** and airway adjunct (e.g., face mask, supraglotic airway, endotracheal tube, tracheal tube). However in both Figs. 38a and 38b it can be seen incomplete installation of the airway adjunct connector **68** into the convertible ventilator circuit patient manifold airway adjunct receptacle **23** causes loss of the electrical connectivity between the convertible ventilator circuit patient airway adjunct transmitting electrode ring **26a** and the airway adjunct transmission signal ring **69a**. Similarly, incomplete installation of the airway adjunct connector **68** into the convertible ventilator circuit patient manifold airway adjunct receptacle **23** causes loss of the electrical connectivity between the convertible ventilator circuit patient airway adjunct receiving electrode ring **26b** and the patient airway adjunct receiving signal ring **69b**.

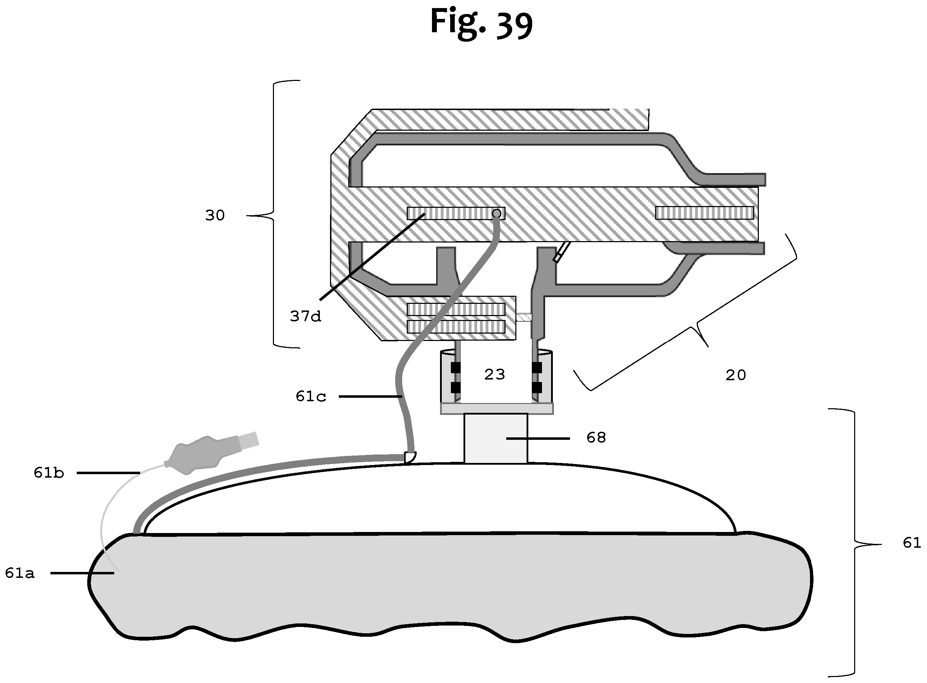

Fig. 39 shows a DAU **30** installed on a convertible ventilator circuit patient manifold **20** and a patient airway adjunct, in this case a face mask **61**, having a patient airway adjunct connector **68** that is installed in a convertible ventilator circuit patient manifold airway adjunct receptacle **23**. The face mask **61** has a face mask cuff **61a** that is inflated with air (or other fluid) that is intended to conform to the contours of a patient's face in order to achieve an airtight seal such that ventilatory gas can substantively flow between the convertible ventilator circuit patient manifold **20** and the nose and mouth of the patient. The face mask **61** further has a face mask cuff inflation/deflation valve **61b** that is compatible with a generic syringe which can be used to adjust pressure in the face mask cuff **61a**. The face mask **61** further has a face mask cuff pressure tube **61c** having two ends that is preferably flexible but of low compliance such that it is able to optimally propagate internal pressures from one end to the opposite end. One end of the face mask cuff pressure tube **61c** communicates with the interior of the face mask cuff **61a** with the other end connecting to a DAU auxiliary sensor **37d** which comprises a pressure sensor. This combination of components of the present invention enables a DAU **30** to monitor and transmit measurements of pressure in the face mask cuff **61a**.

Continuing to refer to Fig. 39, it can be seen the flexible face mask cuff **61a** is in a partially inflated state resulting in the surfaces of the face mask cuff **61a** being substantially slack.

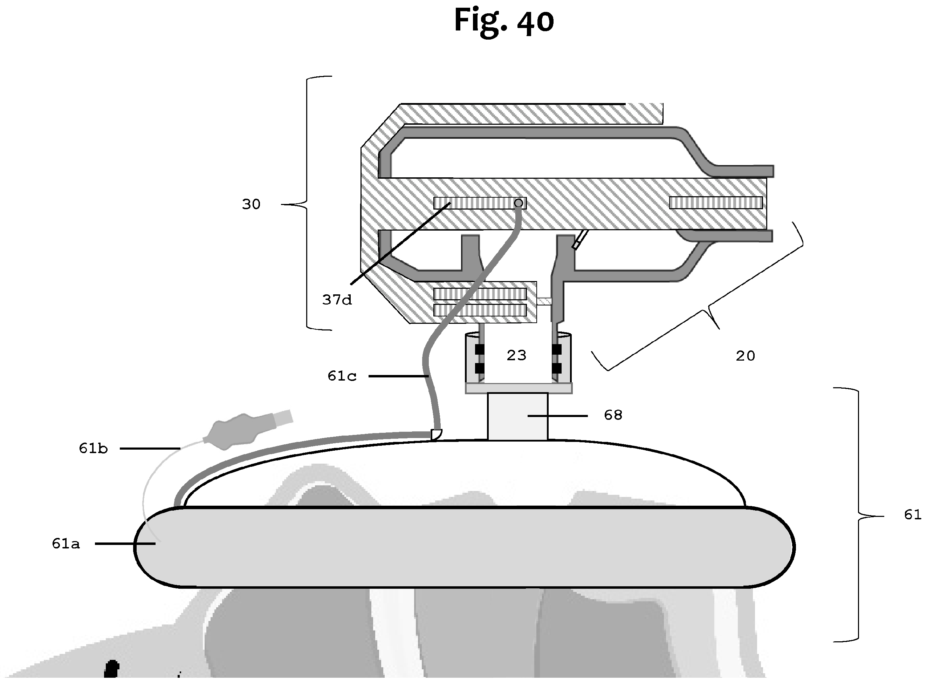

Fig. 40 shows the identical combination of components of the invention previously shown in Fig. 39. It can be seen the face mask **61** is pressed against the face of a patient, causing the face mask cuff **61a** to compress into a substantially taut condition. Due to the compression of the face mask cuff **61a**, the pressure inside the face mask cuff **61a** will increase, causing an increase in pressure in the face mask cuff pressure tube **61c** that can be subsequently sensed by a DAU auxiliary sensor **37d** which comprises a pressure sensor.

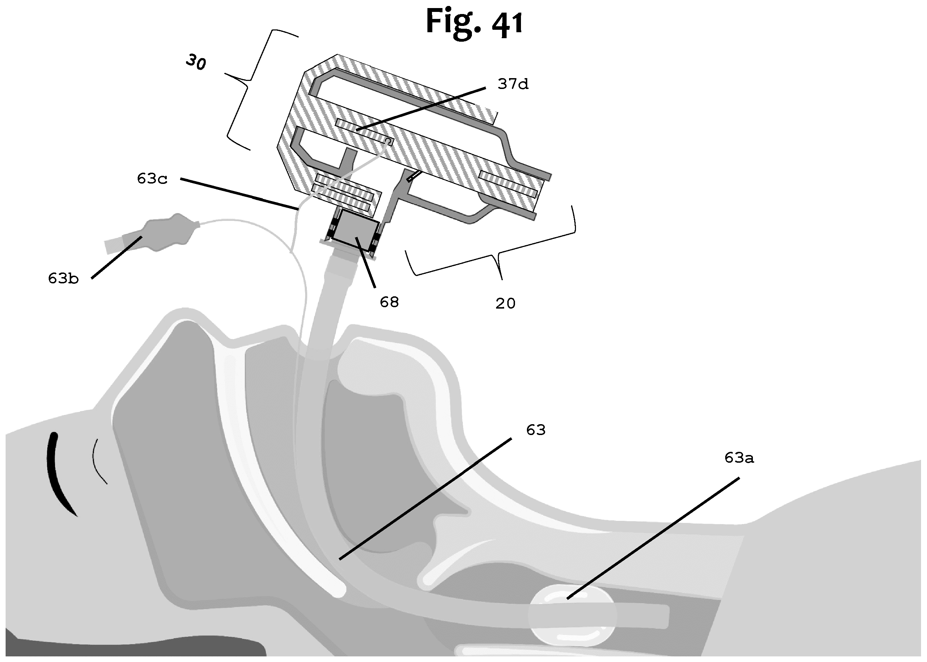

Fig. 41 shows a DAU **30** installed on a convertible ventilator circuit patient manifold **20** and a patient airway adjunct, in this case an endotracheal tube **63**, having a patient airway adjunct connector **68** that is installed in a convertible ventilator circuit patient manifold **20**. The endotracheal tube has an endotracheal tube cuff **63a** that is inflated with air (or other fluid) that is intended to conform to the contours of a patient's trachea in order to achieve an airtight seal such that ventilatory gas can substantively flow between the convertible ventilator circuit patient manifold **20** and the trachea of the patient. The endotracheal tube **63** further has an endotracheal tube cuff inflation/deflation valve **63b** that is compatible with a generic syringe which can be used to adjust pressure in the endotracheal tube cuff **63a**. The endotracheal tube **63** further has an endotracheal tube cuff pressure tube **63c** having two ends that is preferably flexible but of low compliance such that it is able to optimally propagate internal pressures from one end to the opposite end. One end of the endotracheal tube cuff pressure tube **63c** communicates with the interior of the endotracheal tube cuff **63a** with the other end connecting to a DAU auxiliary sensor **37d** which comprises a pressure sensor. This combination of components of the present invention enables a DAU **30** to monitor and transmit measurements of pressure in the endotracheal tube cuff **63a**.

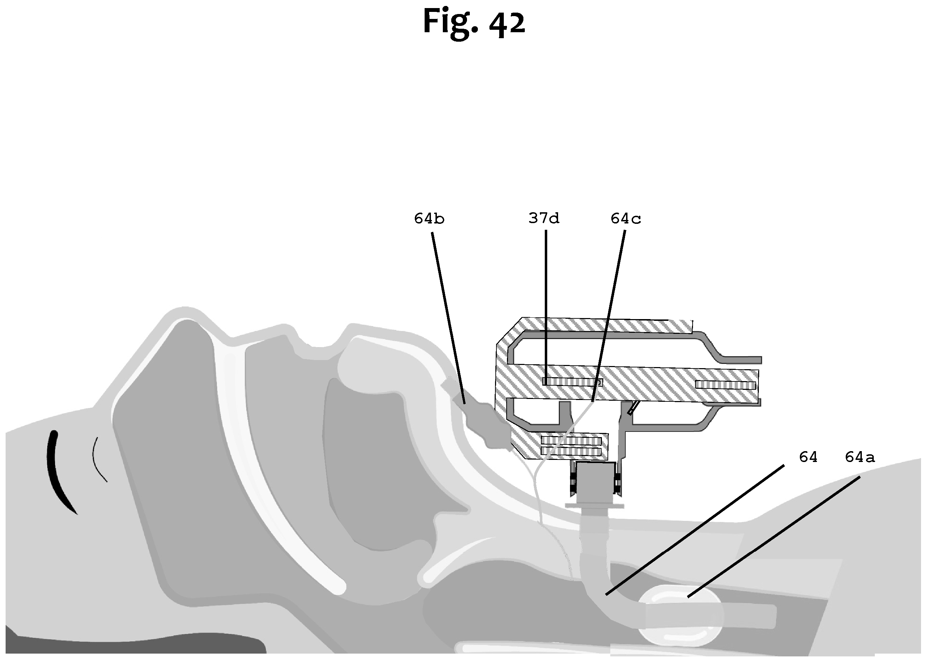

Fig. 42 shows the same components as previously shown and labeled in Fig. 41 except in place of an endotracheal tube, a tracheal tube **64** is shown. The tracheal tube **64** is connected to the convertible ventilator circuit patient manifold upon which a DAU is installed as previously described. The tracheal tube **64** is connected to the convertible ventilator circuit patient manifold as previously described for an endotracheal tube and as shown in Fig. 41. The tracheal tube has a tracheal tube cuff **64a** that is inflated with air (or other fluid) that is intended to conform to the contours of a patient's trachea in order to achieve an airtight seal such that ventilatory gas can substantively flow between the convertible ventilator circuit patient manifold and the trachea of the patient. The tracheal tube **64** further has a tracheal tube cuff inflation/deflation valve **64b** that is compatible with a generic syringe which can be used to adjust pressure in the tracheal tube cuff **64a**. The tracheal tube **64** further has a tracheal tube cuff pressure tube **64c** having two ends that is preferably flexible but of low compliance such that it is able to optimally propagate internal pressures from one end to the opposite end. One end of the tracheal tube cuff pressure tube **64c** communicates with the interior of the tracheal tube cuff **64a** with the other end connecting to a DAU auxiliary sensor **37d** which comprises a pressure sensor. This combination of components of the present invention enables a DAU **30** to monitor and transmit measurements of pressure in the tracheal tube cuff **64a**.

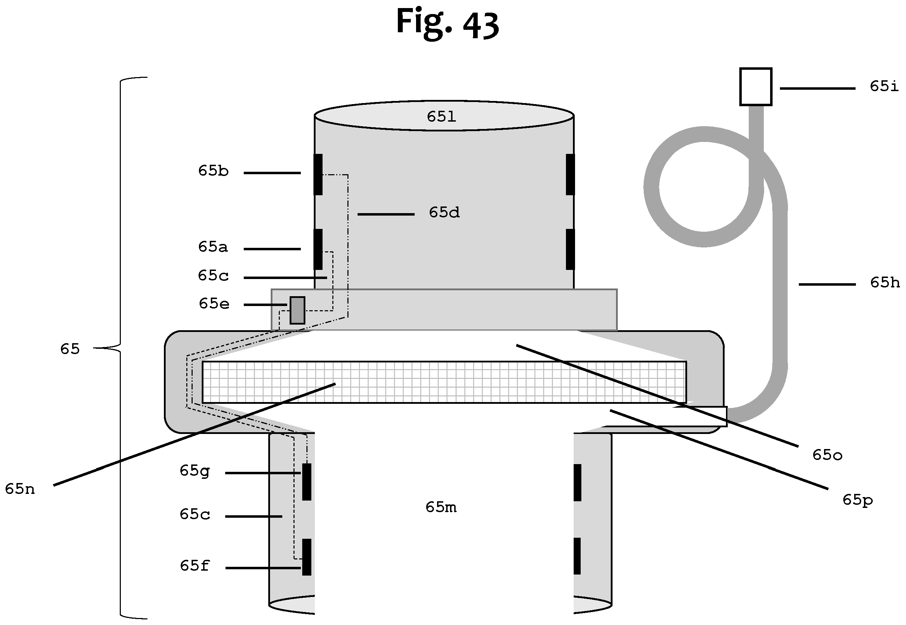

Fig. 43 shows a side view of a heat-moisture exchanger (HME) **65** that is designed to be an intermediate component to be inserted between a convertible ventilator circuit patient manifold airway adjunct receptacle (item **23** in Fig. 36) and an airway adjunct connector (item **68** in Fig. 36). An HME convertible ventilator patient manifold connector **65l** is identically profiled in shape and size to an airway adjunct connector (item **68** in Fig. 36) such that the HME convertible ventilator patient manifold connector **65l** is able to be inserted into a convertible ventilator patient manifold receptacle (item **23** in Fig. 36) precisely as previously described and as shown in Fig. 36 for the connection between a convertible ventilator patient manifold receptacle (item **23** in Fig. 36) and an airway adjunct connector (item **68** in Fig. 36).

Fig. 43 also shows HME **65** having a heat and moisture recapture element **65n** that is permeable to bidirectional gas flow but captures heated moisture exhaled by the patient during each exhalation such that it can pre-heat and humidify fresh inspiratory gas during the next inhalation phase of a singular breath cycle. The location of the heat and moisture recapture element **65n** is located within the gas passageway, such that a heat and moisture non-recapture zone **65o** is between the heat and moisture recapture element **65n** and HME convertible ventilator patient manifold connector **65l**, whereby ventilatory gases passing in either direction in this area are not substantially heated and/or humidified. In contrast, a heat and moisture recapture zone **65p** is positioned between the heat and moisture recapture element **65n** and an HME airway adjunct connector **65m**, such that ventilator gases passing in either direction in this area are substantially exposed to recaptured heat and humidification from the patient.

Fig. 43 shows HME **65** having an airway adjunct receptacle **65m** that is identically profiled in shape and size to the convertible ventilator patient manifold receptacle (item **23** in Fig. 36), such that an airway adjunct connector (item **68** in Fig. 36) is able to be connected to an HME airway adjunct receptacle **65m** precisely as previously described and as shown in Fig. 36 for the connection between a convertible ventilator patient manifold receptacle (item **23** in Fig. 36) and an airway adjunct connector (item **68** in Fig. 36).

Fig. 43 further shows the HME **65** having an HME signal transmission ring **65a** that is electrically continuous with an HME signal transmission conduit **65c** that is in electrical continuity with an HME identification chip **65e**. The HME transmission signal conduit **65c** continues from the HME identification chip **65e** to connect with an HME airway adjunct transmission signal relay ring **65f**. The HME **65** further has an HME airway adjunct receiving signal relay ring **65g** that is electrically continuous with an HME receiving signal ring **65b** via an HME receiving signal conduit **65d**.

Fig. 43 shows the HME **65** also having an HME pressure tube **65h** having two ends that is preferably flexible but of low compliance such that it is able to optimally propagate internal pressures from one end to the opposite end. One end of the HME pressure tube **65h** communicates with the heat and moisture recapture zone **65p**, with the opposite end forming an HME pressure tube auxiliary sensor connector **65i** that is able to connect to a DAU auxiliary sensor (item **37d** as shown in Fig. 39) which comprises a pressure sensor.

A preferred embodiment of HME pressure tube connector **65i** can be capped or plugged to prevent gas leakage when the HME **65** is utilized without being connected to a DAU auxiliary sensor (item **37d** as shown in Fig. 39).

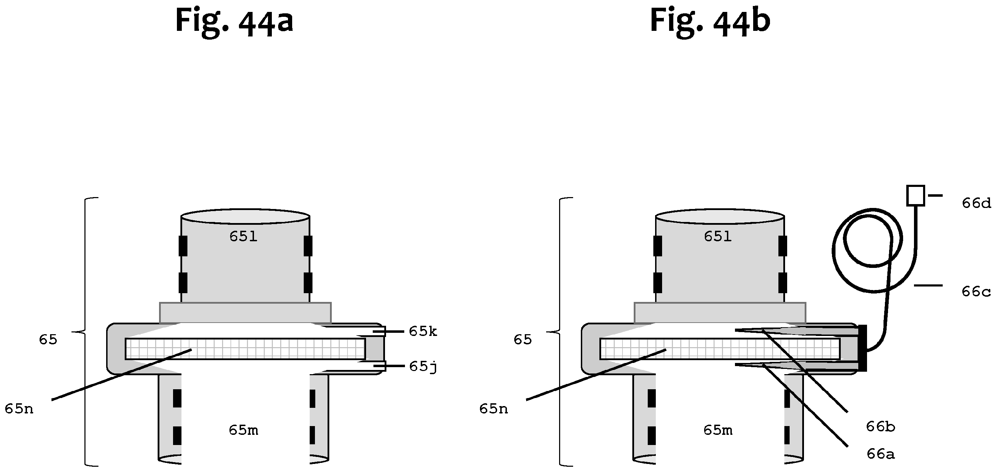

Fig. 44a shows a preferred embodiment of the HME **65** as previously described and shown in Fig. 43 but shown in a side view rotated approximately 15 degrees on the horizontal plane, which reveals a recapture zone temperature and/or humidity probe port **65j** and, directly above it, a nonrecapture zone temperature and/or humidity probe port **65k**. Preferred embodiments for a recapture zone temperature and/or humidity probe port **65j** and nonrecapture zone temperature and/or humidity probe port **65k** include a passive, multi-cuspid, cross-slit valve design similar to a human heart valve where the pressure inside the circuit will press the valve closed to prevent leakage when no temperature and/or humidity probe is present, but will deflect under pressure upon the tip of a temperature and/or humidity probe contacting the exterior surface of each valve, such that proper positioning of a temperature and/or humidity sensor will cause the recapture zone temperature and/or humidity probe port **65j** and nonrecapture zone temperature and/or humidity probe port **65k** to open and permit communicable access of temperature and/or humidity probes into the gas passageway. It should be understood that common alternative forms of one-way valves are to be construed to be provided by the present invention. For example, duckbill, ball, umbrella, and other common one-way valve designs would function in the role described in the present invention.

Fig. 44b shows the preferred embodiment of the HME **65** as previously described and shown in Fig. 44a, further showing a recapture zone temperature and/or humidity probe **66a** installed in the recapture zone temperature and/or humidity probe port **65j**. Similarly, a nonrecapture zone temperature and/or humidity probe **66b** is shown installed in the nonrecapture zone temperature and/or humidity probe port **65k**. Both temperature and/or humidity probes are electrically connected to a temperature and/or humidity probe connection cable **66c** that itself leads to a temperature and/or humidity probe connection cable connector **66d** that is capable of connecting with a DAU, thereby enabling temperature and/or humidity data to be acquired by the DAU for subsequent processing by a DPU.

Alternatives to the preferred embodiment described in Figs. 44a and 44b are easily derived from the detailed description provided. For example, and alternative of the HME **65** may only have a recapture zone temperature and/or humidity port **65j** intended to be used with a single recapture zone temperature and/or humidity probe **66a**. Additionally, the relative positioning of recapture and nonrecapture zone ports and probes may be modified from the specific depiction described without diverging from the scope of the invention.

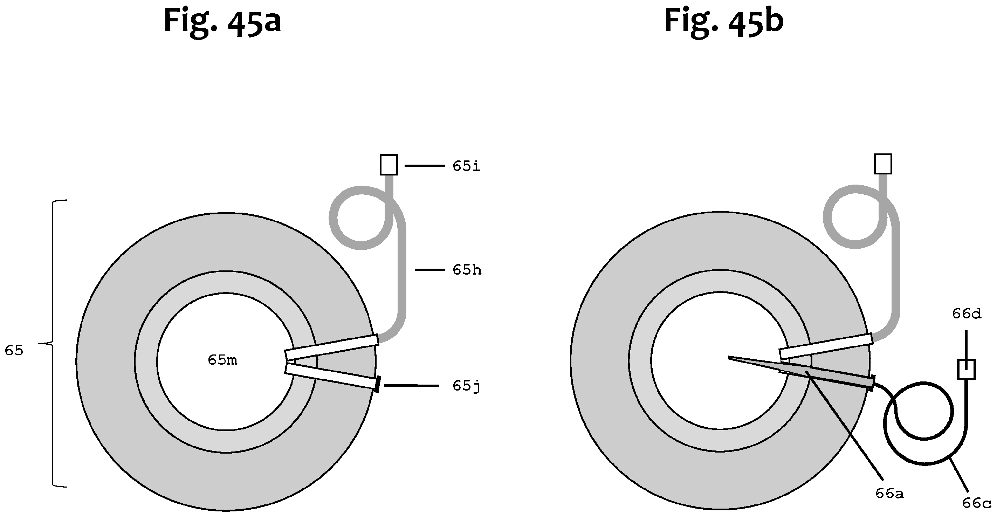

Fig. 45a shows the components of Fig. 44a in a bottom-up view, which shows the relative positioning of the HME pressure tube **65h** and recapture zone temperature and/or humidity probe port **65j**, the latter of which appears in the foreground obscuring the view of a nonrecapture zone temperature and/or humidity probe port that is in the immediate background in this view. The HME pressure tube auxiliary sensor connector **65i** is also visible in this view.

Fig. 45b shows the same components of Fig. 44a in the identical view, also showing a recapture zone temperature and/or humidity probe **66a** installed in the recapture zone temperature and/or humidity probe port **65j**. The temperature and/or humidity probe connection cable **66c** and temperature and/or humidity probe connection cable connector **66d** are also visible in this view.

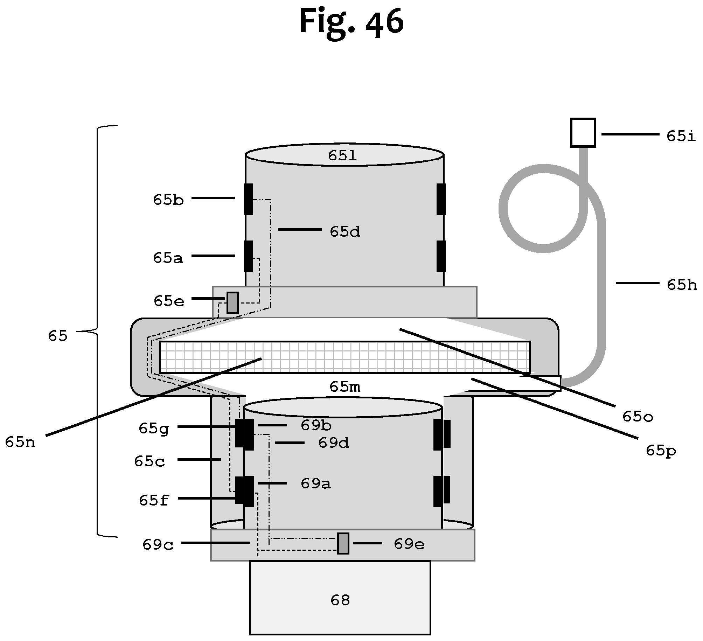

Fig. 46 shows the same components of Fig. 43, with the addition of an airway adjunct connector **68** installed in the HME airway adjunct receptacle **65m**. As previously described, the HME airway adjunct receptacle **65m** is identically profiled in shape and size to the convertible ventilator patient manifold receptacle (item **23** in Fig. 36) such that an airway adjunct connector **68** is able to be connected precisely as previously described and as shown in Fig. 36 for the connection between a convertible ventilator patient manifold receptacle (item **23** in Fig. 36) and an airway adjunct connector **68**. This allows the HME **65** to be inserted as an intermediary component between a convertible ventilator circuit patient manifold receptacle (item **23** in Fig. 36) and an airway adjunct connector **68**.

Fig. 46 further shows continuity of all electrical and sensor components between the HME **65** and airway adjunct connector **68**. It can be seen the HME signal transmission ring **65a** is electrically continuous with an HME signal transmission conduit **65c** that is in electrical continuity with an HME identification chip **65e**, which provides further electrical continuity with an HME airway adjunct transmission signal relay ring **65f** that is in electrical contact with an airway adjunct transmission signal ring **69a**. Electrical continuity continues from the airway adjunct transmission signal ring **69a** to an airway adjunct identification chip **69e**, which is then connected to an airway adjunct receiving signal ring **69b** via an airway adjunct receiving signal conduit **69d**. The airway adjunct receiving signal ring **69b** has electrical continuity with the HME receiving signal relay ring **65g**, which has electrical continuity with the HME receiving signal ring **65b** via an HME receiving signal conduit **65d**.

Fig. 47 shows a DAU **30** that is installed on a convertible ventilator circuit patient manifold **20**. The convertible ventilator patient manifold **20** is connected to an HME **65**. The HME **65** is connected to the airway adjunct connector **68** of an endotracheal tube **64**. This combination of components provided by the invention enables gas flow between the convertible ventilator circuit patient manifold **20**, an HME 65, and endotracheal tube **64**. Referring to the electrical connections described in the previous paragraph and as shown in Fig. 46, the cumulative result of this combination of components provided by the invention enables also results in electrical continuity for both transmission and receiving signals between: (1) the DAU **30**; (2) the convertible ventilator circuit patient manifold **20**; (3) the HME **65**; and (4) the airway adjunct connector **68** of the endotracheal tube **63**.

Fig. 47 also shows the HME pressure tube **65h** and HME pressure tube connector **65i** connected to a DAU auxiliary sensor **37d**. Similarly, the HME temperature and/or humidity cable **66c** is shown, which is connected to a DAU auxiliary sensor **37d** via a HME temperature and/or humidity cable connection **66d**. A preferred embodiment of the HME temperature and/or humidity cable connection **66d** is that it is shaped in size and profile of a DAU auxiliary sensor **37d** such that the HME temperature and/or humidity cable connection **66d** can connect directly to the DAU **30** as would a DAU auxiliary sensor **37d**, thereby eliminating the need for a DAU auxiliary sensor **37d** to be fitted as an intermediate component in order for a DAU **30** to have electrical continuity with an HME temperature and/or humidity cable connection **66d**.

The connections depicted in Fig. 47 with regards to an endotracheal tube are exemplar, with identical connections being provided by the invention should an alternative airway adjunct of the invention (e.g., a face mask, supraglottic airway, nasotracheal tube, or tracheal tube) be utilized.

Where electrical connections are described, alternative means of data exchange can be provided for while remaining within the teachings of the present invention. For example, fiber optics can be substituted in place of electrical contacts and/or conduits to provide the same means for data exchange between, for example, a DAU and convertible ventilator circuit patient manifold, and/or between a DPU and manual ventilator, and/or between a DPU and mechanical ventilator. Magnetic and/or optical sensors, and/or pressure and/or piezoelectric switches, or any combination thereof, could all be used to determine relative position of rotational and/or linear controls used on manual ventilators.

Accordingly, the components and functions as described provide a means for multiple new methodologies to utilize one or more combinations of components to partially or fully address one or more of the aforementioned unaddressed challenges in the field.

Referring to Fig. 1, the invention provides a method to operate a manual ventilator **70** that is compatible with and connected to a ventilator circuit **10** having an inspiratory limb **11**, expiratory limb **12**, and patient manifold **20**, whereby the patient manifold **20** is itself compatible with a combined DAU/DPU **30** + **42** having a display and audible emitter **42**, whereby the manual ventilation **70** is operated by sequentially squeezing the manual ventilator **70** with one or two hands, with such action constituting an inspiratory phase of a singular breath cycle, followed by releasing the grip on the manual ventilator **70** sufficient to allow it to re-expand while continuing to hold the manual ventilator **70**, with such action constituting an expiratory phase of a singular breath cycle.

Continuing to refer to Fig. 1, a preferred embodiment of the invention includes a manual ventilator **70** having one or more adjustable ventilatory parameters that are set by one or more controls, with said controls being of a rotational, linear or other type. This adds a further methodology for use of the invention relying on one or more adjustable ventilatory parameters to achieve substantial control over one or more ventilation delivery parameters during manual ventilation, with such methodology comprising: (1) a step to adjust one or more ventilatory parameters to the desired and/or prescribed setting; followed by (2) relying on the mechanism of the manual ventilator **70** providing for one or more adjustable ventilatory parameters to function in accordance with its design in order to deliver manual ventilation as previously described within the constraints of the one or more adjustable ventilatory parameters.

Still referring to Fig. 1, a preferred embodiment of the invention also includes a ventilator circuit **10** that contains an inspiratory limb **11** having an inspiratory particle, poison and pathogen filter **15**. This provides a further methodology for the invention of protecting patients from particles, poisons and/or pathogens that would otherwise represent a hazard during manual ventilation, comprising the steps of: (1) providing a preferred embodiment of ventilator circuit **10** as described that contains an inspiratory limb **11** having an inspiratory particle, poison and pathogen filter **15**; (2) compressing the manual ventilator **70** as previously described to achieve an inspiratory phase of a singular breath cycle, whereby inspiratory gas ejected by the manual ventilator **70** necessarily passes through the ventilator inspiratory connection **13**, followed by passage through the inspiratory particle, poison and pathogen filter **15**, whereby the inspiratory gas delivered to the patient is substantially free of particles, poisons and/or pathogens.

Referring again to Fig. 1, a preferred embodiment of the invention also includes a ventilator circuit **10** that contains an expiratory limb **12** having an expiratory pathogen filter **16.** This provides a further methodology for the invention of protecting providers and/or other proximate patients and/or persons from pathogens that, if present in the patient undergoing manual ventilation, could represent a hazard to said providers and/or other proximate patients and/or persons when exhaled into the ambient environment during expiratory phases of manual ventilation. The methodology comprises the steps of: (1) providing a preferred embodiment of the invention as described that contains a ventilator circuit **10** that contains an expiratory limb **12** having an expiratory pathogen filter **16**; (2) following delivery of a breath as previously described, releasing the grip on the manual ventilator **70** which causes the patient to passively exhale, whereby exhaled gas from the patient traverse the expiratory limb **12** and necessarily passes through the expiratory pathogen filter **16** and ventilator expiratory connection **14** prior to being vented into the ambient atmosphere.

A preferred embodiment of the invention also includes a ventilator circuit **10** whereby the inspiratory limb **11** and expiratory limb **12** are comprised of corrugated tubing or a similar tubing design that enables a methodology whereby the inspiratory limb **11** and expiratory limb **12** can be preferably in a compressed or retracted position as shown in Fig. 3 during use with a manual ventilator **70**, but that provides an ability for the inspiratory limb **11** and expiratory limb **12** to be changed to an expanded or lengthened position as shown in Fig. 4 during use with a mechanical ventilator (item **80** of Fig. 4). This constitutes a method whereby a ventilator circuit **10** as shown in Fig. 3 being a convertible ventilator circuit, whereby the convertible ventilator circuit **10** can be changeably modified between a compressed configuration for use with a manual ventilator **70** as shown in Fig. 3 and an elongated configuration for use with a mechanical ventilator (item **80** of Fig. 4), with said methodology achieved by applying manual traction with two hands between two points along the length of the ventilator circuit **10** resulting in expanding the convertible ventilator circuit **10**. Similarly, this constitutes a method whereby a ventilator circuit **10** as shown in Fig. 4 being a convertible ventilator circuit, whereby the convertible ventilator circuit **10** can be changeably modified between an elongated configuration for use with a mechanical ventilator **80** as shown in Fig. 4 and a compressed configuration for use with a manual ventilator (item **70** of Fig. 3), with said methodology achieved by applying manual compression with two hands between two points along the length of the ventilator circuit **10** resulting in compressing the convertible ventilator circuit **10**.

These methodologies for achieving conversion of the convertible ventilator circuit between substantially elongated and substantially compressed configurations can also be used to optimize the configuration of said convertible ventilator circuit even when no conversions between manual and mechanical ventilation are taking place. For example, a convertible ventilator circuit that is fully elongated, as may be generally optimal for use in a floor-standing mechanical ventilator, can be partially and/or fully compressed in the event the patient needs to be changed to a portable mechanical ventilator that is physically located a shorter distance from the patient. Similarly, a convertible ventilator circuit that is fully compressed, as may be generally optimal for use during manual ventilation, can be partially and/or fully elongated in the event the patient is being moved, such as extrication from an on-scene emergency through a narrow passageway whereby it would be impossible for a provider to walk beside the patient and must instead maintain a position ahead or behind the patient.

Referring to Fig. 2, the invention provides a methodology for supporting the weight of a DPU **40** during manual ventilation by providing a separate DPU housing **41** that can be physically connected to the ventilator inspiratory connection **13** that comprises that part of the inspiratory limb **11** that connects with an inspiratory limb connection **71** of a manual ventilator **70**. Separately, or additionally, a DPU housing **41** may also be physically connected to the ventilator expiratory connection (item **14** in Fig. 1) that connects with an expiratory limb connection (item **72** in Fig. 1) of a manual ventilator **70**. During the course of holding the manual ventilator **70** while delivering manual ventilation, the user also holds and supports the weight of the DPU **40** and that portion of the convertible ventilator circuit **10** that is above the level of the convertible ventilator circuit patient manifold **20**. This methodology enables the weight of the DPU **40** to be minimized, contributing to minimized forces exerted on a patient airway connected to the convertible ventilator circuit patient manifold **20**.

Referring to Figs. 1, 2 and 3, the invention provides a methodology of preparing a convertible ventilator circuit (item **10** in Fig. 2) having a patient manifold **20** for use with a compatible manual ventilator **70**, DAU **30**, and DPU **40**, comprising the steps of: (1) providing all the components shown in Fig. 1; (2) removing any packaging that may keep components isolated from potential contaminants prior to use; (3) referring to Fig. 1, connecting the ventilator inspiratory connection **13** to the inspiratory limb connection **71**; (4) connecting the ventilator expiratory connection **14** to the expiratory limb connection **72**; (5) connecting the DPU (item **40** in Fig. 2) to the ventilator inspiratory connection **13** and ventilator expiratory connection **14**; and (6) sliding the DAU (item **30** in Fig. 2) over the convertible ventilator circuit patient manifold **20** so that, at the completion of steps 1-6, the invention is in the configuration as shown in Fig. 3.

In the event a combination DAU + DPU (item **30+40** in Fig. 1) is being used instead of the preferred embodiment as previously described, then step 5 of the methodology previously described may be omitted, with step 6 simultaneously resulting in the installation of the DAU + DPU (item **30+40** in Fig. 1).

Referring to Fig. 3, once components of the combination are prepared as previously described, the methodology comprises a further step of powering on the DPU **40** and following visual and/or audible instructions conveyed to the user by means of the display and audible emitter **42**. During the power on process, the DAU **30** and/or DPU **40** can perform a series of self-checks to confirm appropriate function of internal components, and also test functional electronic connectivity with any sensors that are fitted to the DAU **30**.

Referring to Figs. 3 and 21, the methodology may comprise a further step of requiring the user to interact with the DPU (**40** in Fig. 3) as part of a process confirming the presence of sensors that are fitted to the DAU (**30** in Fig. 21). For example, the DPU (**40** in Fig. 3) may inform the user that it is sensing: (1) the presence of an inspiratory zone sensor **37a** that, for example, is a pressure sensor; (2) an expiratory zone sensor **37b** that, for example, is also a pressure sensor; (3) a patient zone sensor **37c** that is, for example, also a pressure sensor; (4) a patient zone sensor **37c** that is, for example, a carbon dioxide concentration sensor; and (5) the presence of one or more auxiliary sensors **37d** that may also be pressure or other sensors. Referring to Fig. 22, the methodology may comprise further steps whereby the DPU (**40** in Fig. 3) informs the user that it is also sensing: (6) a proximal flow sensor **38a**; and a (7) a distal flow sensor **38b** that works in conjunction with a flow sensor screen **28** of the convertible ventilator circuit patient manifold **20** that forms a combination comprising a flow sensor.

In the event sensors are placed, but are not among those sensors that the DPU confirms as being actively sensed by the DAU, then then the methodology provides for an exemplary troubleshooting procedure comprising: (1) removing non-detected sensors off the DAU (**30** in Figs. 21 and/or 22); (2) re-installing the non-detected sensors back on the DAU (**30** in Figs. 21 and/or 22); (3) determining as previously described whether the DAU now detects the re-installed sensor(s); and (4) if not, substituting the non-detected sensor(s) with one or more replacement sensor(s).

As previously described and shown in Figs. 33 and 34, the invention provides a preferred embodiment of manual ventilator **70** that has one or more controls for one or more ventilatory parameters, with said manual ventilator **70** having the ability to be interfaced with a DPU **40** via the connectivity as previously described between: (1) the DPU; (2) the convertible ventilator circuit inspiratory and expiratory limb connections; and (3) the preferred embodiment of manual ventilator. Referring to Figs. 27a and 27b, the invention comprises a preferred methodology step whereby a signal is sent from the DPU to the control setting electrode **74b** in the manual ventilator, whereby the control setting electrode **74b** enables the DPU to ascertain which type and/or size of manual ventilator is physically connected. This can be achieved by one or more generic methodologies, including the control setting electrode **74b** modifying the signal sent from the DPU in one or more ways that are specific to a control setting electrode **74b** inserted into a specific type and/or size manual ventilator. The methodology can then include a step whereby the DPU informs the user that it is actively detecting the presence of the manual ventilator, with the user confirming the presence of the type and/or size of the manual ventilator.

In the event the manual ventilator is not detected by the DPU, and/or the DPU is detecting a manual ventilator of type and/or size other than that which is actually connected, then then the methodology provides for a troubleshooting procedure comprising: (1) removing the manual ventilator through reversal of steps previously described; (2) re-installing the manual ventilator back on the DPU as previously described; (3) determining whether the DPU has obtained ability to detect the re-installed manual ventilator; and (4) if not, substituting the non-detected manual ventilator with a replacement manual ventilator. In the event the DPU is detecting the presence of a manual ventilator, but the type and/or size of the manual ventilator being detected is not that which is actively connected, then the DPU may provide an override function whereby the user can manually enter the correct type and/or size of manual ventilator connected.

The methodology may further provide a process prior to use whereby the user confirms proper function of one or more ventilator parameter controls on a preferred embodiment of manual ventilator. Referring to Figs. 29a and 29b, the DPU may prompt the user to confirm the current parameter setting stop **74a** of a rotational parameter adjustor **74,** followed by a prompt for the user to adjust the rotational parameter adjustor **74** to a new parameter setting stop **74a**, after which the DPU prompts the user to confirm the a new parameter setting stop **74a** as a means to confirm proper DPU detection of the rotational parameter control.

In the event the rotational parameter adjustor on a manual ventilator is not being properly detected by the DPU, then the methodology provides for a troubleshooting procedure comprising (1): re-prompting from the DPU confirming whether the proper type/size of manual ventilator is present; if yes, (2) removing the manual ventilator; (3) re-installing the manual ventilator back on the DPU; (4) repeating steps as previously described to determine whether the DPU is now able to properly confirm settings of the rotational parameter adjustor; and, if not, (5) providing an override function whereby the user can manually enter the correct settings into the DPU for each parameter setting stop of the rotational adjustor.

The methodologies just described for confirming a DPU is properly detecting settings of a rotational control, and troubleshooting procedures in the event it is not, is wholly applicable to a linear control system as previously described and shown in Figs. 31a, 31b, 32a, and 32b, or other manual control that may be installed on a manual ventilator and instrumented to a DAU and/or DPU.

As previously described and shown in Figs. 41 - 43, the invention provides multiple preferred embodiments of airway adjuncts that all have enhancements, as previously described and shown in Figs. 35 - 37, that enable the said airway adjuncts to have the ability to be interfaced with a DAU via the connectivity as previously described between: (1) the DAU; (2) the convertible ventilator circuit patient manifold; and (3) the preferred embodiments of airway adjuncts. Referring to Fig. 35, the airway adjunct connector **68** includes an airway adjunct identification chip **69e** whereby, as an additional step in the methodology preparing the system for use with a manual ventilator, a signal is sent from the DPU (**40** in Fig. 3) to the DAU (**30** in Fig. 3) via a wired connection (**36** in Fig. 3) and, from there, to the airway adjunct identification chip (**69e** in Fig. 35), whereby the airway adjunct identification chip **69e** enables the DPU to ascertain which type and/or size of airway adjunct is physically connected. This can be achieved by one or more methodologies, including the airway adjunct identification chip **69e** modifying the signal sent from the DPU in one or more ways that are specific to an airway adjunct identification chip **69e** inserted into a specific type and/or size of airway adjunct. For example, if the airway adjunct is a face mask, the particular type and size (e.g., adult or pediatric or infant) could be ascertained by the DPU. The additional step during preparation can then include a step whereby the DPU informs the user that it is actively detecting the presence of the airway adjunct of a particular type and size, with the user confirming the presence of the type and/or size of the airway adjunct connected.

In the event the airway adjunct is not detected by the DPU, and/or the DPU is detecting an airway adjunct of type and/or size other than that which is actually connected, then then the methodology provides for a troubleshooting procedure comprising: (1) removing the airway adjunct; (2) re-installing the airway adjunct; (3) determining whether the DPU has obtained ability to correctly detect the re-installed airway adjunct; and (4) if not, substituting the non-detected or erroneously detected airway adjunct with a replacement airway adjunct. In the event the DPU is detecting the presence of an airway adjunct, but the type and/or size of the airway adjunct being detected is not that which is actually connected, then the DPU may provide an override function whereby the user can manually enter the correct type and/or size of airway adjunct connected.

The methodology for preparing the system for use with a manual ventilator may provide further steps that may be particularly suitable under certain circumstances of anticipated use. For example, the DPU may include steps requiring the user performing the pre-use and setup procedure to enter in information identifying themselves (either by name, a user number, and/or other means). The methodology may include a step requiring the user to enter into the DPU one or more lot numbers of ventilator circuit, manual ventilator, and/or airway adjuncts that are connected prior to use. Based on the detailed description herein, one or more additional steps as part of preparing the ventilator circuit for use with a manual ventilator can be ascertained by one of ordinary skill in the art while remaining within the intended scope of the present invention.

The methodology for preparing the system for use with a manual ventilator may provide a further step whereby the DPU is connected to a power source to ensure that, upon being put into service, the system has adequate battery charge.

Upon being pressed into service for use on a specific patient, the methodology provides a step whereby the DPU requires the user to enter certain essential patient demographics. For example, the DPU may require an approximate patient height to be entered and whether the patient is male or female. Alternatively, the DPU may obtain this information through an interfaced device, such as an electronic health record, patient data chip, or other means.

Upon being pressed into service for use on a specific patient, the methodology provides a step where the DPU assigns a control number or other means to enable users to link data obtained, stored, archived and/or transmitted by the DPU to a specific patient without having to utilize individually identifying information of the patient. For example, a control number assigned by the DPU can be provided by the DPU to a user, for the user to subsequently enter the control number into a confidential patient record enabling an authorized person having access to a confidential patient record to match stored, archived and/or transmitted DPU data to a specific patient.

As previously described, the invention provides a ventilator circuit that is compatible with both manual ventilators and mechanical ventilators. Accordingly, in addition to the methodologies previously described to use the invention with a manual ventilator, the invention provides corresponding methodologies to use the ventilator circuit with mechanical ventilators that are generally the same for connecting and preparing the ventilator circuit to a mechanical ventilator instead of a manual ventilator.

For example, referring to Fig. 4, the invention comprises a method of connecting a ventilator circuit **10** having a patient manifold **20**, DAU **30** and DPU **40** with a mechanical ventilator **80**. The method includes steps as previously described and shown in Fig. 1 for connecting a ventilator circuit inspiratory limb **11** and expiratory limb **12** to a manual ventilator **70**, which are identical for connecting a ventilator circuit **10** to a mechanical ventilator (**80** in Fig. 4).

Fig. 4 also shows an exemplar mechanical ventilator **80** having an interface receptacle **87**. A further methodology step comprises placing a DPU **40** into an interface receptacle **87** enabling the DPU to transfer data between a mechanical ventilator utilizing one or more generic methodologies for data exchange between two electronic devices, while also obtaining an electrical charge that ensures the DPU **40** has sufficient battery power to operate upon removal from the mechanical ventilator receptacle **87**, utilizing one or more generic methodologies for an electrical charge to be provided from one electronic device to another.

Referring still to Fig. 4, a wired connection **36** provides data exchange between a DAU **30** and DPU **40**. A further methodology step comprises utilizing data from the DAU **30** by a mechanical ventilator **80** for purposes of supplanting and/or supplementing sensor data that may be obtained from one or more sensors contained within said mechanical ventilator **80**. For example, referring to Fig. 22, a mechanical ventilator may utilize data from a DAU **30** obtained from one or more sensors interfaced with a convertible ventilator circuit patient manifold **20** to provide supplemental and/or enhanced accuracy based on said sensors being in closer proximity to a patient compared to one or more sensors that may be internally contained within said mechanical ventilator and subject to dampening, error and/or artifacts arising from the higher aggregate volume of sampled breathing gas contained in an inspiratory and/or expiratory limb prior to measurement by one or more sensors contained within a mechanical ventilator.

The invention also comprises a method for a DPU to determine the end of an inspiratory phase of a singular breath cycle during manual ventilation, comprising: (1) providing a DPU (**40** in Fig. 3) capable of measuring and processing gas flow measurements during inspiration; (2) providing a DAU (**30** in Fig. 3) interfaced with the said DPU (**40** in Fig. 3), with said DAU also interfaced with a proximal flow sensor (**38a** in Fig. 22) and a distal flow sensor (**38b** in Fig. 22), whereby data from the proximal flow sensor (**38a** in Fig. 22) and a distal flow sensor (**38b** in Fig. 22) is used by the DPU to calculate gas flow, whereby the DPU determines whether gas flow is positive in a direction toward the patient, which such directional flow being specific to identifying an inspiratory phase of a singular breath cycle; and (3) a determination of the DPU that an inspiratory phase has ended when the flow measurement as previously described reaches zero.

Similarly, the invention comprises a method for a DPU to determine the end of an expiratory phase of a singular breath cycle during manual ventilation, comprising: (1) providing a DPU (**40** in Fig. 3) capable of measuring and processing gas flow measurements during expiration; (2) providing a DAU (**30** in Fig. 3) interfaced with the said DPU (**40** in Fig. 3), with said DAU also interfaced with a proximal flow sensor (**38a** in Fig. 22) and a distal flow sensor (**38b** in Fig. 22), whereby data from the proximal flow sensor (**38a** in Fig. 22) and a distal flow sensor (**38b** in Fig. 22) is used by the DPU to calculate gas flow, whereby the DPU determines whether gas flow is negative in a direction from the patient, which such directional flow being specific to identifying an expiratory phase of a singular breath cycle; and (3) a determination of the DPU that an expiratory phase has ended when the flow measurement from the flow sensor reaches zero, and remains zero, until immediately prior to a point when positive flow is first detected at the initiation of the inspiratory phase of the following breath cycle.

Another methodology provided by the invention is the ability to perform an inspiratory pause maneuver during manual ventilation, comprising: (1) providing a ventilator circuit (**10** in Fig. 3) having a patient manifold (**20** in Fig. 3) that itself has an active expiratory valve (**25a** in Fig. 6) that is capable of being triggered by a DAU (**30** in Fig. 3) as previously described; (2) providing a DPU (**40** in Fig. 3) having the capability for detecting the end of an inspiratory phase of a singular inspiratory cycle during manual ventilation as previously described; (3) providing a manual ventilator (**70** in Fig. 3) compatible with and interfaced to, said ventilator circuit (**10** in Fig. 3); (4) utilizing said manual ventilator (**70** in Fig. 3) to deliver a breath as previously described; (5) the activation of the active expiratory valve (**25a** in Fig 6) by the DPU (**40** in Fig. 3) upon DPU determination of the end of the inspiratory phase of a singular breath cycle as previously described; (6) elapse of a pause of designated length whereby gas flow is able to settle in the immediate area of the one or more patient zone sensors (**37c** in Fig. 22); and (7) upon expiration of the designated pause, enabling the DPU (**40** in Fig. 3) to deactivate the active expiratory valve (**25a** in Fig. 6), thereby allowing the expiratory phase of a singular breath cycle to commence.

The invention provides an additional methodology providing a new ability to measure driving pressure during manual ventilation, comprising: (1) providing a ventilator circuit (**10** in Fig. 3) having a patient manifold (**20** in Fig. 3) that itself has an active expiratory valve (**25a** in Fig. 6) that is capable of being triggered by a DAU (**30** in Fig. 3) as previously described; (2) providing a DPU (**40** in Fig. 3) having the capability for detecting the end of an inspiratory phase of a singular inspiratory cycle during manual ventilation as previously described; (3) providing a convertible ventilator circuit patient manifold (**20** in Fig. 22) having a patient zone sensor (**37c** in Fig. 22) that is a pressure sensor; (4) providing a DPU (**40** in Fig. 3) measuring pressure from a patient zone sensor (**37c** in Fig. 22) that is a pressure sensor at the end of the expiratory phase, such that this pressure measurement constitutes a measurement of end-expiratory pressure; (5) providing a DPU (item **40** in Fig. 3) measuring pressure from a patient zone sensor (**37c** in Fig. 22) that is a pressure sensor at the end of an inspiratory pause maneuver, such that this pressure measurement constitutes a measurement of plateau pressure; and (6) providing a DPU (**40** in Fig. 3) calculating plateau pressure minus end-expiratory pressure, such that this constitutes a measurement for driving pressure obtained during manual ventilation.

The invention provides a new methodology for measuring end-tidal carbon dioxide concentration during manual ventilation, comprising: (1) providing a ventilator circuit (**10** in Fig. 3) having a patient manifold (**20** in Fig. 3), with said ventilator circuit patient manifold having a patient zone sensor (**37c** in Fig. 22) that is a capnography sensor; and (2) providing a DPU (**40** in Fig. 3) measuring carbon dioxide concentration from a patient zone sensor (**37c** in Fig. 22) that is a capnography sensor, with said measurement occurring at the end of an expiratory phase of a singular breath cycle during manual ventilation as previously described.

An additional methodology for measuring inspiratory oxygen concentration is provided by the invention, comprising: (1) providing a ventilator circuit (**10** in Fig. 3) having a patient manifold (**20** in Fig. 3), with said ventilator circuit patient manifold having a patient zone sensor (**37c** in Fig. 22) that is an oxygen sensor; (2) providing a manual ventilator (**70** in Fig. 3) compatible with and interfaced to, said ventilator circuit (**10** in Fig. 3); (3) utilizing said manual ventilator (**70** in Fig. 3) to deliver a breath as previously described; and (4) providing a DPU (**40** in Fig. 3) measuring oxygen concentration from a patient zone sensor (**37c** in Fig. 22) that is an oxygen sensor during the inspiratory phase of a singular breath cycle generated from a manual ventilator.

A methodology for measuring end-expiratory oxygen concentration is also provided by the invention, comprising: (1) providing a ventilator circuit (**10** in Fig. 3) having a patient manifold (**20** in Fig. 3), with said ventilator circuit patient manifold having a patient zone sensor (**37c** in Fig. 22) that is an oxygen sensor; (2) providing a manual ventilator (**70** in Fig. 3) compatible with and interfaced to, said ventilator circuit (**10** in Fig. 3); (3) utilizing said manual ventilator (**70** in Fig. 3) to deliver a breath as previously described; (4) providing a DPU (**40** in Fig. 3) capable of determining the end of an expiratory phase of a singular breath cycle as previously described; and (5) providing a DPU that measures oxygen concentration from a patient zone sensor (**37c** in Fig. 22) that is an oxygen sensor, with said measurement occurring at the end of an expiratory phase of a singular breath cycle during manual ventilation.

The invention provides a methodology for measuring plateau pressure during mechanical ventilation with enhanced accuracy, comprising: (1) providing a ventilator circuit (**10** in Fig. 4) having a patient manifold (**20** in Figs. 4) that itself has an active expiratory valve (**25a** in Fig. 6) capable of being triggered by a DAU (**30** in Fig. 4) as previously described; (2) providing a DPU (**40** in Fig. 4) having the capability for detecting the end of an inspiratory phase of a singular inspiratory cycle during mechanical ventilation as previously described, or, in place of a DPU, an internal capability of a mechanical ventilator electronically interfaced with said DPU to detect the end of an inspiratory phase of a singular inspiratory cycle; (3) providing a patient zone sensor (**37c** in Fig. 22) that is a pressure sensor; (4) providing said DPU (**40** in Fig. 3) that is also capable of measuring pressure from a patient zone sensor (**37c** in Fig. 22) that is a pressure sensor at the end of the expiratory phase, such that this pressure measurement constitutes a measurement of end-expiratory pressure, or, in place of a DPU, an internal capability of a mechanical ventilator electronically interfaced with said DPU to measure pressure from a patient zone sensor (**37c** in Fig. 22) that is a pressure sensor at the end of the expiratory phase; (5) providing the said a DPU (**40** in Fig. 3) capable of measuring pressure from a patient zone sensor (**37c** in Fig. 22) that is a pressure sensor, with such pressure measurement occurring at the end of an inspiratory pause maneuver, such that this pressure measurement constitutes a measurement of plateau pressure, or, in place of a DPU, an internal capability of a mechanical ventilator electronically interfaced with said DPU to measure pressure from a patient zone sensor (**37c** in Fig. 22) that is a pressure sensor at the end of an inspiratory pause maneuver, such that this pressure measurement constitutes a measurement of plateau pressure, such that the plateau pressure measured from a patient zone sensor (**37c** in Fig. 22) that is a pressure sensor is measuring pressure of an aggregate of gases limited to the deadspace area (**18** in Fig. 9) of the ventilator circuit patient manifold (**20** in Fig. 9), whereby this said deadspace area is substantially less than the comparable deadspace area (**18** in Fig. 10) of a conventional ventilator circuit not having a patient manifold, whereby this deadspace area (**18** in Fig. 10) is a comparably larger aggregate of gases contained in the entirety of the ventilator circuit to include the inspiratory limb (**11** in Fig. 10) and expiratory limb (**12** in Fig. 10), whereby the compressibility of gases contained in said inspiratory limb (**11** in Fig. 10) and expiratory limb (**12** in Fig. 10) materially reduces and/or dampens the plateau pressure measurement, such that this reduction and/or dampening introduces error to the plateau pressure measured by an internal sensor located inside the mechanical ventilator (**80** in Fig. 10).

The above-described techniques can be implemented in digital and/or analog electronic circuitry, or in computer hardware, firmware, software, or in combinations of them. The implementation can be as a computer program product, i.e., a computer program tangibly embodied in a machine-readable storage device, for execution by, or to control the operation of, a data processing apparatus, e.g., a programmable processor, a computer, and/or multiple computers. A computer program can be written in any form of computer or programming language, including source code, compiled code, interpreted code and/or machine code, and the computer program can be deployed in any form, including as a stand-alone program or as a subroutine, element, or other unit suitable for use in a computing environment. A computer program can be deployed to be executed on one computer or on multiple computers at one or more sites. The computer program can be deployed in a cloud computing environment (e.g., Amazon^{®} AWS, Microsoft^{®} Azure, IBM^{®}).

Method steps can be performed by one or more processors executing a computer program to perform functions of the invention by operating on input data and/or generating output data. Method steps can also be performed by, and an apparatus can be implemented as, special purpose logic circuitry, e.g., a FPGA (field programmable gate array), a FPAA (field-programmable analog array), a CPLD (complex programmable logic device), a PSoC (Programmable System-on-Chip), ASIP (application-specific instruction-set processor), or an ASIC (application-specific integrated circuit), or the like. Subroutines can refer to portions of the stored computer program and/or the processor, and/or the special circuitry that implement one or more functions.

Processors suitable for the execution of a computer program include, by way of example, special purpose microprocessors specifically programmed with instructions executable to perform the methods described herein, and any one or more processors of any kind of digital or analog computer. Generally, a processor receives instructions and data from a read-only memory or a random access memory or both. The essential elements of a computer are a processor for executing instructions and one or more memory devices for storing instructions and/or data. Memory devices, such as a cache, can be used to temporarily store data. Memory devices can also be used for long-term data storage. Generally, a computer also includes, or is operatively coupled to receive data from or transfer data to, or both, one or more mass storage devices for storing data, e.g., magnetic, magneto-optical disks, or optical disks. A computer can also be operatively coupled to a communications network in order to receive instructions and/or data from the network and/or to transfer instructions and/or data to the network. Computer-readable storage mediums suitable for embodying computer program instructions and data include all forms of volatile and non-volatile memory, including by way of example semiconductor memory devices, e.g., DRAM, SRAM, EPROM, EEPROM, and flash memory devices; magnetic disks, e.g., internal hard disks or removable disks; magneto-optical disks; and optical disks, e.g., CD, DVD, HD-DVD, and Blu-ray disks. The processor and the memory can be supplemented by and/or incorporated in special purpose logic circuitry.

To provide for interaction with a user, the above described techniques can be implemented on a computing device in communication with a display device, e.g., a CRT (cathode ray tube), plasma, or LCD (liquid crystal display) monitor, a mobile device display or screen, a holographic device and/or projector, for displaying information to the user and a keyboard and a pointing device, e.g., a mouse, a trackball, a touchpad, or a motion sensor, by which the user can provide input to the computer (e.g., interact with a user interface element). Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, and/or tactile input.

The above-described techniques can be implemented in a distributed computing system that includes a back-end component. The back-end component can, for example, be a data server, a middleware component, and/or an application server. The above described techniques can be implemented in a distributed computing system that includes a front-end component. The front-end component can, for example, be a client computer having a graphical user interface, a Web browser through which a user can interact with an example implementation, and/or other graphical user interfaces for a transmitting device. The above described techniques can be implemented in a distributed computing system that includes any combination of such back-end, middleware, or front-end components.

The components of the computing system can be interconnected by transmission medium, which can include any form or medium of digital or analog data communication (e.g., a communication network). Transmission medium can include one or more packet-based networks and/or one or more circuit-based networks in any configuration. Packet-based networks can include, for example, the Internet, a carrier internet protocol (IP) network (e.g., local area network (LAN), wide area network (WAN), campus area network (CAN), metropolitan area network (MAN), home area network (HAN)), a private IP network, an IP private branch exchange (IPBX), a wireless network (e.g., radio access network (RAN), Bluetooth, near field communications (NFC) network, Wi-Fi, WiMAX, general packet radio service (GPRS) network, HiperLAN), and/or other packet-based networks. Circuit-based networks can include, for example, the public switched telephone network (PSTN), a legacy private branch exchange (PBX), a wireless network (e.g., RAN, code-division multiple access (CDMA) network, time division multiple access (TDMA) network, global system for mobile communications (GSM) network), and/or other circuit-based networks.

Information transfer over transmission medium can be based on one or more communication protocols. Communication protocols can include, for example, Ethernet protocol, Internet Protocol (IP), Voice over IP (VOIP), a Peer-to-Peer (P2P) protocol, Hypertext Transfer Protocol (HTTP), Session Initiation Protocol (SIP), H.323, Media Gateway Control Protocol (MGCP), Signaling System #7 (SS7), a Global System for Mobile Communications (GSM) protocol, a Push-to-Talk (PTT) protocol, a PTT over Cellular (POC) protocol, Universal Mobile Telecommunications System (UMTS), 3GPP Long Term Evolution (LTE) and/or other communication protocols.

Devices of the computing system can include, for example, a computer, a computer with a browser device, a telephone, an IP phone, a mobile device (e.g., cellular phone, personal digital assistant (PDA) device, smart phone, tablet, laptop computer, electronic mail device), and/or other communication devices. The browser device includes, for example, a computer (e.g., desktop computer and/or laptop computer) with a World Wide Web browser (e.g., Chrome^{™} from Google, Inc., Microsoft^{®} Internet Explorer^{®} available from Microsoft Corporation, and/or Mozilla^{®} Firefox available from Mozilla Corporation). Mobile computing device include, for example, a Blackberry^{®} from Research in Motion, an iPhone^{®} from Apple Corporation, and/or an Android^{™}-based device. IP phones include, for example, a Cisco^{®} Unified IP Phone 7985G and/or a Cisco^{®} Unified Wireless Phone 7920 available from Cisco Systems, Inc.

The above-described techniques can be implemented using supervised learning and/or machine learning algorithms. Supervised learning is the machine learning task of learning a function that maps an input to an output based on example input-output pairs. It infers a function from labeled training data consisting of a set of training examples. Each example is a pair consisting of an input object and a desired output value. A supervised learning algorithm or machine learning algorithm analyzes the training data and produces an inferred function, which can be used for mapping new examples.

Comprise, include, and/or plural forms of each are open ended and include the listed parts and can include additional parts that are not listed. And/or is open ended and includes one or more of the listed parts and combinations of the listed parts.

One skilled in the art will realize the subject matter may be embodied in other specific forms without departing from the spirit or essential characteristics thereof. The foregoing embodiments are therefore to be considered in all respects illustrative rather than limiting of the subject matter described herein.
The application contains the following embodiments:
[1]. A system for artificial ventilation comprising:
   a convertible ventilator circuit comprising at least one inspiratory filter and at least one expiratory filter,
   said convertible ventilator circuit compatible with and configured to be used with either a manual ventilator or a mechanical ventilator, wherein the convertible ventilator circuit is configured to convert between manual ventilation use and mechanical ventilation use;
   a patient manifold comprising one or more sensor ports; and
   a data acquisition unit comprising one or more sensors configured to interface with the one or more sensor ports of the patient manifold, wherein the data acquisition unit is configured to receive electronic data associated with transmission of respiratory gases through the convertible ventilator circuit.
[2] The system of embodiment [1], wherein the patient manifold comprises one or more valves configured to direct or pause inspiratory gases to the convertible ventilator circuit and direct or pause expiratory gases from the convertible ventilator circuit.
[3] The system of embodiment [1], wherein the data acquisition unit comprises one or more additional sensors that interface with one or more sensor ports of an airway adjunct in physical contact with a patient.
[4] The system of embodiment [3], wherein the one or more additional sensors comprise at least one of:
   a flow sensor;
   a pressure sensor;
   a humification sensor;
   a temperature sensor; or
   a partial pressure gas sensor for at least one of oxygen, carbon dioxide, or nitrogen.
[5] The system of embodiment [1], further comprising a data processing unit configured to:
   process the electronic data received by the data acquisition unit;
   store the electronic data received by the data acquisition unit; and
   electronically transmit the electronic data received by the data acquisition unit to a neighboring device.
[6] The system of embodiment [5], wherein the data processing unit is housed in a single housing with the data acquisition unit.
[7] The system of embodiment [5], wherein the data processing unit is housed in a housing of the neighboring device.
[8] The system of embodiment [5], wherein the neighboring device comprises one of the mechanical ventilator, a bedside monitor, a cardiac monitor or defibrillator, an electronic health record device, or a capnography device.
[9] The system of embodiment [5], further comprising a manual ventilator compatible with the convertible ventilator circuit, said manual ventilator comprising one or more settings configured to control one or more characteristics of manual ventilation.
[10] The system of embodiment [9], wherein the characteristics of manual ventilation comprise at least one of tidal volume or positive end-expiratory pressure.
[11] A system for artificial ventilation comprising:
   a convertible ventilator circuit comprising at least one inspiratory filter and at least one expiratory filter,
   wherein the convertible ventilator circuit comprises at least one inspiratory tube segment and at least one expiratory tube segment configured to contain breathing gas transmission;
   said convertible ventilator circuit compatible with and configured to be used with either a manual ventilator or a mechanical ventilator, wherein the convertible ventilator circuit is configured to convert between manual ventilation use and mechanical ventilation use;
   a data acquisition unit configured to receive electronic data associated with transmission of respiratory gases through the convertible ventilator circuit and electronic data from one or more identification chips of the artificial ventilation system; and
   a data processing unit configured to:
      process the electronic data received by the data acquisition unit;
      store the electronic data received by the data acquisition unit; and
      electronically transmit the electronic data received by the data acquisition unit to a neighboring device.
[12] The system of embodiment [11], further comprising a manual ventilator compatible with the convertible ventilator circuit, said manual ventilator comprising an inspiratory port configured to communicatively couple to the at least one inspiratory tube segment of the convertible ventilator circuit and an expiratory port configured to communicatively couple to the at least one expiratory tube segment of the convertible ventilator circuit.
[13] The system of embodiment [12], wherein the data acquisition unit is configured to electronically detect the manual ventilator when communicatively coupled to at least one of the at least one inspiratory tube segment or the at least one expiratory tube segment of the convertible ventilator circuit.
[14] The system of embodiment [11], further comprising a mechanical ventilator compatible with the convertible ventilator circuit, said mechanical ventilator comprising an inspiratory port configured to communicatively couple to the at least one inspiratory tube segment of the convertible ventilator circuit and an expiratory port configured to communicatively couple to the at least one expiratory tube segment of the convertible ventilator circuit.
[15] The system of embodiment [14], wherein the data acquisition unit is configured to electronically detect the mechanical ventilator when communicatively coupled to at least one of the at least one inspiratory tube segment or the at least one expiratory tube segment of the convertible ventilator circuit.
[16] The system of embodiment [11], further comprising one or more airway adjuncts compatible with the convertible ventilator circuit, wherein the one or more airway adjuncts comprise at least one of a face mask, a supraglottic airway, or an endotracheal tube, wherein the data acquisition unit is configured to electronically detect the one or more airway adjuncts when communicatively coupled to a patient port of the convertible ventilator circuit.
[17] The system of embodiment [16], wherein the one or more airway adjuncts comprise one or more identification chips comprising stored parameters, wherein the stored parameters comprise at least one of airway adjunct size, shape, manufacturing lot number, cuff size, cuff shape, or previously recorded data indicating use on a prior patient.
[18] The system of embodiment [16], wherein the one or more airway adjuncts comprise one or more cuff pressure tubes configured to monitor cuff pressure and compatible with the data acquisition unit.
[19] The system of embodiment [11], further comprising a cap configured to cover and seal a patient port of the convertible ventilator circuit, wherein the data acquisition unit is configured to electronically detect the cap when communicatively coupled to the patient port of the convertible ventilator circuit.
[20] The system of embodiment [11], further comprising a heat-moisture exchanger compatible with the convertible ventilator circuit and comprising one or more identification chips comprising stored parameter, wherein the stored parameters comprise at least one of heat-moisture exchanger size, shape, manufacturing lot number, or previously recorded data indicating use on a prior patient.
[21] The system of embodiment [20], wherein the heat-moisture exchanger comprises at least one of one or more pressure sensors or one or more temperature sensors compatible with the data acquisition unit..
[22] The system of embodiment [21], wherein the at least one of the one or more pressure sensors or the one or more temperature sensors comprise ports emerging from a side of the heat-moisture exchanger.
[23] A system for performing an inspiratory pause maneuver during manual ventilation, the system comprising:
   a convertible ventilator circuit communicatively coupled to a manual ventilator and having a patient manifold comprising an active expiratory valve;
   a data processing unit comprising:
      means for determining an end of an inspiratory phase of a breath to a patient using the manual ventilator communicatively coupled to the convertible ventilator circuit;
      means for activating the active expiratory valve of the patient manifold of the convertible ventilator circuit at the end of the inspiratory phase of the breath;
      means for determining that a pause of designated length has elapsed; and
      means for deactivating the active expiratory valve of the patient manifold of the convertible ventilator circuit after the pause of designated length has elapsed.
[24] The system of embodiment [23], wherein the data processing unit further comprises:
   means for measuring a first pressure using a patient zone sensor of the convertible ventilator circuit corresponding to the end of the inspiratory phase of the breath;
   means for measuring a second pressure using the patient zone sensor of the convertible ventilator circuit after the pause of designated length has elapsed; and
   means for calculating a driving pressure based on the first pressure and the second pressure.
[25] The system of embodiment [24], wherein the data processing unit further comprises:
   means for measuring an oxygen concentration using the patient zone sensor of the convertible ventilator circuit during the inspiratory phase of the breath.
[26] The system of embodiment [24], wherein the data processing unit further comprises:
   means for determining, an end of an expiratory phase of the breath; and
   means for measuring an oxygen concentration using the patient zone sensor of the ventilator circuit corresponding to the end of the expiratory phase of the breath.

## Claims

1. A patient-side respiratory data acquisition system for use during artificial ventilation of a patient, comprising:
a patient manifold comprising a gas passageway configured for fluid communication with an airway adjunct of the patient and configured for fluid connection with a ventilator;
at least one respiratory sensing arrangement associated with the patient manifold, the at least one respiratory sensing arrangement comprising at least one of:
(a) one or more sensor ports providing access to the gas passageway and one or more respiratory sensors configured to interface with the one or more sensor ports; or
(b) a flow-sensing structure associated with the gas passageway and configured to enable determination of respiratory gas flow through the patient manifold;
a data acquisition unit configured to acquire respiratory data using the at least one respiratory sensing arrangement, the respiratory data comprising one or more directly sensed respiratory measurements; and
a data processing unit operably coupled to the data acquisition unit and configured to process and retain the respiratory data as a patient respiratory data record.

2. The patient-side respiratory data acquisition system of claim 1, wherein the patient manifold and the at least one respiratory sensing arrangement are configured for use with different ventilators such that respiratory data associated with ventilation by each of the different ventilators is acquirable using the same patient-side respiratory sensing arrangement independent of respiratory sensing provided by the different ventilators.

3. The patient-side respiratory data acquisition system of claim 1, wherein the respiratory data further comprises one or more respiratory parameters determined from the directly sensed respiratory measurements.

4. The system of any preceding claim, wherein the data processing unit is housed separately from the data acquisition unit.

5. The system of any preceding claim, wherein the data processing unit is housed in a single housing with the data acquisition unit.

6. The system of claim 1, wherein the at least one respiratory sensing arrangement comprises the one or more sensor ports and the one or more respiratory sensors configured to interface with the one or more sensor ports, optionally wherein the one or more respiratory sensors are collectively configured to acquire respiratory data during both inspiration and expiration and further optionally wherein at least one of the one or more sensor ports is positioned in proximity to an airway-adjunct receptacle of the patient manifold such that a respiratory sensor interfacing with the sensor port is in fluid communication with respiratory gases during both inspiration and expiration.

7. The system of any preceding claim, wherein the at least one respiratory sensing arrangement comprises the flow-sensing structure configured to enable determination of respiratory gas flow through the patient manifold in both inspiratory and expiratory directions, optionally wherein the flow-sensing structure comprises a flow sensor screen positioned in the gas passageway of the patient manifold.

8. The system of any preceding claim, wherein the respiratory data comprises one or more of respiratory gas pressure, respiratory gas flow, respiratory gas volume, concentration or partial pressure of one or more respiratory gases, or one or more respiratory-cycle timing parameters, optionally, wherein at least one of the respiratory gas flow or the respiratory gas volume is determined based on the one or more directly sensed respiratory measurements.

9. The system of any preceding claim, wherein the data acquisition unit is further configured to acquire accessory data from one or more devices associated with artificial ventilation, wherein the one or more devices are operably connected to the patient manifold, the data acquisition unit, or both.

10. The system of any preceding claim, wherein the patient manifold and the at least one respiratory sensing arrangement are configured to remain in fluid communication with the airway adjunct upon disconnection of a current ventilator from fluid communication with the patient manifold.

11. The system of claim 10, wherein the patient manifold is configured for subsequent fluid connection with another ventilator while remaining in fluid communication with the airway adjunct.

12. The system of claim 11, wherein the data processing unit is configured to retain respiratory data acquired during ventilation by the current ventilator and respiratory data acquired during ventilation by the subsequently connected ventilator as portions of the same patient respiratory data record.

13. The system of claim 12, wherein the respiratory data retained in the same patient respiratory data record is generated using the same patient-side respiratory sensing arrangement during ventilation by the current ventilator and during ventilation by the subsequently connected ventilator.

14. The system of claim 12 or claim 13, wherein the data processing unit is configured to maintain the patient respiratory data record through a plurality of ventilator exchanges while the patient manifold and the at least one respiratory sensing arrangement remain operatively associated with the patient, and wherein at least one of the plurality of ventilator exchanges comprises replacement of a first mechanical ventilator with a second mechanical ventilator.

15. The system of any of claims 11-14, wherein one of the current ventilator and the subsequently connected ventilator is a manual ventilator and the other is a mechanical ventilator, and wherein the data acquisition unit is configured to acquire respiratory data beginning upon initiation of artificial ventilation using the manual ventilator and the data processing unit is configured to retain the acquired respiratory data as an initial portion of the patient respiratory data record.
